# EUROPEAN PATENT APPLICATION

(11) **EP 4 821 953 A1**
(43) Date of publication of application: **14.10.2026**
(21) Application number: 24899942.7
(22) Date of filing: 06.12.2024
(51) Int. Cl.: C07K 19/00, C07K 16/28, C12N 15/13, A61K 39/395, A61P 35/00

(54) **FUSION PROTEIN AND USE THEREOF**

(30) Priority: 08.12.2023 CN 202311692182
(71) Applicant: Bio-Thera Solutions, Ltd., Guangzhou, Guangdong 510005 (CN)
(72) Inventor: LIU, Guosheng, Guangzhou, Guangdong 510005 (CN); LI, Chuang, Guangzhou, Guangdong 510005 (CN); CHEN, Caiyan, Guangzhou, Guangdong 510005 (CN); CHEN, Yifan, Guangzhou, Guangdong 510005 (CN)
(74) Representative: Engelhard, Markus
(86) International application number: PCT/CN2024/137294
(87) International publication number: WO 2025/119308

(57) **Abstract**

The present disclosure provides a fusion protein and use thereof, wherein the fusion protein comprises an anti-PD-L1 antibody or an antigen-binding fragment, IL-15 or a fragment thereof, and IL-15Rα or a sushi domain thereof. The fusion protein of the present disclosure can be used for the prevention, treatment, or amelioration of a disease.

## Description

### TECHNICAL FIELD

The present disclosure belongs to the field of bio-pharmaceuticals, and particularly relates to a fusion protein and use thereof.

### BACKGROUND

Programmed death receptor-1 (PD-1) is a type I transmembrane glycoprotein with a molecular weight of about 55 kDa. PD-1 is an immunoinhibitory receptor expressed on activated T cells, B cells, and myeloid cells, and is a member of the CD28 immunoglobulin superfamily. As a ligand of PD-1, PD-L1 is also a type I transmembrane glycoprotein, which is widely distributed, and is expressed on the surface of antigen-presenting cells such as B cells, T cells, dendritic cells, and macrophages, as well as in tumor tissues.

The interaction between PD-1 and PD-L1 negatively regulates antigen receptor signal transduction, and weakens T cell response. So far, there are numerous studies showing that the interaction between PD-1 and PD-L1 results in a decrease in tumor-infiltrating lymphocytes, a decrease in T cell receptor-mediated proliferation, and immune evasion of cancer cells. Blocking the interaction between PD-1 and PD-L1 can increase T cell proliferation and cytokine production, improve the immunity of tumor-specific CD8⁺ T cells, and help the immune system to clear tumor cells.

IL-15 is a 14-15 kDa glycoprotein having 114 amino acids and belongs to the family of shared cytokine receptor γ chains that also includes IL-2, IL-4, IL-7, IL-9, and IL-21. IL-15 is secreted by macrophages, dendritic cells, and monocytes. IL-15 can stimulate central memory CD8⁺ cells to exert immunity, but has no regulatory effect on other T cells. Furthermore, IL-15 can activate NK cells as well as effector and memory CD8⁺ T cells and can rescue T cells from regulatory T cell (Treg)-induced apoptosis. The IL-15 receptor is composed of the following three subunits: IL-15Rα, IL-15Rβ, and IL-15Rγ. IL-15 typically forms a complex with the IL-15 receptor α expressed on APCs prior to binding to functional IL-15Rβ and γ units on T cells and NK cells. The sushi domain (7.5 kDa) of IL-15Rα plays a key role in the formation of the complex of IL-15 with IL-15Rα.

### SUMMARY

The present disclosure provides a fusion protein and use thereof, wherein the fusion protein comprises an anti-PD-L1 antibody or an antigen-binding fragment, IL-15 or a fragment thereof, and IL-15Rα or a sushi domain thereof.

In some embodiments, the anti-PD-L1 antibody or the antigen-binding fragment specifically binds to PD-L1, and comprises:
(a) HCDR1 comprising DSWIH; and/or
(b) HCDR2 comprising WISPYGGSTYYADX₁X₂X₃X₄ (SEQ ID NO: 86), wherein X₁ is S, D, H, G, P, or Y, X₂ is V, F, L, M, or Y, X₃ is K, R, G, S, V, or H, and X₄ is G, H, D, Q, S, or A; and/or
(c) HCDR3 comprising RHWPGGX₅X₆X₇ (SEQ ID NO: 87), wherein X₅ is F or L, X₆ is D or L, and X₇ is Y or P.

In some embodiments, the anti-PD-L1 antibody or the antigen-binding fragment specifically binds to PD-L1, and comprises:
(a) HCDR1 comprising DSWIH;
(b) HCDR2 comprising WISPYGGSTYYADX₁X₂X₃X₄ (SEQ ID NO: 86), wherein X₁ is S, D, H, G, P, or Y, X₂ is V, F, L, M, or Y, X₃ is K, R, G, S, V, or H, and X₄ is G, H, D, Q, S, or A; and
(c) HCDR3 comprising RHWPGGX₅X₆X₇ (SEQ ID NO: 87), wherein X₅ is F or L, X₆ is D or L, and X₇ is Y or P.

In some embodiments, the anti-PD-L1 antibody or the antigen-binding fragment specifically binds to PD-L1, and comprises:
(a) HCDR1 comprising DSWIH; and/or
(b) HCDR2 comprising WISPYGGSTYYADX₁X₂X₃X₄ (SEQ ID NO: 86), wherein X₁ is S, D, H, G, P, or Y, X₂ is V, F, L, M, or Y, X₃ is K, R, G, S, V, or H, and X₄ is G, H, D, Q, S, or A; and/or
(c) HCDR3 comprising RHWPGGX₅X₆X₇ (SEQ ID NO: 87), wherein X₅ is F or L, X₆ is D or L, and X₇ is Y or P; and/or
(d) LCDR1 comprising X₈ASQX₉IX₁₀X₁₁X₁₂LX₁₃ (SEQ ID NO: 88), wherein X₈ is L, Q, or R, X₉ is D, T, or G, X₁₀ is G or S, X₁₁ is K, T, or S, X₁₂ is H, W, F, or Y, and X₁₃ is N or A; and/or
(e) LCDR2 comprising X₁₄ASX₁₅LX₁₆X₁₇ (SEQ ID NO: 89), wherein X₁₄ is A or G, X₁₅ is T, N, S, or R, X₁₆ is Q or K, and X₁₇ is S or T; and/or
(f) LCDR3 comprising QQX₁₈X₁₉X₂₀TPX₂₁T (SEQ ID NO: 90), wherein X₁₈ is Y or S, X₁₉ is Y or F, X₂₀ is S or T, and X₂₁ is R or Y.

In some embodiments, the anti-PD-L1 antibody or the antigen-binding fragment specifically binds to PD-L1, and comprises:
(a) HCDR1 comprising DSWIH;
(b) HCDR2 comprising WISPYGGSTYYADX₁X₂X₃X₄ (SEQ ID NO: 86), wherein X₁ is S, D, H, G, P, or Y, X₂ is V, F, L, M, or Y, X₃ is K, R, G, S, V, or H, and X₄ is G, H, D, Q, S, or A;
(c) HCDR3 comprising RHWPGGX₅X₆X₇ (SEQ ID NO: 87), wherein X₅ is F or L, X₆ is D or L, and X₇ is Y or P;
(d) LCDR1 comprising X₈ASQX₉IX₁₀X₁₁X₁₂LX₁₃ (SEQ ID NO: 88), wherein X₈ is L, Q, or R, X₉ is D, T, or G, X₁₀ is G or S, X₁₁ is K, T, or S, X₁₂ is H, W, F, or Y, and X₁₃ is N or A;
(e) LCDR2 comprising X₁₄ASX₁₅LX₁₆X₁₇ (SEQ ID NO: 89), wherein X₁₄ is A or G, X₁₅ is T, N, S, or R, X₁₆ is Q or K, and X₁₇ is S or T; and
(f) LCDR3 comprising QQX₁₈X₁₉X₂₀TPX₂₁T (SEQ ID NO: 90), wherein X₁₈ is Y or S, X₁₉ is Y or F, X₂₀ is S or T, and X₂₁ is R or Y.

In some embodiments, the HCDR1 comprises the amino acid sequence set forth in SEQ ID NO: 1 or a variant thereof with a substitution, deletion or insertion at a single site. In some embodiments, the HCDR2 comprises an amino acid sequence set forth in any one of SEQ ID NOs: 2-11 or a variant thereof with a substitution, deletion or insertion at a single site. In some embodiments, the HCDR3 comprises an amino acid sequence set forth in SEQ ID NO: 12 or 13 or a variant thereof with a substitution, deletion or insertion at a single site. In some embodiments, the variant with the substitution is a variant with a conservative amino acid substitution.

In some embodiments, the HCDR1 comprises an amino acid sequence set forth in any one of SEQ ID NOs: 91-95 or a variant thereof with a substitution, deletion or insertion at a single site. In some embodiments, the HCDR2 comprises an amino acid sequence set forth in any one of SEQ ID NOs: 2-11 or a variant thereof with a substitution, deletion or insertion at a single site. In some embodiments, the HCDR3 comprises an amino acid sequence set forth in SEQ ID NO: 12 or 13 or a variant thereof with a substitution, deletion or insertion at a single site. In some embodiments, the variant with the substitution is a variant with a conservative amino acid substitution.

In some embodiments, the HCDR1 comprises the amino acid sequence set forth in SEQ ID NO: 1, the HCDR2 comprises the amino acid sequence set forth in SEQ ID NO: 2, and the HCDR3 comprises the amino acid sequence set forth in SEQ ID NO: 12.

In some embodiments, the HCDR1 comprises the amino acid sequence set forth in SEQ ID NO: 1, the HCDR2 comprises the amino acid sequence set forth in SEQ ID NO: 3, and the HCDR3 comprises the amino acid sequence set forth in SEQ ID NO: 12.

In some embodiments, the HCDR1 comprises the amino acid sequence set forth in SEQ ID NO: 1, the HCDR2 comprises the amino acid sequence set forth in SEQ ID NO: 4, and the HCDR3 comprises the amino acid sequence set forth in SEQ ID NO: 12.

In some embodiments, the HCDR1 comprises the amino acid sequence set forth in SEQ ID NO: 1, the HCDR2 comprises the amino acid sequence set forth in SEQ ID NO: 5, and the HCDR3 comprises the amino acid sequence set forth in SEQ ID NO: 12.

In some embodiments, the HCDR1 comprises the amino acid sequence set forth in SEQ ID NO: 1, the HCDR2 comprises the amino acid sequence set forth in SEQ ID NO: 6, and the HCDR3 comprises the amino acid sequence set forth in SEQ ID NO: 12.

In some embodiments, the HCDR1 comprises the amino acid sequence set forth in SEQ ID NO: 1, the HCDR2 comprises the amino acid sequence set forth in SEQ ID NO: 7, and the HCDR3 comprises the amino acid sequence set forth in SEQ ID NO: 12.

In some embodiments, the HCDR1 comprises the amino acid sequence set forth in SEQ ID NO: 1, the HCDR2 comprises the amino acid sequence set forth in SEQ ID NO: 8, and the HCDR3 comprises the amino acid sequence set forth in SEQ ID NO: 12.

In some embodiments, the HCDR1 comprises the amino acid sequence set forth in SEQ ID NO: 1, the HCDR2 comprises the amino acid sequence set forth in SEQ ID NO: 9, and the HCDR3 comprises the amino acid sequence set forth in SEQ ID NO: 12.

In some embodiments, the HCDR1 comprises the amino acid sequence set forth in SEQ ID NO: 1, the HCDR2 comprises the amino acid sequence set forth in SEQ ID NO: 10, and the HCDR3 comprises the amino acid sequence set forth in SEQ ID NO: 12.

In some embodiments, the HCDR1 comprises the amino acid sequence set forth in SEQ ID NO: 1, the HCDR2 comprises the amino acid sequence set forth in SEQ ID NO: 11, and the HCDR3 comprises the amino acid sequence set forth in SEQ ID NO: 12.

In some embodiments, the HCDR1 comprises the amino acid sequence set forth in SEQ ID NO: 1, the HCDR2 comprises the amino acid sequence set forth in SEQ ID NO: 2, and the HCDR3 comprises the amino acid sequence set forth in SEQ ID NO: 13.

In some embodiments, the HCDR1 comprises the amino acid sequence set forth in SEQ ID NO: 91, the HCDR2 comprises the amino acid sequence set forth in SEQ ID NO: 2, and the HCDR3 comprises the amino acid sequence set forth in SEQ ID NO: 12.

In some embodiments, the HCDR1 comprises the amino acid sequence set forth in SEQ ID NO: 91, the HCDR2 comprises the amino acid sequence set forth in SEQ ID NO: 3, and the HCDR3 comprises the amino acid sequence set forth in SEQ ID NO: 12.

In some embodiments, the HCDR1 comprises the amino acid sequence set forth in SEQ ID NO: 91, the HCDR2 comprises the amino acid sequence set forth in SEQ ID NO: 4, and the HCDR3 comprises the amino acid sequence set forth in SEQ ID NO: 12.

In some embodiments, the HCDR1 comprises the amino acid sequence set forth in SEQ ID NO: 91, the HCDR2 comprises the amino acid sequence set forth in SEQ ID NO: 5, and the HCDR3 comprises the amino acid sequence set forth in SEQ ID NO: 12.

In some embodiments, the HCDR1 comprises the amino acid sequence set forth in SEQ ID NO: 91, the HCDR2 comprises the amino acid sequence set forth in SEQ ID NO: 6, and the HCDR3 comprises the amino acid sequence set forth in SEQ ID NO: 12.

In some embodiments, the HCDR1 comprises the amino acid sequence set forth in SEQ ID NO: 91, the HCDR2 comprises the amino acid sequence set forth in SEQ ID NO: 7, and the HCDR3 comprises the amino acid sequence set forth in SEQ ID NO: 12.

In some embodiments, the HCDR1 comprises the amino acid sequence set forth in SEQ ID NO: 91, the HCDR2 comprises the amino acid sequence set forth in SEQ ID NO: 8, and the HCDR3 comprises the amino acid sequence set forth in SEQ ID NO: 12.

In some embodiments, the HCDR1 comprises the amino acid sequence set forth in SEQ ID NO: 91, the HCDR2 comprises the amino acid sequence set forth in SEQ ID NO: 9, and the HCDR3 comprises the amino acid sequence set forth in SEQ ID NO: 12.

In some embodiments, the HCDR1 comprises the amino acid sequence set forth in SEQ ID NO: 91, the HCDR2 comprises the amino acid sequence set forth in SEQ ID NO: 10, and the HCDR3 comprises the amino acid sequence set forth in SEQ ID NO: 12.

In some embodiments, the HCDR1 comprises the amino acid sequence set forth in SEQ ID NO: 91, the HCDR2 comprises the amino acid sequence set forth in SEQ ID NO: 11, and the HCDR3 comprises the amino acid sequence set forth in SEQ ID NO: 12.

In some embodiments, the HCDR1 comprises the amino acid sequence set forth in SEQ ID NO: 92, the HCDR2 comprises the amino acid sequence set forth in SEQ ID NO: 2, and the HCDR3 comprises the amino acid sequence set forth in SEQ ID NO: 12.

In some embodiments, the HCDR1 comprises the amino acid sequence set forth in SEQ ID NO: 93, the HCDR2 comprises the amino acid sequence set forth in SEQ ID NO: 2, and the HCDR3 comprises the amino acid sequence set forth in SEQ ID NO: 12.

In some embodiments, the HCDR1 comprises the amino acid sequence set forth in SEQ ID NO: 94, the HCDR2 comprises the amino acid sequence set forth in SEQ ID NO: 2, and the HCDR3 comprises the amino acid sequence set forth in SEQ ID NO: 12.

In some embodiments, the HCDR1 comprises the amino acid sequence set forth in SEQ ID NO: 95, the HCDR2 comprises the amino acid sequence set forth in SEQ ID NO: 2, and the HCDR3 comprises the amino acid sequence set forth in SEQ ID NO: 12.

In some embodiments, the HCDR1 comprises the amino acid sequence set forth in SEQ ID NO: 91, the HCDR2 comprises the amino acid sequence set forth in SEQ ID NO: 2, and the HCDR3 comprises the amino acid sequence set forth in SEQ ID NO: 13.

In some embodiments, the LCDR1 comprises an amino acid sequence set forth in any one of SEQ ID NOs: 14-18 or a variant thereof with a substitution, deletion or insertion at a single site. In some embodiments, the LCDR2 comprises an amino acid sequence set forth in any one of SEQ ID NOs: 19-22 or a variant thereof with a substitution, deletion or insertion at a single site. In some embodiments, the LCDR3 comprises an amino acid sequence set forth in any one of SEQ ID NOs: 23-26 or a variant thereof with a substitution, deletion or insertion at a single site. In some embodiments, the variant with the substitution is a variant with a conservative amino acid substitution.

In some embodiments, the LCDR1 comprises the amino acid sequence set forth in SEQ ID NO: 14, the LCDR2 comprises the amino acid sequence set forth in SEQ ID NO: 19, and the LCDR3 comprises the amino acid sequence set forth in SEQ ID NO: 23.

In some embodiments, the LCDR1 comprises the amino acid sequence set forth in SEQ ID NO: 15, the LCDR2 comprises the amino acid sequence set forth in SEQ ID NO: 20, and the LCDR3 comprises the amino acid sequence set forth in SEQ ID NO: 24.

In some embodiments, the LCDR1 comprises the amino acid sequence set forth in SEQ ID NO: 16, the LCDR2 comprises the amino acid sequence set forth in SEQ ID NO: 21, and the LCDR3 comprises the amino acid sequence set forth in SEQ ID NO: 25.

In some embodiments, the LCDR1 comprises the amino acid sequence set forth in SEQ ID NO: 17, the LCDR2 comprises the amino acid sequence set forth in SEQ ID NO: 22, and the LCDR3 comprises the amino acid sequence set forth in SEQ ID NO: 26.

In some embodiments, the LCDR1 comprises the amino acid sequence set forth in SEQ ID NO: 18, the LCDR2 comprises the amino acid sequence set forth in SEQ ID NO: 21, and the LCDR3 comprises the amino acid sequence set forth in SEQ ID NO: 25.

In some embodiments, the LCDR1 comprises the amino acid sequence set forth in SEQ ID NO: 14, the LCDR2 comprises the amino acid sequence set forth in SEQ ID NO: 21, and the LCDR3 comprises the amino acid sequence set forth in SEQ ID NO: 26.

In some embodiments, the anti-PD-L1 antibody or the antigen-binding fragment specifically binds to PD-L1, and comprises: (a) HCDR1 comprising the amino acid sequence set forth in SEQ ID NO: 1 or a variant thereof with a substitution, deletion or insertion at a single site; and/or (b) HCDR2 comprising an amino acid sequence set forth in any one of SEQ ID NOs: 2-11 or a variant thereof with a substitution, deletion or insertion at a single site; and/or (c) HCDR3 comprising an amino acid sequence set forth in SEQ ID NO: 12 or 13 or a variant thereof with a substitution, deletion or insertion at a single site; and/or (d) LCDR1 comprising an amino acid sequence set forth in any one of SEQ ID NOs: 14-18 or a variant thereof with a substitution, deletion or insertion at a single site; and/or (e) LCDR2 comprising an amino acid sequence set forth in any one of SEQ ID NOs: 19-22 or a variant thereof with a substitution, deletion or insertion at a single site; and/or (f) LCDR3 comprising an amino acid sequence set forth in any one of SEQ ID NOs: 23-26 or a variant thereof with a substitution, deletion or insertion at a single site.

In some embodiments, the anti-PD-L1 antibody or the antigen-binding fragment at least comprises one, two, three, four, five, or all of the HCDR1 set forth in SEQ ID NO: 1, the HCDR2 set forth in any one of SEQ ID NOs: 2-11, the HCDR3 set forth in SEQ ID NO: 12 or 13, the LCDR1 set forth in any one of SEQ ID NOs: 14-18, the LCDR2 set forth in any one of SEQ ID NOs: 19-22, and the LCDR3 set forth in any one of SEQ ID NOs: 23-26.

In some embodiments, the anti-PD-L1 antibody or the antigen-binding fragment comprises the HCDR1 set forth in SEQ ID NO: 1, the HCDR2 set forth in any one of SEQ ID NOs: 2-11, the HCDR3 set forth in SEQ ID NO: 12 or 13, the LCDR1 set forth in any one of SEQ ID NOs: 14-18, the LCDR2 set forth in any one of SEQ ID NOs: 19-22, and the LCDR3 set forth in any one of SEQ ID NOs: 23-26.

In some embodiments, the anti-PD-L1 antibody or the antigen-binding fragment specifically binds to PD-L1, and comprises: (a) HCDR1 comprising an amino acid sequence set forth in any one of SEQ ID NOs: 91-95 or a variant thereof with a substitution, deletion or insertion at a single site; and/or (b) HCDR2 comprising an amino acid sequence set forth in any one of SEQ ID NOs: 2-11 or a variant thereof with a substitution, deletion or insertion at a single site; and/or (c) HCDR3 comprising an amino acid sequence set forth in SEQ ID NO: 12 or 13 or a variant thereof with a substitution, deletion or insertion at a single site; and/or (d) LCDR1 comprising an amino acid sequence set forth in any one of SEQ ID NOs: 14-18 or a variant thereof with a substitution, deletion or insertion at a single site; and/or (e) LCDR2 comprising an amino acid sequence set forth in any one of SEQ ID NOs: 19-22 or a variant thereof with a substitution, deletion or insertion at a single site; and/or (f) LCDR3 comprising an amino acid sequence set forth in any one of SEQ ID NOs: 23-26 or a variant thereof with a substitution, deletion or insertion at a single site.

In some embodiments, the anti-PD-L1 antibody or the antigen-binding fragment at least comprises one, two, three, four, five, or all of the HCDR1 set forth in any one of SEQ ID NOs: 91-95, the HCDR2 set forth in any one of SEQ ID NOs: 2-11, the HCDR3 set forth in SEQ ID NO: 12 or 13, the LCDR1 set forth in any one of SEQ ID NOs: 14-18, the LCDR2 set forth in any one of SEQ ID NOs: 19-22, and the LCDR3 set forth in any one of SEQ ID NOs: 23-26.

In some embodiments, the anti-PD-L1 antibody or the antigen-binding fragment comprises the HCDR1 set forth in any one of SEQ ID NOs: 91-95, the HCDR2 set forth in any one of SEQ ID NOs: 2-11, the HCDR3 set forth in SEQ ID NO: 12 or 13, the LCDR1 set forth in any one of SEQ ID NOs: 14-18, the LCDR2 set forth in any one of SEQ ID NOs: 19-22, and the LCDR3 set forth in any one of SEQ ID NOs: 23-26.

In some embodiments, the anti-PD-L1 antibody or the antigen-binding fragment comprises HCDR1 set forth in SEQ ID NO: 1, HCDR2 set forth in SEQ ID NO: 2, HCDR3 set forth in SEQ ID NO: 12, LCDR1 set forth in SEQ ID NO: 14, LCDR2 set forth in SEQ ID NO: 19, and LCDR3 set forth in SEQ ID NO: 23.

In some embodiments, the anti-PD-L1 antibody or the antigen-binding fragment comprises HCDR1 set forth in SEQ ID NO: 1, HCDR2 set forth in SEQ ID NO: 2, HCDR3 set forth in SEQ ID NO: 12, LCDR1 set forth in SEQ ID NO: 15, LCDR2 set forth in SEQ ID NO: 20, and LCDR3 set forth in SEQ ID NO: 24.

In some embodiments, the anti-PD-L1 antibody or the antigen-binding fragment comprises HCDR1 set forth in SEQ ID NO: 1, HCDR2 set forth in SEQ ID NO: 2, HCDR3 set forth in SEQ ID NO: 12, LCDR1 set forth in SEQ ID NO: 16, LCDR2 set forth in SEQ ID NO: 21, and LCDR3 set forth in SEQ ID NO: 25.

In some embodiments, the anti-PD-L1 antibody or the antigen-binding fragment comprises HCDR1 set forth in SEQ ID NO: 1, HCDR2 set forth in SEQ ID NO: 2, HCDR3 set forth in SEQ ID NO: 12, LCDR1 set forth in SEQ ID NO: 17, LCDR2 set forth in SEQ ID NO: 22, and LCDR3 set forth in SEQ ID NO: 26.

In some embodiments, the anti-PD-L1 antibody or the antigen-binding fragment comprises HCDR1 set forth in SEQ ID NO: 1, HCDR2 set forth in SEQ ID NO: 2, HCDR3 set forth in SEQ ID NO: 12, LCDR1 set forth in SEQ ID NO: 18, LCDR2 set forth in SEQ ID NO: 21, and LCDR3 set forth in SEQ ID NO: 25.

In some embodiments, the anti-PD-L1 antibody or the antigen-binding fragment comprises HCDR1 set forth in SEQ ID NO: 1, HCDR2 set forth in SEQ ID NO: 2, HCDR3 set forth in SEQ ID NO: 12, LCDR1 set forth in SEQ ID NO: 14, LCDR2 set forth in SEQ ID NO: 21, and LCDR3 set forth in SEQ ID NO: 26.

In some embodiments, the anti-PD-L1 antibody or the antigen-binding fragment comprises HCDR1 set forth in SEQ ID NO: 1, HCDR2 set forth in SEQ ID NO: 3, HCDR3 set forth in SEQ ID NO: 12, LCDR1 set forth in SEQ ID NO: 14, LCDR2 set forth in SEQ ID NO: 19, and LCDR3 set forth in SEQ ID NO: 23.

In some embodiments, the anti-PD-L1 antibody or the antigen-binding fragment comprises HCDR1 set forth in SEQ ID NO: 1, HCDR2 set forth in SEQ ID NO: 4, HCDR3 set forth in SEQ ID NO: 12, LCDR1 set forth in SEQ ID NO: 14, LCDR2 set forth in SEQ ID NO: 19, and LCDR3 set forth in SEQ ID NO: 23.

In some embodiments, the anti-PD-L1 antibody or the antigen-binding fragment comprises HCDR1 set forth in SEQ ID NO: 1, HCDR2 set forth in SEQ ID NO: 5, HCDR3 set forth in SEQ ID NO: 12, LCDR1 set forth in SEQ ID NO: 14, LCDR2 set forth in SEQ ID NO: 19, and LCDR3 set forth in SEQ ID NO: 23.

In some embodiments, the anti-PD-L1 antibody or the antigen-binding fragment comprises HCDR1 set forth in SEQ ID NO: 1, HCDR2 set forth in SEQ ID NO: 6, HCDR3 set forth in SEQ ID NO: 12, LCDR1 set forth in SEQ ID NO: 14, LCDR2 set forth in SEQ ID NO: 19, and LCDR3 set forth in SEQ ID NO: 23.

In some embodiments, the anti-PD-L1 antibody or the antigen-binding fragment comprises HCDR1 set forth in SEQ ID NO: 1, HCDR2 set forth in SEQ ID NO: 7, HCDR3 set forth in SEQ ID NO: 12, LCDR1 set forth in SEQ ID NO: 14, LCDR2 set forth in SEQ ID NO: 19, and LCDR3 set forth in SEQ ID NO: 23.

In some embodiments, the anti-PD-L1 antibody or the antigen-binding fragment comprises HCDR1 set forth in SEQ ID NO: 91, HCDR2 set forth in SEQ ID NO: 2, HCDR3 set forth in SEQ ID NO: 12, LCDR1 set forth in SEQ ID NO: 14, LCDR2 set forth in SEQ ID NO: 19, and LCDR3 set forth in SEQ ID NO: 23.

In some embodiments, the anti-PD-L1 antibody or the antigen-binding fragment comprises HCDR1 set forth in SEQ ID NO: 91, HCDR2 set forth in SEQ ID NO: 2, HCDR3 set forth in SEQ ID NO: 12, LCDR1 set forth in SEQ ID NO: 15, LCDR2 set forth in SEQ ID NO: 20, and LCDR3 set forth in SEQ ID NO: 24.

In some embodiments, the anti-PD-L1 antibody or the antigen-binding fragment comprises HCDR1 set forth in SEQ ID NO: 91, HCDR2 set forth in SEQ ID NO: 2, HCDR3 set forth in SEQ ID NO: 12, LCDR1 set forth in SEQ ID NO: 16, LCDR2 set forth in SEQ ID NO: 21, and LCDR3 set forth in SEQ ID NO: 25.

In some embodiments, the anti-PD-L1 antibody or the antigen-binding fragment comprises HCDR1 set forth in SEQ ID NO: 91, HCDR2 set forth in SEQ ID NO: 2, HCDR3 set forth in SEQ ID NO: 12, LCDR1 set forth in SEQ ID NO: 17, LCDR2 set forth in SEQ ID NO: 22, and LCDR3 set forth in SEQ ID NO: 26.

In some embodiments, the anti-PD-L1 antibody or the antigen-binding fragment comprises HCDR1 set forth in SEQ ID NO: 91, HCDR2 set forth in SEQ ID NO: 2, HCDR3 set forth in SEQ ID NO: 12, LCDR1 set forth in SEQ ID NO: 18, LCDR2 set forth in SEQ ID NO: 21, and LCDR3 set forth in SEQ ID NO: 25.

In some embodiments, the anti-PD-L1 antibody or the antigen-binding fragment comprises HCDR1 set forth in SEQ ID NO: 91, HCDR2 set forth in SEQ ID NO: 2, HCDR3 set forth in SEQ ID NO: 12, LCDR1 set forth in SEQ ID NO: 14, LCDR2 set forth in SEQ ID NO: 21, and LCDR3 set forth in SEQ ID NO: 26.

In some embodiments, the anti-PD-L1 antibody or the antigen-binding fragment comprises HCDR1 set forth in SEQ ID NO: 91, HCDR2 set forth in SEQ ID NO: 3, HCDR3 set forth in SEQ ID NO: 12, LCDR1 set forth in SEQ ID NO: 14, LCDR2 set forth in SEQ ID NO: 19, and LCDR3 set forth in SEQ ID NO: 23.

In some embodiments, the anti-PD-L1 antibody or the antigen-binding fragment comprises HCDR1 set forth in SEQ ID NO: 91, HCDR2 set forth in SEQ ID NO: 4, HCDR3 set forth in SEQ ID NO: 12, LCDR1 set forth in SEQ ID NO: 14, LCDR2 set forth in SEQ ID NO: 19, and LCDR3 set forth in SEQ ID NO: 23.

In some embodiments, the anti-PD-L1 antibody or the antigen-binding fragment comprises HCDR1 set forth in SEQ ID NO: 91, HCDR2 set forth in SEQ ID NO: 5, HCDR3 set forth in SEQ ID NO: 12, LCDR1 set forth in SEQ ID NO: 14, LCDR2 set forth in SEQ ID NO: 19, and LCDR3 set forth in SEQ ID NO: 23.

In some embodiments, the anti-PD-L1 antibody or the antigen-binding fragment comprises HCDR1 set forth in SEQ ID NO: 91, HCDR2 set forth in SEQ ID NO: 6, HCDR3 set forth in SEQ ID NO: 12, LCDR1 set forth in SEQ ID NO: 14, LCDR2 set forth in SEQ ID NO: 19, and LCDR3 set forth in SEQ ID NO: 23.

In some embodiments, the anti-PD-L1 antibody or the antigen-binding fragment comprises HCDR1 set forth in SEQ ID NO: 91, HCDR2 set forth in SEQ ID NO: 7, HCDR3 set forth in SEQ ID NO: 12, LCDR1 set forth in SEQ ID NO: 14, LCDR2 set forth in SEQ ID NO: 19, and LCDR3 set forth in SEQ ID NO: 23.

In some embodiments, the anti-PD-L1 antibody or the antigen-binding fragment comprises a heavy chain variable region (VH) and a light chain variable region (VL).

In some embodiments, the heavy chain variable region comprises the structure heavy chain FR1-HCDR1-heavy chain FR2-HCDR2-heavy chain FR3-HCDR3-heavy chain FR4.

In some embodiments, the light chain variable region comprises the structure light chain FR1-LCDR1-light chain FR2-LCDR2-light chain FR3-LCDR3-light chain FR4.

In some embodiments, the heavy chain variable region of the anti-PD-L1 antibody or the antigen-binding fragment comprises an amino acid sequence set forth in any one of SEQ ID NOs: 27-41, or an amino acid sequence having at least 90% identity to a sequence set forth in any one of SEQ ID NOs: 27-41, or an amino acid sequence having one or more conservative amino acid substitutions as compared to a sequence set forth in any one of SEQ ID NOs: 27-41.

In some embodiments, the light chain variable region of the anti-PD-L1 antibody or the antigen-binding fragment comprises an amino acid sequence set forth in any one of SEQ ID NOs: 42-47, or an amino acid sequence having at least 90% identity to a sequence set forth in any one of SEQ ID NOs: 42-47, or an amino acid sequence having one or more conservative amino acid substitutions as compared to a sequence set forth in any one of SEQ ID NOs: 42-47.

In some embodiments, the heavy chain variable region of the anti-PD-L1 antibody or the antigen-binding fragment comprises an amino acid sequence set forth in any one of SEQ ID NOs: 27-41, and the light chain variable region of the anti-PD-L1 antibody or the antigen-binding fragment comprises an amino acid sequence set forth in any one of SEQ ID NOs: 42-47.

In some embodiments, the heavy chain variable region of the anti-PD-L1 antibody or the antigen-binding fragment comprises the amino acid sequence set forth in SEQ ID NO: 27, and the light chain variable region of the anti-PD-L1 antibody or the antigen-binding fragment comprises the amino acid sequence set forth in SEQ ID NO: 42.

In some embodiments, the heavy chain variable region of the anti-PD-L1 antibody or the antigen-binding fragment comprises the amino acid sequence set forth in SEQ ID NO: 27, and the light chain variable region of the anti-PD-L1 antibody or the antigen-binding fragment comprises the amino acid sequence set forth in SEQ ID NO: 43.

In some embodiments, the heavy chain variable region of the anti-PD-L1 antibody or the antigen-binding fragment comprises the amino acid sequence set forth in SEQ ID NO: 27, and the light chain variable region of the anti-PD-L1 antibody or the antigen-binding fragment comprises the amino acid sequence set forth in SEQ ID NO: 44.

In some embodiments, the heavy chain variable region of the anti-PD-L1 antibody or the antigen-binding fragment comprises the amino acid sequence set forth in SEQ ID NO: 27, and the light chain variable region of the anti-PD-L1 antibody or the antigen-binding fragment comprises the amino acid sequence set forth in SEQ ID NO: 45.

In some embodiments, the heavy chain variable region of the anti-PD-L1 antibody or the antigen-binding fragment comprises the amino acid sequence set forth in SEQ ID NO: 27, and the light chain variable region of the anti-PD-L1 antibody or the antigen-binding fragment comprises the amino acid sequence set forth in SEQ ID NO: 46.

In some embodiments, the heavy chain variable region of the anti-PD-L1 antibody or the antigen-binding fragment comprises the amino acid sequence set forth in SEQ ID NO: 27, and the light chain variable region of the anti-PD-L1 antibody or the antigen-binding fragment comprises the amino acid sequence set forth in SEQ ID NO: 47.

In some embodiments, the heavy chain variable region of the anti-PD-L1 antibody or the antigen-binding fragment comprises the amino acid sequence set forth in SEQ ID NO: 28, and the light chain variable region of the anti-PD-L1 antibody or the antigen-binding fragment comprises the amino acid sequence set forth in SEQ ID NO: 42.

In some embodiments, the heavy chain variable region of the anti-PD-L1 antibody or the antigen-binding fragment comprises the amino acid sequence set forth in SEQ ID NO: 29, and the light chain variable region of the anti-PD-L1 antibody or the antigen-binding fragment comprises the amino acid sequence set forth in SEQ ID NO: 42.

In some embodiments, the heavy chain variable region of the anti-PD-L1 antibody or the antigen-binding fragment comprises the amino acid sequence set forth in SEQ ID NO: 30, and the light chain variable region of the anti-PD-L1 antibody or the antigen-binding fragment comprises the amino acid sequence set forth in SEQ ID NO: 42.

In some embodiments, the heavy chain variable region of the anti-PD-L1 antibody or the antigen-binding fragment comprises the amino acid sequence set forth in SEQ ID NO: 31, and the light chain variable region of the anti-PD-L1 antibody or the antigen-binding fragment comprises the amino acid sequence set forth in SEQ ID NO: 42.

In some embodiments, the heavy chain variable region of the anti-PD-L1 antibody or the antigen-binding fragment comprises the amino acid sequence set forth in SEQ ID NO: 32, and the light chain variable region of the anti-PD-L1 antibody or the antigen-binding fragment comprises the amino acid sequence set forth in SEQ ID NO: 42.

In some embodiments, the antibody or the antigen-binding fragment further comprises a heavy chain constant region, a light chain constant region, an Fc region, or a combination thereof. In some embodiments, the light chain constant region is a κ or λ chain constant region. In some embodiments, the antibody or the fragment thereof is of one of the isotypes IgG, IgM, IgA, IgE or IgD. In some embodiments, the isotype is IgG1, IgG2, IgG3 or IgG4. In some embodiments, the antibody or the antigen-binding fragment is a murine antibody, a chimeric antibody, a humanized antibody, or a fully human antibody.

In some embodiments, the Fc is a variant Fc region. In some embodiments, the variant Fc region has one or more amino acid modifications, such as substitutions, deletions, or insertions, relative to the parent Fc region. In some embodiments, the amino acid modification of the Fc region alters effector function activity, relative to the activity of the parent Fc region. In some embodiments, the variant Fc region can have altered (i.e., increased or decreased) antibody-dependent cellular cytotoxicity (ADCC), complement-dependent cytotoxicity (CDC), phagocytosis, opsonization, or cell binding. In some embodiments, the amino acid modification of the Fc region can alter the affinity of the variant Fc region for an FcγR (Fcγ receptor), relative to the parent Fc region. In some embodiments, the Fc region is derived from IgG1 or IgG4. In some embodiments, the Fc region mutation is N297A.

In some embodiments, the antibody or the antigen-binding fragment is an isolated antibody or an antigen-binding fragment. In some embodiments, the antibody or the antigen-binding fragment is an scFv, a Fab, a F(ab)₂, or an IgG. In some embodiments, the antibody or the antigen-binding fragment is a monoclonal antibody. In one embodiment, the antigen-binding fragment is a Fab antibody, an Fv antibody, or an scFv antibody.

In some embodiments, the anti-PD-L1 antibody or the antigen-binding fragment further comprises a heavy chain constant region (CH) and a light chain constant region (CL).

In some embodiments, the heavy chain constant region of the anti-PD-L1 antibody or the antigen-binding fragment comprises an amino acid sequence set forth in SEQ ID NO: 48 or 49, or an amino acid sequence having at least 90% identity to the sequence set forth in SEQ ID NO: 48 or 49, or an amino acid sequence having one or more conservative amino acid substitutions as compared to the sequence set forth in SEQ ID NO: 48 or 49; and/or
the light chain constant region of the anti-PD-L1 antibody or the antigen-binding fragment comprises the amino acid sequence set forth in SEQ ID NO: 50, or an amino acid sequence having at least 90% identity to the sequence set forth in SEQ ID NO: 50, or an amino acid sequence having one or more conservative amino acid substitutions as compared to the sequence set forth in SEQ ID NO: 50.

In some embodiments, the heavy chain constant region of the anti-PD-L1 antibody or the antigen-binding fragment comprises the amino acid sequence set forth in SEQ ID NO: 48, and the light chain constant region of the anti-PD-L1 antibody or the antigen-binding fragment comprises the amino acid sequence set forth in SEQ ID NO: 50.

In some embodiments, the heavy chain constant region of the anti-PD-L1 antibody or the antigen-binding fragment comprises the amino acid sequence set forth in SEQ ID NO: 49, and the light chain constant region of the anti-PD-L1 antibody or the antigen-binding fragment comprises the amino acid sequence set forth in SEQ ID NO: 50.

In some embodiments, the anti-PD-L1 antibody or the antigen-binding fragment further comprises a heavy chain constant region a (CHa), a heavy chain constant region b (CHb), and a light chain constant region (CL).

In some embodiments, the heavy chain constant region a and the heavy chain constant region b form a "Knobs-into-Holes" stable association.

In some embodiments, the heavy chain constant region a and/or the heavy chain constant region b comprises an amino acid mutation selected from Y349C, S354C, T366W, T366S, L368A, and Y407V, wherein the amino acid position is Eu numbered.

In some embodiments, the heavy chain constant region a and/or the heavy chain constant region b comprises the following amino acid mutation: K447A, wherein the amino acid position is Eu numbered.

In some embodiments, the heavy chain constant region a comprises an amino acid mutation selected from S354C and T366W, and/or the heavy chain constant region b comprises an amino acid mutation selected from Y349C, T366S, L368A, and Y407V, wherein the amino acid position is Eu numbered.

In some embodiments, the heavy chain constant region a comprises the following amino acid mutations: S354C and T366W, and/or the heavy chain constant region b comprises the following amino acid mutations: Y349C, T366S, L368A, and Y407V, wherein the amino acid position is Eu numbered.

In some embodiments, the heavy chain constant region a comprises the following amino acid mutations: S354C and T366W, and the heavy chain constant region b comprises the following amino acid mutations: Y349C, T366S, L368A, and Y407V, wherein the amino acid position is Eu numbered.

In some embodiments, the heavy chain constant region a comprises the following amino acid mutations: S354C, T366W, and K447A, and/or the heavy chain constant region b comprises the following amino acid mutations: Y349C, T366S, L368A, Y407V, and K447A, wherein the amino acid position is Eu numbered.

In some embodiments, the heavy chain constant region a comprises the following amino acid mutations: S354C, T366W, and K447A, and the heavy chain constant region b comprises the following amino acid mutations: Y349C, T366S, L368A, Y407V, and K447A, wherein the amino acid position is Eu numbered.

In some embodiments, the heavy chain constant region a comprises the amino acid sequence set forth in SEQ ID NO: 77, or an amino acid sequence having at least 90% identity to the sequence set forth in SEQ ID NO: 77, or an amino acid sequence having one or more conservative amino acid substitutions as compared to the sequence set forth in SEQ ID NO: 77; and/or
the heavy chain constant region b comprises the amino acid sequence set forth in SEQ ID NO: 78, or an amino acid sequence having at least 90% identity to the sequence set forth in SEQ ID NO: 78, or an amino acid sequence having one or more conservative amino acid substitutions as compared to the sequence set forth in SEQ ID NO: 78; and/or
the light chain constant region comprises the amino acid sequence set forth in SEQ ID NO: 50, or an amino acid sequence having at least 90% identity to the sequence set forth in SEQ ID NO: 50, or an amino acid sequence having one or more conservative amino acid substitutions as compared to the sequence set forth in SEQ ID NO: 50.

In some embodiments, the heavy chain constant region a comprises the amino acid sequence set forth in SEQ ID NO: 77, or an amino acid sequence having at least 90% identity to the sequence set forth in SEQ ID NO: 77, or an amino acid sequence having one or more conservative amino acid substitutions as compared to the sequence set forth in SEQ ID NO: 77; the heavy chain constant region b comprises the amino acid sequence set forth in SEQ ID NO: 78, or an amino acid sequence having at least 90% identity to the sequence set forth in SEQ ID NO: 78, or an amino acid sequence having one or more conservative amino acid substitutions as compared to the sequence set forth in SEQ ID NO: 78; the light chain constant region comprises the amino acid sequence set forth in SEQ ID NO: 50, or an amino acid sequence having at least 90% identity to the sequence set forth in SEQ ID NO: 50, or an amino acid sequence having one or more conservative amino acid substitutions as compared to the sequence set forth in SEQ ID NO: 50.

In some embodiments, the heavy chain constant region a comprises the amino acid sequence set forth in SEQ ID NO: 77, the heavy chain constant region b comprises the amino acid sequence set forth in SEQ ID NO: 78, and the light chain constant region comprises the amino acid sequence set forth in SEQ ID NO: 50.

In some embodiments, the anti-PD-L1 antibody comprises a heavy chain and a light chain.

In some embodiments, the heavy chain of the anti-PD-L1 antibody comprises an amino acid sequence set forth in any one of SEQ ID NOs: 51-65, or an amino acid sequence having at least 90% identity to a sequence set forth in any one of SEQ ID NOs: 51-65, or an amino acid sequence having one or more conservative amino acid substitutions as compared to a sequence set forth in any one of SEQ ID NOs: 51-65; and/or
the light chain of the anti-PD-L1 antibody comprises an amino acid sequence set forth in any one of SEQ ID NOs: 66-71, or an amino acid sequence having at least 90% identity to a sequence set forth in any one of SEQ ID NOs: 66-71, or an amino acid sequence having one or more conservative amino acid substitutions as compared to a sequence set forth in any one of SEQ ID NOs: 66-71.

In some embodiments, the heavy chain of the anti-PD-L1 antibody comprises the amino acid sequence set forth in SEQ ID NO: 51, and the light chain of the anti-PD-L1 antibody comprises the amino acid sequence set forth in SEQ ID NO: 66.

In some embodiments, the heavy chain of the anti-PD-L1 antibody comprises the amino acid sequence set forth in SEQ ID NO: 51, and the light chain of the anti-PD-L1 antibody comprises the amino acid sequence set forth in SEQ ID NO: 67.

In some embodiments, the heavy chain of the anti-PD-L1 antibody comprises the amino acid sequence set forth in SEQ ID NO: 51, and the light chain of the anti-PD-L1 antibody comprises the amino acid sequence set forth in SEQ ID NO: 68.

In some embodiments, the heavy chain of the anti-PD-L1 antibody comprises the amino acid sequence set forth in SEQ ID NO: 51, and the light chain of the anti-PD-L1 antibody comprises the amino acid sequence set forth in SEQ ID NO: 69.

In some embodiments, the heavy chain of the anti-PD-L1 antibody comprises the amino acid sequence set forth in SEQ ID NO: 51, and the light chain of the anti-PD-L1 antibody comprises the amino acid sequence set forth in SEQ ID NO: 70.

In some embodiments, the heavy chain of the anti-PD-L1 antibody comprises the amino acid sequence set forth in SEQ ID NO: 51, and the light chain of the anti-PD-L1 antibody comprises the amino acid sequence set forth in SEQ ID NO: 71.

In some embodiments, the heavy chain of the anti-PD-L1 antibody comprises the amino acid sequence set forth in SEQ ID NO: 52, and the light chain of the anti-PD-L1 antibody comprises the amino acid sequence set forth in SEQ ID NO: 66.

In some embodiments, the heavy chain of the anti-PD-L1 antibody comprises the amino acid sequence set forth in SEQ ID NO: 53, and the light chain of the anti-PD-L1 antibody comprises the amino acid sequence set forth in SEQ ID NO: 66.

In some embodiments, the heavy chain of the anti-PD-L1 antibody comprises the amino acid sequence set forth in SEQ ID NO: 54, and the light chain of the anti-PD-L1 antibody comprises the amino acid sequence set forth in SEQ ID NO: 66.

In some embodiments, the heavy chain of the anti-PD-L1 antibody comprises the amino acid sequence set forth in SEQ ID NO: 55, and the light chain of the anti-PD-L1 antibody comprises the amino acid sequence set forth in SEQ ID NO: 66.

In some embodiments, the heavy chain of the anti-PD-L1 antibody comprises the amino acid sequence set forth in SEQ ID NO: 56, and the light chain of the anti-PD-L1 antibody comprises the amino acid sequence set forth in SEQ ID NO: 66.

In one embodiment, the antibody or the antigen-binding fragment is a monoclonal antibody (including a full-length monoclonal antibody), a polyclonal antibody, or a multispecific antibody or an antigen-binding fragment (e.g., a bispecific antibody or an antigen-binding fragment).

In some embodiments, the anti-PD-L1 antibody comprises a heavy chain a, a heavy chain b, and a light chain.

In some embodiments, the heavy chain a comprises the amino acid sequence set forth in SEQ ID NO: 79, or an amino acid sequence having at least 90% identity to the sequence set forth in SEQ ID NO: 79, or an amino acid sequence having one or more conservative amino acid substitutions as compared to the sequence set forth in SEQ ID NO: 79; and/or
the heavy chain b comprises the amino acid sequence set forth in SEQ ID NO: 80, or an amino acid sequence having at least 90% identity to the sequence set forth in SEQ ID NO: 80, or an amino acid sequence having one or more conservative amino acid substitutions as compared to the sequence set forth in SEQ ID NO: 80; and/or
the light chain comprises the amino acid sequence set forth in SEQ ID NO: 66, or an amino acid sequence having at least 90% identity to the sequence set forth in SEQ ID NO: 66, or an amino acid sequence having one or more conservative amino acid substitutions as compared to the sequence set forth in SEQ ID NO: 66.

In some embodiments, the heavy chain a comprises the amino acid sequence set forth in SEQ ID NO: 79, the heavy chain b comprises the amino acid sequence set forth in SEQ ID NO: 80, and the light chain comprises the amino acid sequence set forth in SEQ ID NO: 66.

In some embodiments, the IL-15 comprises the amino acid sequence set forth in SEQ ID NO: 82, or an amino acid sequence having at least 90% identity to the sequence set forth in SEQ ID NO: 82, or an amino acid sequence having one or more conservative amino acid substitutions as compared to the sequence set forth in SEQ ID NO: 82.

In some embodiments, the IL-15Rα or the sushi domain thereof comprises the amino acid sequence set forth in SEQ ID NO: 81, or an amino acid sequence having at least 90% identity to the sequence set forth in SEQ ID NO: 81, or an amino acid sequence having one or more conservative amino acid substitutions as compared to the sequence set forth in SEQ ID NO: 81.

In some embodiments, the anti-PD-L1 antibody or the antigen-binding fragment is linked to the IL-15 or the fragment thereof and the IL-15Rα or the sushi domain thereof via a linker.

In some embodiments, the C-terminus of one heavy chain of the anti-PD-L1 antibody is linked to the IL-15 or the fragment thereof via a linker, and the C-terminus of the other heavy chain of the anti-PD-L1 antibody is linked to the IL-15Rα or the sushi domain thereof via a linker.

In some embodiments, the linker is a GS linker. In some embodiments, the linker is independently selected from GS, GGS, GGGS, GGGGS, SGGGS, GGSS, (GGGGS)₂, (GGGGS)₃, or any combination thereof. In some embodiments, the linker is (GₘS)ₙ, wherein each m is independently 1, 2, 3, 4, 5, or 6, and n is 1, 2, 3, 4, or 5.

In some embodiments, the fusion protein comprises a first polypeptide, a second polypeptide, and a third polypeptide, wherein
the first polypeptide comprises the amino acid sequence set forth in SEQ ID NO: 83, or an amino acid sequence having at least 90% identity to the sequence set forth in SEQ ID NO: 83, or an amino acid sequence having one or more conservative amino acid substitutions as compared to the sequence set forth in SEQ ID NO: 83; and/or
the second polypeptide comprises the amino acid sequence set forth in SEQ ID NO: 84, or an amino acid sequence having at least 90% identity to the sequence set forth in SEQ ID NO: 84, or an amino acid sequence having one or more conservative amino acid substitutions as compared to the sequence set forth in SEQ ID NO: 84; and/or
the third polypeptide comprises the amino acid sequence set forth in SEQ ID NO: 66, or an amino acid sequence having at least 90% identity to the sequence set forth in SEQ ID NO: 66, or an amino acid sequence having one or more conservative amino acid substitutions as compared to the sequence set forth in SEQ ID NO: 66.

In some embodiments, the first polypeptide comprises the amino acid sequence set forth in SEQ ID NO: 83, the second polypeptide comprises the amino acid sequence set forth in SEQ ID NO: 84, and the third polypeptide comprises the amino acid sequence set forth in SEQ ID NO: 66.

In one embodiment, the fusion protein is an isolated fusion protein. The present disclosure further provides a polynucleotide encoding the fusion protein or a portion thereof. In some embodiments, the polynucleotide is an isolated polynucleotide.

The present disclosure further provides a vector comprising the polynucleotide. In some embodiments, the vector is an isolated vector. In some embodiments, the vector is a nucleic acid fragment, a plasmid, a phage, or a virus.

The present disclosure further provides a host cell comprising the polynucleotide or the vector. In some embodiments, the host cell is an isolated host cell. In some embodiments, the host cell is a CHO cell, a HEK cell (e.g., a HEK293F cell), a BHK cell, a Cos1 cell, a Cos7 cell, a CV1 cell, or a murine L cell.

The present disclosure further provides a pharmaceutical composition comprising the fusion protein described herein. In some embodiments, the pharmaceutical composition further comprises a pharmaceutically acceptable excipient.

The present disclosure further provides a treatment method and use. In some embodiments, provided is a method for preventing, treating, or ameliorating a disease, comprising administering to a patient an effective amount of the fusion protein or the pharmaceutical composition described herein. In some embodiments, provided is use of the fusion protein or the pharmaceutical composition described herein in preventing, treating, or ameliorating a disease. In some embodiments, provided is use of the fusion protein or the pharmaceutical composition described herein in preparing a medicament for preventing, treating, or ameliorating a disease.

In some embodiments, the disease includes, but is not limited to, an infection (e.g., an infection caused by bacteria, viruses, fungi, or protozoa), an autoimmune disease, a cancer, and a tumor. In some embodiments, the autoimmune disease includes, but is not limited to, alopecia areata, autoimmune hepatitis, celiac disease, Graves' disease, Guillain-Barre syndrome, Hashimoto's disease, hemolytic anemia, inflammatory bowel disease, inflammatory myopathy, multiple sclerosis, primary biliary cirrhosis, psoriasis, rheumatoid arthritis, scleroderma, Sjögren's syndrome, systemic lupus erythematosus, vitiligo, autoimmune pancreatitis, autoimmune urticaria, autoimmune thrombocytopenic purpura, Crohn's disease, type I diabetes, eosinophilic fasciitis, eosinophilic gastroenteritis, Goodpasture's syndrome, myasthenia gravis, psoriatic arthritis, rheumatic fever, ulcerative colitis, vasculitis, and Wegener's granulomatosis. In some embodiments, the cancer and the tumor include, but are not limited to, breast cancer, lung cancer, colon cancer, ovarian cancer, melanoma, bladder cancer, renal cancer, liver cancer, salivary gland cancer, gastric cancer, glioma, thyroid cancer, thymus cancer, epithelial cancer, head cancer, neck cancer, and pancreatic cancer.

The anti-PD-L1 antibody of the fusion protein of the present disclosure is in the form of an intact antibody, which can well relieve the immunosuppression caused by tumor cells. Meanwhile, the activity of IL-15 is weakened, which can avoid the systemic immune activation. The anti-PD-L1 antibody end can enrich IL-15 in the tumor microenvironment to generate local immune activation, which can not only improve the anti-tumor effect but also increase the safety. Compared with the anti-PD-L1 antibody, the fusion protein of the present disclosure can significantly activate the proliferation activity of CTLL-2 cells, can significantly promote the expansion of CD8⁺ T cells, NK cells, and NKT cells, and has a better tumor inhibition effect.

### BRIEF DESCRIPTION OF THE DRAWINGS

FIG. 1 shows a schematic structural diagram of fusion protein A.
FIG. 2 shows an experiment on the binding of fusion protein A to CTLL-2 cells.
FIG. 3 shows an experiment in which the activity of CTLL-2 cells is stimulated by fusion protein A.
FIG. 4 shows an experiment on the binding of fusion protein A to HH cells.
FIG. 5 shows an experiment on the activation of HH cells by fusion protein A.
FIG. 6 shows an experiment on the binding of fusion protein A to CHO-K1-CD122-CD132 cells.
FIG. 7 shows an experiment on the activation of PBMCs by fusion protein A *in vitro*, wherein FIG. 7a shows the total cell number, FIG. 7b shows the proportion of CD8⁺ T cells, FIG. 7c shows the proportion of NKT cells, and FIG. 7d shows the proportion of NK cells.
FIG. 8 shows an experiment in which PBMCs are activated to release cytokines in a solid phase state, wherein FIGs. 8a-8e show concentrations of IL-2, IFN-γ, IL-6, IL-10, and TNF-α, respectively; in the figure, donor 1, donor 2, donor 3, and donor 4 are PBMC cells from different individuals.
FIG. 9 shows an experiment in which PBMCs are activated to release cytokines in a liquid phase state, wherein FIGs. 9a-9e show concentrations of IL-2, IFN-γ, IL-6, IL-10, and TNF-α, respectively; in the figure, donor 1, donor 2, donor 3, and donor 4 are PBMC cells from different individuals.
FIG. 10 shows an experiment in which the growth of melanoma in mice is inhibited by fusion protein A.

### DETAILED DESCRIPTION

Unless otherwise specified, each of the following terms shall have the meaning described below.

### Definitions

It should be noted that the term "an" entity refers to one or more of the entities. For example, "an antibody" should be interpreted as one or more antibodies. As such, the terms "a" (or "an"), "one or more", and "at least one" can be used interchangeably herein.

As used herein, the term "comprise", "contain", or "include" means that the antibodies, compositions, methods, or the like include the recited elements, such as components or steps, and do not exclude the others. "Consisting essentially of..." means that the antibodies, compositions, methods, or the like exclude other elements that have a fundamental impact on the characteristics of the combination, but do not exclude elements that do not substantially affect the antibodies, compositions, methods, or the like. "Consisting of..." means that elements not specifically recited are excluded.

The term "antibody" as used herein refers to immunoglobulin (Ig) molecules and immunologically active portions of immunoglobulin molecules, i.e., molecules that contain an antigen-binding site that specifically binds to (immunoreacts with) an antigen. Antibodies include, but are not limited to, monoclonal antibodies, chimeric antibodies, dAbs (domain antibodies), single-chain antibodies (scFvs), Fab, Fab' and F(ab')₂ fragments, Fv, and Fab expression libraries.

The antibodies, antigen-binding units, or derivatives disclosed herein include, but are not limited to, polyclonal antibodies, monoclonal antibodies, multispecific antibodies, fully human antibodies, humanized antibodies, primatized antibodies, chimeric antibodies, single-chain antibodies (scFvs), and epitope-binding fragments (e.g., Fab, Fab', and F(ab')₂).

The term "monoclonal antibody" (mAb) refers to a population of antibody molecules that contain only one molecular species of the antibody molecules consisting of a unique light chain gene product and a unique heavy chain gene product. In particular, the complementarity determining regions (CDRs) of the monoclonal antibody are identical in all the molecules of the population. MAbs contain an antigen-binding site capable of immunoreacting with a particular epitope of an antigen.

The term "single-chain antibody" (scFv) refers to an antibody in which its heavy chain variable region (VH) and light chain variable region (VL) are linked by a linker of 15-20 amino acids. The linker may be enriched with glycine to improve flexibility, and enriched with serine or threonine to improve solubility, and may link the N-terminus of VH and the C-terminus of VL, or vice versa. Although the protein has the constant region removed and the linker introduced, it retains the specificity of the original immunoglobulin. ScFv molecules are generally known in the art and are described, for example, in U.S. Pat. No. 5,892,019.

Those skilled in the art will appreciate that the classes of heavy chains include gamma, mu, alpha, delta, or epsilon (γ, µ, α, δ, or ε), and some subclasses (e.g., γ1-γ4). The nature of this chain determines that the "type" of the antibody is IgG, IgM, IgA, IgD, or IgE. Immunoglobulin subclasses (isotypes), such as IgG1, IgG2, IgG3, and IgG4, have been well characterized and the functional specificity imparted is also known. All types of immunoglobulins are within the protection scope of the present disclosure. In one or more embodiments, the type of the immunoglobulin molecule is IgG. Two heavy chains and two light chains are connected in a "Y" configuration through disulfide bonds, wherein the light chain starts at the opening of "Y" configuration and extends through the variable region to surround the heavy chain. Light chains can be classified into kappa (κ) or lambda (λ). Each heavy chain may be connected with a κ or λ light chain. In general, when an immunoglobulin is produced by a hybridoma, a B cell or a genetically engineered host cell, the light and heavy chains are connected by covalent bonds, and the "tail" portions of the two heavy chains are connected by covalent disulfide bonds or non-covalent bonds. In the heavy chains, the amino acid sequence extends from the N terminus at the forked end of the Y configuration to the C terminus at the bottom of each chain. The immunoglobulin κ light chain variable region is V_{κ}; and the immunoglobulin λ light chain variable region is V_{λ}.

The light chain variable region (VL) and the heavy chain variable region (VH) of the antibody determine the antigen recognition and specificity. The light chain constant region (CL) and the heavy chain constant region (CH) impart important biological properties such as secretion, transplacental movement, Fc receptor binding, and complement fixation. By convention, the numbering of amino acids in the constant regions increases as they become further away from the antigen-binding site of the antibody or amino terminus. The N-terminal portion is the variable region, and the C-terminal portion is the constant region; for example, the CH3 and CL domains of IgG1 antibodies comprise the carboxyl termini of the heavy chain and light chain, respectively.

In naturally occurring antibodies, the six "complementarity determining regions" or "CDRs" present in each antigen-binding domain are short, non-contiguous, antigen-specific binding amino acid sequences that form the antigen-binding domain, assuming that the antibody is present in its three-dimensional configuration in an aqueous environment. The remaining amino acids in the antigen-binding domain, referred to as the "framework" ("FR") region, exhibit little intermolecular variability. Most of the framework regions adopt a β-sheet conformation, with the CDRs forming a loop structure connected to, or in some cases forming part of the β-sheet structure. Thus, the framework regions position the CDRs in a correct orientation by interchain non-covalent interactions through forming a scaffold. The antigen-binding domain with the specifically positioned CDRs forms a surface complementary to an epitope on the antigen that facilitates non-covalent binding of the antibody to its antigenic epitope. Generally, in an antibody molecule, the heavy and light chains each have three CDRs, referred to as HCDR1, HCDR2, HCDR3, LCDR1, LCDR2, and LCDR3. In positional order, the heavy chain variable region typically comprises VH FR1, HCDR1, VH FR2, HCDR2, VH FR3, HCDR3, and VH FR4, and the light chain variable region comprises VL FR1, LCDR1, VL FR2, LCDR2, LFR3, LCDR3, and VL FR4. For a given heavy or light chain variable region, amino acids comprising the CDRs and the framework regions may be identified by those of ordinary skill in the art according to known methods (see Kabat, E., et al., U.S. Department of Health and Human Services, Sequences of Proteins of Immunological Interest, (1983), Chothia and Lesk, J. Mol. Biol., 196:901-917 (1987), etc.).

The framework and CDR regions of a humanized antibody need not correspond precisely to the parent sequences, e.g., the donor antibody CDR or the consensus framework may be mutagenized by substitution, insertion, and/or deletion of at least one amino acid residue so that the CDR or framework residue at that site does not correspond to either the donor antibody or the consensus framework. Generally, at least 80%, at least 85%, at least 90%, or at least 95% of the humanized antibody residues will correspond to those of the parent FR and CDR sequences. As used herein, the term "consensus framework" refers to the framework region in the consensus immunoglobulin sequence. As used herein, the term "consensus immunoglobulin sequence" refers to the sequence formed from the most frequently occurring amino acids (or nucleotides) in a family of related immunoglobulin sequences (see, e.g., Winnaker, From Genes to Clones (Verlagsgesellschaft, Weinheim, Germany 1987)). In a family of immunoglobulins, each position in the consensus sequence is occupied by the amino acid occurring most frequently at that position in the family. If two amino acids occur equally frequently, either can be included in the consensus sequence.

Where the terms used and/or accepted in the art have two or more definitions, the definitions of the terms used herein include all of these meanings unless explicitly indicated as opposite. One specific example is the use of the term "complementarity determining regions" ("CDRs") to describe non-contiguous antigen-binding sites found within the variable regions of heavy and light chain polypeptides. This specific region is described in Kabat et al., U.S. Dept. of Health and Human Services, Sequences of Proteins of Immunological Interest (1983) and Chothia et al., J. Mol. Biol. 196:901-917 (1987), which are incorporated herein by reference in their entireties.

Kabat et al. also defines a numbering system applicable to the variable region sequence of any antibody. Those of ordinary skill in the art can apply the "Kabat numbering" system to any variable region sequence without depending on other experimental data beyond the sequence itself. "Kabat numbering" refers to the numbering system proposed by Kabat et al., U.S. Dept. of Health and Human Services in "Sequence of Proteins of Immunological Interest" (1983). EU or Chothia, AbM, Contact, IMGT, and other numbering systems can be also applicable to the antibody.

The antibodies disclosed herein may be derived from any animal, including but not limited to fish, birds, and mammals. Preferably, the antibody is derived from a human, a mouse, a donkey, a rabbit, a goat, a camel, a llama, a horse, or a chicken source. In another embodiment, the variable region may be derived from a condricthoid source (e.g., from a shark).

The "heavy chain constant region" comprises at least one of a CH1 domain, a hinge (e.g., upper, middle, and/or lower hinge region) domain, a CH2 domain, a CH3 domain, or a variant or a fragment. The heavy chain constant region of the antibody may be derived from different immunoglobulin molecules. For example, the heavy chain constant region of the antibody may include a CH1 domain derived from an IgG1 molecule and a hinge region derived from an IgG3 molecule. In another embodiment, the heavy chain constant region may comprise a hinge region derived partially from an IgG1 molecule and partially from an IgG3 molecule. In another embodiment, a portion of the heavy chain may comprise a chimeric hinge region derived partially from an IgG1 molecule and partially from an IgG4 molecule.

"Light chain constant region" includes a part of amino acid sequence from the light chain of an antibody. Preferably, the light chain constant region comprises at least one of a constant κ domain or a constant λ domain. "Light chain-heavy chain pair" refers to a collection of light and heavy chains that can form dimers through disulfide bonds between the CL domain of the light chain and the CH1 domain of the heavy chain.

"Disulfide bond" refers to a covalent bond formed between two sulfur atoms. The thiol group of cysteine can form a disulfide bond or a bridge with a second thiol group. In most naturally occurring IgG molecules, the CH1 and CL regions are linked by a disulfide bond.

"Chimeric antibody" refers to any antibody in which the variable region of the antibody is obtained or derived from a first species, and constant region thereof (which may be intact, partial, or modified) is derived from a second species. In certain embodiments, the variable region is derived from a non-human source (e.g., mouse or primate) and the constant region is derived from a human source.

The term "epitope" as used herein includes any protein determining region that can specifically bind to an immunoglobulin or a fragment thereof or a T cell receptor. The epitope determinant generally consists of chemically active surface groups of molecules (such as amino acids or sugar side chains), and generally has particular three-dimensional structural properties and specific charge properties.

As used herein, the term "specific binding" or "immune response" refers to a non-covalent interaction between an immunoglobulin molecule and one or more antigenic determinants of its target antigen. The strength or affinity of an immunological binding interaction can be expressed in terms of the equilibrium dissociation constant (KD) of the interaction, where a smaller KD represents a greater affinity. The immunological binding properties of the selected polypeptides may be quantified using methods well known in the art. One such method requires the measurement of the rates of antigen-binding site/antigen complex formation and dissociation, where those rates depend on the concentration of the complex partners, the affinity of the interaction, and geometric parameters that affect the rates equally in both directions. Thus, both "association rate constant" (kon) and "dissociation rate constant" (koff) can be determined by calculating the concentration and the actual association and dissociation rates (see Malmqvist, M., Nature 361:186-87 (1993)). The ratio koff/kon is capable of eliminating all the parameters that are independent of affinity and is equal to the equilibrium dissociation constant KD (see Davies et al., (1990) Annual Rev Biochem 59:439-473). Specific binding can be measured by radioligand binding assays, surface plasmon resonance (SPR), flow cytometry binding assay, or similar assays known to those skilled in the art.

The term "isolated" as used herein with respect to cells, nucleic acids, polypeptides, and the like, e.g., "isolated" DNA, RNA, or polypeptides, refers to molecules that are separated from one or more other components, e.g., DNA or RNA, in the natural environment of the cell. The term "isolated" as used herein also refers to nucleic acids or peptides that are substantially free of cellular materials, viral materials or cell culture media when produced by recombinant DNA techniques, or free of chemical precursors or other chemicals when chemically synthesized. In addition, "isolated nucleic acid" is intended to include nucleic acid fragments that do not and will not occur in nature. The term "isolated" is also used herein to refer to cells or polypeptides that are separated from other cellular proteins or tissues. Isolated polypeptides are intended to include both purified and recombinant polypeptides. Isolated polypeptides and the like are usually prepared by at least one purification step. In one or more embodiments, the purity of the isolated nucleic acids, polypeptides, and the like is at least about 50%, about 60%, about 70%, about 80%, about 90%, about 95%, about 99%, or a range between any two of these values (inclusive) or any value therein.

The term "encoding" as applied to a polynucleotide refers to a polynucleotide known to "encode" a polypeptide. When in its native state or manipulated by methods well known to those skilled in the art, the polynucleotide can be transcribed and/or translated to produce the polypeptide and/or a fragment thereof.

The term "recombinant", with regard to a polypeptide or polynucleotide, is intended to refer to a polypeptide or polynucleotide that does not occur in nature, and non-limiting examples can be combined to produce a polynucleotide or polypeptide that does not normally occur.

"Amino acid" refers to an organic compound containing both an amino group and a carboxyl group, such as an α-amino acid and a β-amino acid, that can be encoded by a nucleic acid, either directly or in the form of a precursor. A single amino acid is encoded by a nucleic acid consisting of three nucleotides (so-called codon or base triplet). Each amino acid is encoded by at least one codon. The fact that an amino acid is encoded by different codons is known as "codon degeneracy". Amino acids include natural amino acids and non-natural amino acids.

As used herein, the twenty conventional amino acids and their abbreviations follow conventional usage. See Immunology-A Synthesis (second edition, Eds. E. S. Golub and D. R. Gren, Sinauer Associates, Sunderland Mass. (1991)). Stereoisomers (e.g., D-amino acids) of the twenty conventional amino acids, unnatural amino acids (such as α- or α-disubstituted amino acids), N-alkyl amino acids, lactic acid, and other unconventional amino acids can also be suitable components for the polypeptides of the present disclosure. Examples of unconventional amino acids include: 4-hydroxyproline, γ-carboxyglutamate, ε-N,N,N-trimethyllysine, ε-N-acetyllysine, O-phosphoserine, N-acetylserine, N-formylmethionine, 3-methylhistidine, 5-hydroxylysine, σ-N-methylarginine, and other similar amino acids and imino acids (e.g., 4-hydroxyproline). In the polypeptide notation used herein, the left-hand direction is the amino-terminal direction and the right-hand direction is the carboxy-terminal direction, in accordance with standard usage and convention. The conventional (or natural) amino acids include alanine (three-letter code: Ala, one-letter code: A), arginine (Arg, R), asparagine (Asn, N), aspartic acid (Asp, D), cysteine (Cys, C), glutamine (Gln, Q), glutamic acid (Glu, E), glycine (Gly, G), histidine (His, H), isoleucine (Ile, I), leucine (Leu, L), lysine (Lys, K), methionine (Met, M), phenylalanine (Phe, F), proline (Pro, P), serine (Ser, S), threonine (Thr, T), tryptophan (Trp, W), tyrosine (Tyr, Y), valine (Val, V), and the like.

The term "polypeptide" is intended to encompass both the singular form "polypeptide" and the plural form "polypeptides", and refers to a molecule consisting of amino acid monomers linearly linked by amide bonds (also known as peptide bonds). The term "polypeptide" refers to any single chain or multiple chains of two or more amino acids and does not refer to a particular length of the product. Thus, included within the definition of "polypeptide" are peptides, dipeptides, tripeptides, oligopeptides, "proteins", "amino acid chains", or any other term used to refer to chains of two or more amino acids, and the term "polypeptide" may be used in place of, or interchangeably with, any of the above terms. The term "polypeptide" is also intended to refer to a product of post-expression polypeptide modification, including but not limited to glycosylation, acetylation, phosphorylation, amidation, derivatization by known protecting/blocking groups, proteolytic cleavage, or non-naturally occurring amino acid modifications. The polypeptide may be derived from a natural biological source or produced by recombinant techniques, but it is not necessarily translated from a specified nucleic acid sequence, and it may be produced in any manner, including chemical synthesis.

The terms "polynucleotide" and "oligonucleotide" are used interchangeably to refer to a polymeric form of nucleotides of any length, whether deoxyribonucleotides or ribonucleotides or analogs thereof. A polynucleotide consists of a specific sequence of four bases: adenine (A), cytosine (C), guanine (G), and thymine (T)/uracil (U, instead of thymine when the polynucleotide is RNA). A "polynucleotide sequence" can be a letter representation of a polynucleotide molecule. The letter representation can be input into a database in a computer with a central processing unit and used for bioinformatics applications, such as for functional genomics and homology searches. The polynucleotide may have any three-dimensional structure and may perform any function, known or unknown. The following are non-limiting examples of polynucleotides: genes or gene fragments (such as probes, primers, and EST or SAGE tags), exons, introns, messenger RNA (mRNA), transfer RNA, ribosomal RNA, ribozymes, cDNA, dsRNA, siRNA, miRNA, recombinant polynucleotides, branched polynucleotides, plasmids, vectors, DNA, RNA, and nucleic acid probes and primers. Polynucleotides can include modified nucleotides, such as methylated nucleotides and nucleotide analogs. If present, structural modifications to the nucleotide can be made before or after the assembly of the polynucleotide. The sequence of nucleotides can be interrupted by non-nucleotide components. The polynucleotide may be further modified after polymerization, such as by conjugation with a labeling component. This term also refers to double-stranded and single-stranded molecules. Unless otherwise indicated or required, examples of any polynucleotide disclosed herein include a double-stranded form and each of the two complementary single-stranded forms known or predicted to constitute the double-stranded form.

A polynucleotide or a polynucleotide sequence (or a polypeptide or an antibody sequence) having a certain percentage (e.g., 90%, 95%, 98%, or 99%) of "identity or sequence identity" to another sequence means that when the sequences are aligned, the percentage of bases (or amino acids) in the two compared sequences are the same. This alignment and identity percentage or sequence identity can be determined using visual inspection or software programs known in the art, such as the software programs described in Ausubel et al. eds., (2007), Current Protocols in Molecular Biology. Preferably, the alignment is performed using default parameters. One alignment program is BLAST using default parameters, such as BLASTN and BLASTP, both using the following default parameters: Genetic code = standard; filter = none; strand = both; cutoff = 60; expect = 10; Matrix = BLOSUM62; Descriptions = 50 sequences; sort by = HIGHSCORE; Databases = non-redundant; GenBank + EMBL + DDBJ + PDB + GenBank CDS translations + Swiss Protein + SPupdate + PIR. Biologically equivalent polynucleotides are polynucleotides having the above specified percentage identity and encoding polypeptides having identical or similar biological activity.

Minor variations in the amino acid sequences of antibodies or immunoglobulin molecules are contemplated by the present disclosure, provided that the identity of the amino acid sequences is maintained to be at least 90%, such as at least 92%, 95%, 98%, or 99%. In some embodiments, the variations are conservative amino acid substitutions. Conservative amino acid substitutions are those that occur within a family of amino acids that are related in their side chains. Genetically encoded amino acids are generally classified into the following categories: (1) acidic amino acids: aspartate, and glutamate; (2) basic amino acids: lysine, arginine, and histidine; (3) non-polar amino acids: alanine, valine, leucine, isoleucine, proline, phenylalanine, methionine, and tryptophan; and (4) uncharged polar amino acids: glycine, asparagine, glutamine, cysteine, serine, threonine, and tyrosine. Amino acids of the other families include (i) serine and threonine of the aliphatic-hydroxy family; (ii) asparagine and glutamine of the amide-containing family; (iii) alanine, valine, leucine, and isoleucine of the aliphatic family; and (iv) phenylalanine, tryptophan, and tyrosine of the aromatic family. In some embodiments, conservative amino acid substitution groups are valine-leucine-isoleucine, phenylalanine-tyrosine, lysine-arginine, alanine-valine, glutamic acid-aspartic acid, and asparagine-glutamine. For example, it is reasonable to expect that the single replacement of leucine with isoleucine or valine, aspartate with glutamate, threonine with serine, or the similar replacement of an amino acid with a structurally related amino acid, will not have a major effect on the binding or properties of the resulting molecule, particularly when the replacement does not involve an amino acid within a binding site. Whether an amino acid change results in a functional peptide can be readily determined by measuring the specific activity of the polypeptide derivative. The measurement is described in detail herein. Fragments or analogs of antibodies or immunoglobulin molecules can be readily prepared by those of ordinary skill in the art.

In some embodiments, the amino acid substitutions have the following effects: (1) reducing susceptibility to proteolysis, (2) reducing susceptibility to oxidation, (3) altering the binding affinity for formation of protein complexes, (4) altering binding affinity, and (5) conferring or improving other physicochemical or functional properties of such analogs. Analogs can include various mutant proteins with sequences different from those of the naturally occurring peptide sequences. For example, single or multiple amino acid substitutions (preferably conservative amino acid substitutions) may be made in the naturally occurring sequence (preferably in a portion of the polypeptide outside the domains that form intermolecular contacts). A conservative amino acid substitution should not significantly alter the structural characteristics of the parent sequence (e.g., an amino acid for the replacement should not tend to disrupt a helix that occurs in the parent sequence, or disrupt other types of secondary structures that characterize the parent sequence). Examples of secondary and tertiary structures of artificially recognized polypeptides are described in Proteins, Structures and Molecular Principles (Ed. Creighton, W. H. Freeman and Company, New York (1984)); Introduction to Protein Structure (Eds. C.Branden and J.Tooze, Garland Publishing, New York, N.Y.(1991)); and Thornton et al., Nature 354:105 (1991).

The number of amino acids of conservative amino acid substitutions of VL or VH may be about 1, about 2, about 3, about 4, about 5, about 6, about 8, about 9, about 10, about 11, about 13, about 14, about 15 conservative amino acid substitutions, or a range between any two of these values (inclusive) or any value therein. The number of amino acids of conservative amino acid substitutions of a heavy chain constant region, a light chain constant region, a heavy chain, or a light chain may be about 1, about 2, about 3, about 4, about 5, about 6, about 8, about 9, about 10, about 11, about 13, about 14, about 15, about 18, about 19, about 22, about 24, about 25, about 29, about 31, about 35, about 38, about 41, about 45 conservative amino acid substitutions, or a range between any two of these values (inclusive) or any value therein.

As used herein, the term "label" or "labeled" refers to incorporation of a detectable marker, e.g., by incorporation of a radiolabeled amino acid or attachment to a polypeptide of biotinyl moieties that can be detected by marked avidin (e.g., streptavidin containing a fluorescent marker or having enzymatic activity that can be detected by optical or calorimetric methods). In certain instances, the label or labels may also be therapeutic. Various methods for labeling polypeptides and glycoproteins are known in the art and can be used. Examples of labels for polypeptides include, but are not limited to: radioisotopes or radionuclides (e.g., ³H, ¹⁴C, ¹⁵N, ³⁵S, ⁹⁰Y, ⁹⁹Tc, ¹¹¹In, ¹²⁵I, and ¹³¹I), fluorescent labels (e.g., FITC, rhodamine, and lanthanide phosphor), enzymatic labels (e.g., horseradish peroxidase, β-galactosidase, luciferase, and alkaline phosphatase), chemiluminescent labels, biotinyl groups, and predetermined polypeptide epitopes recognized by secondary reporter genes (e.g., leucine zipper pair sequences, secondary antibody-binding sites, metal binding domains, and epitope tags). In some embodiments, the labels are connected by spacer arms of various lengths to reduce potential steric hindrance. The term "medicament" or "drug" refers to a compound or composition that is capable of inducing the desired therapeutic effect when properly administered to a patient.

"About" refers to a general error range for corresponding values as readily understood by those skilled in the relevant art. In some embodiments, the term "about" mentioned herein refers to the values described and a range of ±10%, ±5%, or ±1% thereof.

"EC₅₀", i.e., concentration for 50% of maximal effect, refers to a concentration that causes 50% of maximal effect.

"Treatment" refers to both therapeutic treatment and prophylactic or preventative measures, the purpose of which is to prevent, slow down, ameliorate, or halt undesirable physiological changes or disorders, such as the progression of a disease, including, but not limited to, alleviation of symptoms, diminishment of extent of disease, stabilized (i.e., not worsening) state of disease, delay or slowing of disease progression, amelioration, palliation, alleviation, or elimination (whether partial or total) of state of disease, prolongation of life expectancy in the absence of treatment, and the like, as either detectable or undetectable. Patients in need of treatment include patients already with a condition or disorder, patients susceptible to a condition or disorder, or patients in need of prevention of the condition or disorder, or patients who may or are expected to benefit from administration of the antibody or the pharmaceutical composition disclosed herein for detection, diagnostic procedures, and/or treatment.

The term "tumor" means or is intended to describe the physiological state of a mammal, which is typically characterized by uncontrolled cell growth, including benign tumors and malignant tumors such as cancer. Examples of cancers include, but are not limited to, carcinoma, lymphoma, blastoma, sarcoma or leukemia. More specific examples of such cancers include, but are not limited to, colorectal cancer, lung cancer, ovarian cancer, uterine cancer, endometrial cancer, colon cancer, salivary gland cancer, peritoneal cancer, fallopian tube cancer, pancreatic cancer, thyroid cancer, head and neck squamous cell carcinoma, nasopharyngeal cancer, laryngeal cancer, lung adenocarcinoma, lung squamous cell carcinoma, liver cancer, hepatocellular carcinoma, gastrointestinal cancer, glioblastoma, breast cancer, brain cancer, renal cancer, renal cell carcinoma, rectal cancer, prostate cancer, vulval cancer, testicular cancer, squamous cell carcinoma, small cell lung cancer, cervical cancer, bladder cancer, retinoblastoma, glioblastoma, mesothelioma, oral epithelioid cancer, choriocarcinoma, and head and neck cancer.

As used herein, the terms "give" and "administer" are used interchangeably and refer to the delivery of a substance (e.g., antibody or fusion protein) for therapeutic purposes (e.g., treating a disease associated with PD-L1 or IL-15). The mode of administration may be parenteral, enteral, and topical. Parenteral administration is typically performed by injection, including but not limited to, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, and intrasternal injection and infusion.

As used herein, the term "effective amount" or "therapeutically effective amount" refers to the amount of a drug, e.g., an antibody or fusion protein, that is sufficient to reduce or ameliorate the severity and/or duration of a disorder (e.g., cancer) or one or more symptoms thereof, prevent the progression of a disorder, cause regression of a disorder, prevent the recurrence, development, onset or progression of one or more symptoms associated with a disorder, detect a disorder, or enhance or improve the prophylactic or therapeutic effect of another therapy (e.g., a prophylactic or therapeutic agent). For example, the effective amount of an antibody or fusion protein may inhibit tumor growth (e.g., inhibit an increase in tumor volume), decrease tumor growth (e.g., decrease tumor volume), reduce the number of cancer cells, and/or relieve to some extent one or more of the symptoms associated with the cancer. For example, the effective amount may improve disease free survival (DFS), improve overall survival (OS), or decrease likelihood of recurrence.

The term "patient" refers to any mammal in need of diagnosis, prognosis or treatment, including, but not limited to, humans, dogs, cats, guinea pigs, rabbits, rats, mice, horses, cattle, and the like.

As used herein, the term "in need" means that the patient has been identified as in need of a particular method or treatment. In some embodiments, identification may be made by any diagnostic mode. The patient may be in need of any methods and treatments described herein.

The term "drug for treating a tumor" as used herein refers to an agent having the functional property of inhibiting in a human the development or progression of a tumor, particularly a malignant (cancerous) lesion, such as cancer, sarcoma, lymphoma, or leukemia. Inhibition of metastasis is a property of anti-tumor drugs in many cases.

The "pharmaceutical composition" refers to a mixture of one or more compounds or pharmaceutically acceptable salts or prodrugs thereof, and an additional chemical component, wherein the "additional chemical component" refers to a pharmaceutically acceptable excipient and/or one or more additional therapeutic agents.

The relevant description of publications mentioned herein is incorporated by reference in its entirety.

### Anti-PD-L1 Antibody and Fusion Protein

The present disclosure provides an antibody or an antigen-binding fragment having high affinity for PD-L1 protein. The antibodies exhibit potent binding activity and biological activity, and can be used for therapeutic and diagnostic purposes. For example, these antibodies or antigen-binding fragments can effectively block inhibitory immune checkpoints, activate lymphocytes to release cytokines, and can be used for treating various types of cancers, tumors or infections, and other related diseases.

In some embodiments, the antibody of the present disclosure uses the "Knobs-into-Holes" technique (see, e.g., John B. B. Ridgway et al., 'Knobs-into-holes' engineering of antibody CH3 domains for heavy chain heterodimerization, Protein Engineering, 9(7): p.617-21 (1996); patent US8216805B2). This technique enables the modification of the interfaces between different chains of the antibody, thus facilitating the correct association of the chains of the antibody. Generally, this technique involves introducing a "protuberance" ("knob") at the interface of one chain and introducing a corresponding "cavity"("hole") at the interface of the other chain to be paired with, such that the protuberance can be placed at the cavity. The protuberance can be constructed by replacing amino acid side chains at an interface of the CH3 domain from the heavy chain constant domains of one chain with relatively large side chains (e.g., amino acid replacement T366W (Eu numbering)). The compensating cavity of the same size as, or a similar size to, the protuberance is constructed at an interface of the CH3 domain from the heavy chain constant domains of the other chain to be paired with by replacing large amino acid side chains with relatively small side chains (e.g., amino acid replacements T366S, L368A, and Y407V (Eu numbering)).

In some embodiments, the constant region of one heavy chain of the antibody comprises the following amino acid mutations: Y349C, T366S, L368A, and Y407V (EU numbering), and the constant region of the other heavy chain of the antibody comprises the following amino acid mutations: S354C and T366W (EU numbering), forming a stable association of "Knobs-into-Holes".

It will also be appreciated by those of ordinary skill in the art that the sequence of the antibody or the antigen-binding fragment disclosed herein may be replaced, and the replaced amino acid sequence differs from the naturally occurring amino acid sequence of the antibody. For example, the replaced amino acid sequence can be similar to the starting sequence, for example, having a certain proportion of identity to the starting sequence. For example, the identity to the starting sequence may be about 80%, about 85%, about 90%, about 95%, about 98%, about 99%, or a range between any two of these values (inclusive) or any value therein.

In some embodiments, the antibody or the antigen-binding fragment comprises an amino acid sequence having one or more modification groups. For example, the antibody or the antigen-binding fragment of the present disclosure may comprise a flexible linker sequence, or may be modified to add a functional group (e.g., PEG, a drug, a toxin, or a tag).

The antibody, the antigen-binding fragment, and the fusion protein thereof of the present disclosure include modified derivatives, i.e., modified by covalent linking of any type of molecules to the antibody or the antigen-binding fragment or the fusion protein thereof, wherein the covalent linking does not prevent the antibody or the antigen-binding fragment or the fusion protein thereof from binding to an epitope. The following examples are included but not by way of limitation, wherein an antibody or an antigen-binding fragment or a fusion protein thereof may be modified by glycosylation, acetylation, pegylation, phosphorylation, amidation, derivatization by known protecting/blocking groups, proteolytic cleavage, attachment to cellular ligands or other proteins, or the like. Any of a number of chemical modifications may be made by techniques in the prior art, including but not limited to specific chemical cleavage, acetylation, formylation, metabolic synthesis of tunicamycin, and the like.

In some embodiments, the antibody or the antigen-binding fragment may be conjugated with a therapeutic agent, a prodrug, a peptide, a protein, an enzyme, a virus, a lipid, a biological response modifier, an agent, or PEG.

The antibody or the antigen-binding fragment may be detectably labeled by coupling to a chemiluminescent compound. The presence of the chemiluminescent-labeled antibody or antigen-binding fragment is then determined by detecting the luminescence that occurs during the chemical reaction. Examples of chemiluminescent-labeled compounds include luminol, isoluminol, aromatic acridinium esters, imidazole, acridinium salts, and oxalate esters.

In some embodiments, to facilitate the expression of the antibody in a host cell, signal peptide sequences may be further added to the heavy and light chains of the antibody, for example, a heavy chain signal peptide: MEFGLSWVFLVAILKGVQC (SEQ ID NO: 75) and a light chain signal peptide: MDMRVLAQLLGLLLLCFPGARC (SEQ ID NO: 76).

In some embodiments, the present disclosure further provides a fusion protein comprising a PD-L1-binding domain and a domain that stimulates the activity of NK and T cells. In some embodiments, the PD-L1-binding domain is an anti-PD-L1 antibody or an antigen-binding fragment. In some embodiments, the domain that stimulates NK and T cell activity comprises IL-15 or a receptor-binding fragment thereof or a variant thereof and IL-15Rα or a sushi domain thereof or a variant thereof. The sushi domain binds to IL-15 with high affinity, and the complex of IL-15 with the sushi domain has relatively high activity for stimulating NK and T cell proliferation.

In some embodiments, the fusion protein comprises the anti-PD-L1 antibody or the antigen-binding fragment, IL-15 or a receptor-binding fragment thereof or a variant thereof, and IL-15Rα or a sushi domain thereof or a variant thereof described herein.

In some embodiments, the present disclosure provides a fusion protein comprising the anti-PD-L1 antibody or the antigen-binding fragment, IL-15, and IL-15Rα described herein.

In some embodiments, the present disclosure provides a fusion protein comprising the anti-PD-L1 antibody or the antigen-binding fragment, IL-15, and the sushi domain of IL-15Rα described herein.

In some embodiments, the present disclosure provides a fusion protein comprising the anti-PD-L1 antibody or the antigen-binding fragment, an IL-15 receptor-binding fragment, and the sushi domain of IL-15Rα described herein.

In some embodiments, the present disclosure provides a fusion protein comprising the anti-PD-L1 antibody or the antigen-binding fragment, a variant of IL-15 or a receptor-binding fragment thereof, and a variant of IL-15Rα or the sushi domain thereof described herein.

In some embodiments, the IL-15 comprises the amino acid sequence set forth in SEQ ID NO: 82, or an amino acid sequence having at least 90%, at least 91%, at least 92%, at least 93%, at least 94%, at least 95%, at least 96%, at least 97%, at least 98%, or at least 99% identity to the sequence set forth in SEQ ID NO: 82, or an amino acid sequence having one or more conservative amino acid substitutions as compared to the sequence set forth in SEQ ID NO: 82.

In some embodiments, the IL-15Rα sushi domain comprises the amino acid sequence set forth in SEQ ID NO: 81, or an amino acid sequence having at least 90%, at least 91%, at least 92%, at least 93%, at least 94%, at least 95%, at least 96%, at least 97%, at least 98%, or at least 99% identity to the sequence set forth in SEQ ID NO: 81, or an amino acid sequence having one or more conservative amino acid substitutions as compared to the sequence set forth in SEQ ID NO: 81.

In some embodiments, the variant of IL-15 or a receptor-binding fragment thereof comprises an amino acid sequence having at least 90%, at least 91%, at least 92%, at least 93%, at least 94%, at least 95%, at least 96%, at least 97%, at least 98%, or at least 99% identity to IL-15 or the receptor-binding fragment thereof.

In some embodiments, the variant of IL-15 or the receptor-binding fragment thereof has one or more conservative amino acid substitutions as compared to IL-15 or the receptor-binding fragment thereof.

In some embodiments, the variant of IL-15Rα or the sushi domain thereof comprises an amino acid sequence having at least 90%, at least 91%, at least 92%, at least 93%, at least 94%, at least 95%, at least 96%, at least 97%, at least 98%, or at least 99% identity to IL-15Rα or the sushi domain thereof.

In some embodiments, the variant of IL-15Rα or the sushi domain thereof has one or more conservative amino acid substitutions as compared to IL-15Rα or the sushi domain thereof.

In some embodiments, the anti-PD-L1 antibody or the antigen-binding fragment of the fusion protein is linked to IL-15 or the receptor-binding fragment thereof or the variant thereof and IL-15Rα or the sushi domain thereof or the variant thereof via a linker. In some embodiments, the C-terminus of one heavy chain of the anti-PD-L1 antibody is linked to IL-15 or the receptor-binding fragment thereof or the variant thereof via a linker, and the C-terminus of the other heavy chain of the anti-PD-L1 antibody is linked to IL-15Rα or the sushi domain thereof or the variant thereof via a linker. In some embodiments, the C-terminus of one heavy chain of the anti-PD-L1 antibody is linked to IL-15 via a linker, and the C-terminus of the other heavy chain of the anti-PD-L1 antibody is linked to the sushi domain of IL-15Rα via a linker. In some embodiments, the constant region of one heavy chain of the anti-PD-L1 antibody comprises the following amino acid mutations: Y349C, T366S, L368A, and Y407V (EU numbering), and the constant region of the other heavy chain of the anti-PD-L1 antibody comprises the following amino acid mutations: S354C and T366W (EU numbering), forming a stable association of "Knobs-into-Holes", which greatly promotes the correct assembly of the two heavy chains and minimizes mismatches.

In some embodiments, the linker comprises glycine and serine ("GS linker"). In some embodiments, the linker is (GₘS)ₙ, wherein each m is independently 1, 2, 3, 4, 5, or 6, and n is 1, 2, 3, 4, or 5. In some embodiments, the linker is GGGGS. In some embodiments, the linker is (GGGGS)₂. In some embodiments, the linker is (GGGGS)₃, as set forth in SEQ ID NO: 85. In some embodiments, the linker is (GGGGS)₄. In some embodiments, the linker is (GGGGS)₅.

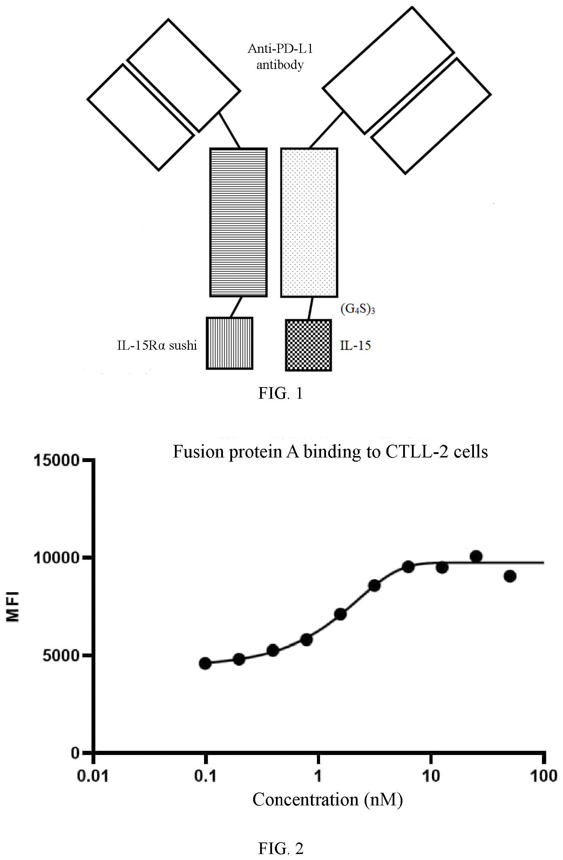

In some embodiments, the fusion protein of the present disclosure comprises a first polypeptide, a second polypeptide, and a third polypeptide. The first polypeptide comprises an anti-PD-L1 heavy chain a, a linker, and IL-15Rα or a sushi domain thereof or a variant thereof. The second polypeptide comprises an anti-PD-L1 heavy chain b, a linker, and IL-15 or a receptor-binding fragment thereof or a variant thereof. The third polypeptide is an anti-PD-L1 light chain. In some embodiments, the schematic structural diagram of the fusion protein is shown in FIG. 1, which consists of 4 polypeptides, including one first polypeptide, one second polypeptide, and two third polypeptides with identical sequences.

In some embodiments, the first polypeptide comprises the amino acid sequence set forth in SEQ ID NO: 83, or an amino acid sequence having at least 90% identity to the sequence set forth in SEQ ID NO: 83, or an amino acid sequence having one or more conservative amino acid substitutions as compared to the sequence set forth in SEQ ID NO: 83.

In some embodiments, the second polypeptide comprises the amino acid sequence set forth in SEQ ID NO: 84, or an amino acid sequence having at least 90% identity to the sequence set forth in SEQ ID NO: 84, or an amino acid sequence having one or more conservative amino acid substitutions as compared to the sequence set forth in SEQ ID NO: 84.

In some embodiments, the third polypeptide comprises the amino acid sequence set forth in SEQ ID NO: 66, or an amino acid sequence having at least 90% identity to the sequence set forth in SEQ ID NO: 66, or an amino acid sequence having one or more conservative amino acid substitutions as compared to the sequence set forth in SEQ ID NO: 66.

In some embodiments, the first polypeptide comprises the amino acid sequence set forth in SEQ ID NO: 83, the second polypeptide comprises the amino acid sequence set forth in SEQ ID NO: 84, and the third polypeptide comprises the amino acid sequence set forth in SEQ ID NO: 66.

In some embodiments, the fusion protein is a fusion protein A.

### Preparation Method for Antibody and Fusion Protein

The present disclosure further discloses a polynucleotide or a nucleic acid molecule encoding the antibody, the antigen-binding fragment, the fusion protein, and a derivative thereof of the present disclosure. The polynucleotide disclosed herein may encode a heavy chain variable region, a light chain variable region, an Fc region, a portion of a heavy chain variable region, a portion of a light chain variable region, a heavy chain, a light chain, a fusion protein, or the like. The methods for preparing the antibody and the fusion protein are well known in the art and are described herein.

In certain embodiments, the prepared antibody does not elicit a deleterious immune response in the animal (e.g., human) to be treated. In some embodiments, the antibody, the antigen-binding fragment, or the derivative disclosed herein is modified using techniques well known in the art to reduce the immunogenicity. For example, the antibody can be humanized, primatized or deimmunized, or a chimeric antibody can be prepared. Such antibodies are derived from non-human antibodies, typically murine or primate antibodies, which retain or substantially retain the antigen-binding properties of the parent antibody but are less immunogenic in humans. This can be accomplished by a variety of methods, including: (a) grafting an entire variable region of a non-human origin to a constant region of a human origin to produce a chimeric antibody; (b) grafting at least a portion of one or more non-human complementarity determining regions (CDRs) to framework regions and a constant region of a human origin, with or without retention of critical framework residues; or (c) grafting an entire variable region of a non-human origin, but "hiding" the surface residues by replacing them with portions of a human-like origin. Generally, framework residues in the human framework regions will be substituted with corresponding residues from the CDR donor antibody, preferably residues that may improve the antigen binding. Such framework substitutions can be identified by methods well known in the art, for example, by modeling the interaction of the CDR and framework residues to identify framework residues that play an important role in antigen binding and by sequence alignment to identify abnormal framework residues at particular positions (see U.S. Pat. No. 5,585,089, which is incorporated herein by reference in its entirety). Antibodies can be humanized using a variety of techniques well known in the art, such as CDR grafting (WO1991009967; U.S. Pat. Nos. 5,225,539, 5,530,101, and 5,585,089), repair or surface rearrangement (EP 592,106; EP 519,596), and chain rearrangement (U.S. Pat. No. 5,565,332), which are incorporated herein by reference in their entireties.

Deimmunization may also be used to reduce the immunogenicity of the antibody. In the present disclosure, the term "deimmunization" includes the alteration of the antibody to modify T cell epitopes (see, e.g., WO2000034317 A2). For example, a heavy chain variable region sequence and a light chain variable region sequence from the original antibody are analyzed, and a "map" of human T cell epitopes from each variable region is generated, showing the positions of the epitopes relative to the complementarity determining regions (CDRs) and other critical residues within the sequence. Individual T cell epitopes from the T cell epitope map are analyzed to identify alternative amino acid substitutions with a lower risk of altering antibody activity. A series of alternative heavy chain variable region sequences and light chain variable region sequences comprising combinations of amino acid substitutions are designed and subsequently incorporated into a series of binding polypeptides. The genes of intact heavy and light chains comprising the modified variable regions and human constant regions are cloned into an expression vector, and the plasmid is then transferred into a cell line to produce an intact antibody. The antibodies are compared in appropriate biochemical and biological experiments to identify the optimal antibody.

The binding specificity of the antibody or the antigen-binding fragment of the present disclosure can be detected by an *in vitro* assay, such as co-immunoprecipitation, radioimmunoassay (RIA) or enzyme-linked immunosorbent assay (ELISA).

For the preparation of scFv, reference can be made to the technology for the production of single-chain units (U.S. Pat. No. 4,946,778). Single chain fusion peptides are produced by amino acid bridging the heavy and light chain fragments of the Fv region to form a single chain unit. The technique for assembling a functional Fv fragment in *E. coli* (Skerra et al., Science 240:1038-1041 (1988)) can also be used.

Examples of techniques that may be used to produce single chain Fvs (scFvs) and antibodies include those described in U.S. Pat. Nos. 4,946,778 and 5,258,498. For certain use, including *in vivo* use of antibodies in humans and *in vitro* detection assays, a chimeric antibody, a humanized antibody or a fully human antibody may be used. Chimeric antibodies are molecules in which different portions of the antibody are derived from different animal species, such as antibodies having variable regions of a murine monoclonal antibody and constant regions of a human immunoglobulin. Methods for producing chimeric antibodies are known in the art. See U.S. Pat. Nos. 5,807,715, 4,816,567, and 4,816,397, which are incorporated herein by reference in their entireties.

In addition, another efficient method for producing recombinant antibodies, which in particular is capable of producing primate antibodies comprising monkey variable region and human constant region sequences, is disclosed in Newman, Biotechnology 10:1455-1460 (1992), which is incorporated herein by reference in its entirety. Furthermore, this technique is also described in U.S. Pat. Nos. 5,658,570, 5,693,780, and 5,756,096, each of which is incorporated herein by reference in its entirety.

The antibodies can be prepared by a variety of methods known in the art, including phage display methods using antibody libraries from immunoglobulin sequences. Reference may also be made to U.S. Pat. Nos. 4,444,887 and 4,716,111, and PCT Publication Nos. WO 1998050433, WO 1998024893, WO 1998016654, WO 1996034096, WO 1996033735, and WO 1991010741, each of which is incorporated herein by reference in its entirety.

In some other embodiments, DNA encoding the desired monoclonal antibody can be isolated and sequenced using conventional methods (e.g., using oligonucleotide probes that can specifically bind to genes encoding the heavy and light chains of an antibody). Isolated and subcloned hybridoma cells can be sources of such DNA. Once isolated, the DNA can be inserted into an expression vector and then transfected into prokaryotic or eukaryotic host cells such as *E. coli* cells, simian COS cells, Chinese hamster ovary (CHO) cells, or myeloma cells that do not produce other immunoglobulins. The isolated DNA (which may be synthetic as described herein) can also be used to prepare sequences of the constant and variable regions of an antibody, as described in U.S. Pat. No. 5,658,570, which is incorporated herein by reference in its entirety. This method extracts RNA from the selected cells and converts it into cDNA, followed by amplification by PCR using Ig-specific primers. Suitable probes for this purpose are also described in U.S. Pat. No. 5,658,570.

In addition, by using conventional recombinant DNA techniques, one or more CDRs of the antibody of the present disclosure can be inserted into framework regions, e.g., into human framework regions to construct a humanized non-fully human antibody. The framework regions may be naturally occurring or consensus framework regions, preferably human framework regions (see Chothia et al., J. Mol. Biol. 278:457-479 (1998), in which a range of human framework regions are listed). Some polynucleotides may encode antibodies produced by the combination of framework regions and CDRs that specifically bind to at least one epitope of an antigen of interest. One or more amino acid substitutions may be made within the framework regions, and amino acid substitutions capable of improving the binding of the antibody to the antigen thereof may be selected. Additionally, substitution or deletion of one or more cysteine residues in the variable regions involved in interchain disulfide bond formation can be made in this manner, thereby producing an antibody molecule lacking one or more interchain disulfide bonds. Other alterations to the polynucleotides made within the technology scope of the art are also encompassed by the present disclosure.

Antibodies or fusion proteins can be prepared by using conventional recombinant DNA techniques. Vectors and cell lines for antibody or fusion protein production can be selected, constructed, and cultured using techniques well known to those skilled in the art. These techniques are described in various laboratory manuals and main publications, such as Recombinant DNA Technology for Production of Protein Therapeutics in Cultured Mammalian Cells, D. L. Hacker, F. M. Wurm, Reference Module in Life Sciences, 2017, the content of which is incorporated herein by reference in its entirety (including the supplements).

In some embodiments, the DNA encoding the antibody or the fusion protein can be designed and synthesized according to the amino acid sequence of the antibody or the fusion protein described herein by conventional methods and inserted into an expression vector, and a host cell is then transfected with the expression vector. The transfected host cell is cultured in a culture medium to produce the monoclonal antibody or the fusion protein. In some embodiments, the vector expressing the antibody or fusion protein comprises at least one promoter element, an antibody or fusion protein encoding sequence, a transcription termination signal, and a polyA tail. Other elements include enhancers, Kozak sequences, and donor and recipient sites flanking the inserted sequence for RNA splicing. Efficient transcription can be obtained by early and late promoters of SV40, long terminal repeats from retroviruses such as RSV, HTLV1, and HIVI, and early promoters of cytomegalovirus, and promoters from other cells such as actin promoter can also be used. Suitable expression vectors may include pIRES1neo, pRetro-Off, pRetro-On, pLXSN, pLNCX, pcDNA3.1(+/-), pcDNA/Zeo(+/-), pcDNA3.1/Hygro(+/-), pSVL, pMSG, pRSVcat, pSV2dhfr, pBC12MI, pCS2, or the like. Commonly used mammalian cells include HEK293 cells, Cos1 cells, Cos7 cells, CV1 cells, murine L cells, CHO cells, and the like.

In some embodiments, the inserted gene fragment should comprise a screening label, common ones of which include screening genes such as dihydrofolate reductase, glutamine synthetase, neomycin resistance, and hygromycin resistance, to facilitate the screening and separation of cells that have been successfully transfected. The constructed plasmid is transfected to a host cell without the above genes, and the successfully transfected cells are cultured in a large quantity in a selective culture medium to produce the desired target protein.

In addition, mutations can be introduced in the nucleotide sequence encoding the antibody or fusion protein of the present disclosure using standard techniques known to those skilled in the art, including but not limited to site-directed mutations resulting in amino acid substitutions and PCR-mediated mutations. The variant (including derivative) encodes a protein having a substitution of less than 50 amino acids, a substitution of less than 40 amino acids, a substitution of less than 30 amino acids, a substitution of less than 25 amino acids, a substitution of less than 20 amino acids, a substitution of less than 15 amino acids, a substitution of less than 10 amino acids, a substitution of less than 5 amino acids, a substitution of less than 4 amino acids, a substitution of less than 3 amino acids, or a substitution of less than 2 amino acids relative to the original target protein. Alternatively, mutations can be introduced randomly along all or part of the coding sequence, for example by saturation mutagenesis, and the resulting mutants can be screened for the biological activity to identify mutants that retain the activity.

### Therapeutic Method

The present disclosure further provides a treatment method and use. In some embodiments, provided is a method for preventing, treating, or ameliorating various types of autoimmune diseases, cancers, tumors or infections, and other related diseases, which comprises administering to a patient an effective amount of an anti-PD-L1 antibody or an antigen-binding fragment or a fusion protein. In some embodiments, provided is use of the anti-PD-L1 antibody or the antigen-binding fragment or the fusion protein in preventing, treating, or ameliorating autoimmune diseases, cancers, tumors or infections, and other related diseases.

In some embodiments, provided is use of the anti-PD-L1 antibody or the antigen-binding fragment or the fusion protein in preparing a medicament for preventing, treating, or ameliorating autoimmune diseases, cancers, tumors or infections, and other related diseases.

The specific dosage and therapeutic regimen for any particular patient will depend on a variety of factors including the particular antibody or fusion protein or derivative used, the age, body weight, general health condition, sex, and diet of the patient, and the time of administration, frequency of excretion, drug combination, and the severity of the particular disease being treated. These factors are judged by medical caregivers included within the scope of those of ordinary skill in the art. The dosage will also depend on the individual patient to be treated, the route of administration, the type of the formulation, the nature of the compound used, the severity of the disease, and the efficacy desired. The dosage employed can be determined by pharmacological and pharmacokinetic principles well known in the art.

The modes of administration for the antibody or the fusion protein or the derivative include, but are not limited to, intradermal, intramuscular, intraperitoneal, intravenous, subcutaneous, nasal, spinal epidural, and oral administration. The pharmaceutical composition may be administered by any convenient route, e.g., by infusion or bolus injection, by absorption through epithelial or cutaneous mucosa (e.g., oral mucosa or rectal and intestinal mucosa), and may be co-administered with other biologically active agents. Thus, the pharmaceutical composition comprising the antibody or the antigen-binding fragment or the fusion protein of the present disclosure can be administered orally, rectally, parenterally, intravesically (e.g., intravesical infusion), intracisternally, intravaginally, intraperitoneally, topically (e.g. by powder, ointment, drop or transdermal patch) or buccally, or by oral or nasal spray.

The term "parenteral" as used herein refers to modes of administration including intravenous, intramuscular, intraperitoneal, intrasternal, subcutaneous, and intraarticular injections and infusions.

The mode of administration may be systemic or local. In addition, it may be desired to introduce the antibody or the fusion protein of the present disclosure into the central nervous system by any suitable route, including intracerebroventricular and intrathecal injections; intracerebroventricular injection may be assisted by an intracerebroventricular catheter connected to, for example, a reservoir (which may be an Ommaya reservoir). Pulmonary administration is also possible, for example, by the use of an inhaler or nebulizer, and by the use of nebulized formulations.

The antibody or the fusion protein of the present disclosure may be administered locally to an area in need of treatment; this may be achieved by (but not limited to) the following: local infusion during surgery (e.g., topical application in combination with a post-operative wound dressing), by injection, by means of a catheter, by means of a suppository, or by means of an implant, the implant being of a porous, non-porous, or gelatinous material, including membranes (such as silicone rubber membranes) or fibers. Preferably, when administering a protein (including an antibody or a fusion protein) of the present disclosure, care must be taken to use materials that do not absorb the protein.

In some embodiments, the present disclosure comprises a nucleic acid or a polynucleotide encoding an antibody or a fusion protein, which can be administered *in vivo* to facilitate expression of the encoded protein by constructing the nucleic acid or the polynucleotide as part of a suitable nucleic acid expression vector; and the nucleic acid or the polynucleotide or the vector described above is administered to be intracellular by the following methods, for example, by using a retroviral vector (see U.S. Pat. No. 4,980,286), by direct injection, by using microprojectile bombardment (e.g., gene gun; Biolistic, Dupont), by coating with lipids or cell surface receptors or transfection reagents, or by ligation with homeobox-like peptides with known capability of entering the nucleus (see, e.g., Joliot et al., 1991, Proc. Natl. Acad. Sci. USA 88:1864-1868). Alternatively, the nucleic acid can be introduced into the cell and integrated into the host cell DNA for expression by homologous recombination.

In some embodiments, the antibody or the fusion protein of the present disclosure is administered to a patient at a dose of 0.01 mg/kg to 100 mg/kg of patient body weight, or 0.1 mg/kg to 20 mg/kg of patient body weight. A second or more doses of the antibody or the antigen-binding fragment or the fusion protein may be administered subsequently to the initial dose, at about the same or less dose as the initial dose, wherein the administration at the subsequent dose may be separated by at least 1 to 3 days; or at least one week. Tolerability can also be increased with a relatively low initial dose, followed by an increased dose. The dose and frequency for administration of the antibody or the fusion protein of the present disclosure can be reduced by enhancing the uptake and tissue penetration ability (e.g., into the brain) of the antibody or the fusion protein through modifications such as lipidation.

Various known delivery systems can be used to administer the antibody or the fusion protein or the derivative, or the polynucleotide encoding the same of the present disclosure, e.g., encapsulation in liposomes, microparticles, microcapsules, recombinant cells capable of expressing the compound, receptor-mediated endocytosis (see, e.g., Wu and Wu, 1987, J. Biol. Chem. 262:4429-4432), or construction of nucleic acids as part of a retrovirus or other vectors.

### Combination Therapy

In some embodiments, the anti-PD-L1 antibody or the antigen-binding fragment or the fusion protein of the present disclosure can be administered in combination with other therapeutic or prophylactic regimens, which includes one or more antibodies or antigen-binding fragments or fusion proteins of the present disclosure are administered together or in combination with one or more other therapeutic agents or methods. In some embodiments, other therapeutic regimens include, but are not limited to, radiation therapy, chemotherapy, hormone therapy, and the like. For combination therapy, the antibody or the fusion protein may be administered simultaneously or separately with other therapeutic agents. When administered separately, the antibody or the fusion protein of the present disclosure can be administered before or after administration of another additional therapeutic agent.

In some embodiments, the antibody or the fusion protein of the present disclosure is administered in combination with a chemotherapeutic agent. In some embodiments, chemotherapeutic agents that can be administered with the antibody or the fusion protein of the present disclosure include, but are not limited to, antibiotic derivatives (e.g., doxorubicin, bleomycin, daunorubicin, and actinomycin D), antiestrogens (e.g., tamoxifen), antimetabolites (e.g., fluorouracil, 5-FU, methotrexate, floxuridine, interferon α-2b, glutamic acid, mithramycin, mercaptopurine, and 6-thioguanine), cytotoxic agents (e.g., carmustine, BCNU, lomustine, CCNU, cytarabine, cyclophosphamide, estramustine, hydroxyurea, procarbazine, mitomycin, busulfan, cisplatin, and vincristine sulfate), hormones (e.g., medroxyprogesterone, estramustine sodium phosphate, ethinylestradiol, estradiol, megestrol acetate, methyltestosterone, stilbestrol diphosphate, chlorotrianisene, and testolactone), nitrogen mustard derivatives (e.g., melphalan, chlorambucil, dichloromethyl diethylamine (mechlorethamine), and thiotepa), steroids and combinations thereof (e.g., betamethasone sodium phosphate), and other compounds (e.g., dacarbazine, asparaginase, mitotane, vincristine sulfate, vinblastine sulfate, and etoposide).

In some embodiments, the anti-PD-L1 antibody or the fusion protein of the present disclosure is administered in combination with a chemotherapeutic agent. Examples of the chemotherapeutic agents include immunotherapeutic agents, including but not limited to therapeutic antibodies suitable for treating a patient. Some examples of the therapeutic antibodies include rituximab, trastuzumab, tositumomab, ibritumomab, alemtuzumab, epratuzumab, bevacizumab, cetuximab, brentuximab, and the like.

### Pharmaceutical Composition

The present disclosure further provides a pharmaceutical composition. Such compositions comprise an anti-PD-L1 antibody or an antigen-binding fragment or a fusion protein and a pharmaceutically acceptable excipient. In some embodiments, the pharmaceutical composition comprises 0.1%-99% of the anti-PD-L1 antibody or the antigen-binding fragment or the fusion protein. In some embodiments, the pharmaceutical composition further comprises an anti-cancer agent (e.g., an immune checkpoint inhibitor).

In some embodiments, the term "pharmaceutically acceptable" refers to a substance approved by a government regulatory agency or listed in commonly-recognized pharmacopeias for use in animals, and particularly in humans. In addition, the "pharmaceutically acceptable excipient" generally refers to any type of non-toxic solid, semi-solid or liquid filler, diluent, an encapsulating material, a formulation additive, or the like.

The term "excipient" refers to a diluent, an adjuvant, an excipient, or a carrier that can be administered to a patient together with the active ingredient. Such pharmaceutical carriers can be sterile liquids, such as water and oils, including petroleum and oils originated from, animal, plant or synthesis, such as peanut oil, soybean oil, mineral oil, and sesame oil. Water is a preferred carrier when the pharmaceutical composition is administered intravenously. A saline aqueous solution, a glucose aqueous solution, and a glycerol solution can also be used as a liquid carrier, especially for injection. Suitable pharmaceutical excipients include starch, glucose, lactose, gelatin, malt, rice, flour, chalk, silica gel, sodium stearate, glycerol monostearate, talc, skimmed milk powder, glycerol, propylene, glycol, water, ethanol, and the like. If desired, the composition may also contain a small amount of a wetting agent, an emulsifier, or a pH buffering agent. Antibacterials such as benzyl alcohol or methylparaben, antioxidants such as ascorbic acid, chelating agents, and tonicity adjusting agents such as dextrose are also contemplated. These compositions may be in the form of solutions, suspensions, emulsions, tablets, pills, capsules, powders, sustained-release formulations, etc. The composition may be formulated as a suppository using conventional binders and carriers such as triglyceride. Oral formulations may comprise standard carriers such as mannitol, lactose, starch, magnesium stearate, sodium saccharin, cellulose, and magnesium carbonate of pharmaceutical grade. Such compositions will contain a clinically effective dose of an antibody or an antigen-binding fragment or a fusion protein, preferably in purified form, together with an appropriate amount of an excipient, to provide a form suitable for administration to a patient. The formulation should be suitable for the administration mode. The parenteral formulation may be enclosed in ampules, disposable syringes or multiple dose vials made of glass or plastic.

In some embodiments, the composition is formulated into a pharmaceutical composition suitable for intravenous injection into a human body according to conventional steps. A composition for intravenous administration is usually a solution in sterile isotonic aqueous buffer. The composition may also comprise a solubilizer and a local anesthetic such as lidocaine to alleviate the pain at the injection site. In general, the active ingredients are provided in a unit dosage form individually or as a mixture. For example, the active ingredients are encapsulated in sealed containers (such as ampoule bottles or sachets) indicating the content of the active ingredient, in the form of lyophilized powder or anhydrous concentrate. Where the composition is administered by infusion, the composition can be dispensed in infusion bottles containing sterile, pharmaceutical grade water or saline. Where the composition is administrated by injection, the active ingredients can be mixed prior to administration using sterile water or saline for injection.

The antibody or the antigen-binding fragment or the fusion protein of the present disclosure may be in a neutral or salt form. Pharmaceutically acceptable salts include salts formed with anions derived from acids such as hydrochloric acid, phosphoric acid, acetic acid, oxalic acid, and tartaric acid, and salts formed with cations derived from, e.g., sodium, potassium, ammonium, calcium, iron hydroxide, isopropylamine, triethylamine, 2-ethylaminoethanol, histidine, and procaine.

### Examples

The technical solution of the present disclosure will be further illustrated below through specific examples, which are not intended to limit the protection scope of the present disclosure. Some nonessential modifications and adjustments made by other people according to the concept of the present disclosure still fall within the protection scope of the present disclosure.

The materials, reagents, and the like used in the following examples can be commercially available, unless otherwise indicated.

### Example 1: Preparation of Anti-PD-L1 Antibody and Fusion Protein A

### 1) Preparation of anti-PD-L1 antibody

The composition and related sequences of the exemplary antibodies are shown in Tables 1-10, wherein the heavy chain and light chain composition of the antibodies is shown in Table 1, the heavy chain and light chain CDR regions of the antibodies are shown in Table 2, the composition of the heavy chain CDR regions of the antibodies is shown in Tables 3 and 4, and the composition of the light chain CDR regions of the antibodies is shown in Table 5.

DNA sequences encoding the heavy chain and the light chain of the antibodies were cloned into expression vectors, and plasmids were separately extracted. The heavy chain and the light chain were transiently transfected into HEK293F cells according to the plasmid molar ratio of 1:1. After cell culture and purification, the anti-PD-L1 antibodies were obtained, and the sequencing results were the same as the expected sequences.

**Table 1. Composition of heavy chain and light chain of antibodies**

| Antibody No. | Heavy chain SEQ ID NO: | Light chain SEQ ID NO: |
|---|---|---|
| L1-R2-4 | 51 | 66 |
| L1-R2-6 | 51 | 67 |
| L1-R2-18 | 51 | 68 |
| L1-R2-78 | 51 | 69 |
| L1-R2-85 | 51 | 70 |
| L1-R2-89 | 51 | 71 |
| L1-R2-4-36 | 52 | 66 |
| L1-R2-4-46 | 53 | 66 |
| L1-R2-4-47 | 54 | 66 |
| L1-R2-4-55 | 55 | 66 |
| L1-R2-4-71 | 56 | 66 |

**Table 2. Heavy chain and light chain CDR regions of antibodies**

| Name | Amino acid sequence | SEQ ID NO: |
|---|---|---|
| HCDR1 | DSWIH | 1 |
| | GFTFSDSWIH | 91 |
| | SFSLKDSWIH | 92 |
| | GWATRDSWIH | 93 |
| | NSHLRDSWIH | 94 |
| | GLRPADSWIH | 95 |
| | WISPYGGSTYYADSVKG | 2 |
| | WISPYGGSTYYADDFRH | 3 |
| | WISPYGGSTYYADSLGD | 4 |
| HCDR2 | WISPYGGSTYYADHLSQ | 5 |
| | WISPYGGSTYYADGMRS | 6 |
| | WISPYGGSTYYADSYRS | 7 |
| | WISPYGGSTYYADPYVG | 8 |
| | WISPYGGSTYYADSLKG | 9 |
| | WISPYGGSTYYADSYRA | 10 |
| | WISPYGGSTYYADYLHG | 11 |
| HCDR3 | RHWPGGFDY | 12 |
| | RHWPGGLLP | 13 |
| LCDR1 | LASQTIGTWLA | 14 |
| | QASQDIGKHLN | 15 |
| | RASQGIGSWLA | 16 |
| | RASQGISSYLA | 17 |
| | RASQGIGKFLA | 18 |
| LCDR2 | AASTLQS | 19 |
| | GASNLKT | 20 |
| | AASSLQS | 21 |
| | AASRLQS | 22 |
| LCDR3 | QQYYSTPRT | 23 |
| | QQSYTTPYT | 24 |
| | QQYFSTPYT | 25 |
| | QQYYSTPYT | 26 |

**Table 3. Composition of heavy chain CDR regions of antibodies**

| Heavy chain name | HCDR1 SEQ ID NO: | HCDR2 SEQ ID NO: | HCDR3 SEQ ID NO: |
|---|---|---|---|
| CP11-36 | 1 | 3 | 12 |
| CP11-46 | 1 | 4 | 12 |
| CP11-47 | 1 | 5 | 12 |
| CP11-55 | 1 | 6 | 12 |
| CP11-71 | 1 | 7 | 12 |
| CP14-16 | 1 | 8 | 12 |
| CP14-80 | 1 | 9 | 12 |
| CP21-8 | 1 | 10 | 12 |
| CP21-47 | 1 | 11 | 12 |
| CP21-59 | 1 | 2 | 12 |
| CP22-34 | 1 | 2 | 12 |
| CP22-45 | 1 | 2 | 12 |
| CP22-44 | 1 | 2 | 12 |
| CP22-40 | 1 | 2 | 13 |
| L1-27 | 1 | 2 | 12 |

**Table 4. Composition of heavy chain CDR regions of antibodies**

| Heavy chain name | HCDR1 SEQ ID NO: | HCDR2 SEQ ID NO: | HCDR3 SEQ ID NO: |
|---|---|---|---|
| CP11-36 | 91 | 3 | 12 |
| CP11-46 | 91 | 4 | 12 |
| CP11-47 | 91 | 5 | 12 |
| CP11-55 | 91 | 6 | 12 |
| CP11-71 | 91 | 7 | 12 |
| CP14-16 | 91 | 8 | 12 |
| CP14-80 | 91 | 9 | 12 |
| CP21-8 | 91 | 10 | 12 |
| CP21-47 | 91 | 11 | 12 |
| CP21-59 | 92 | 2 | 12 |
| CP22-34 | 93 | 2 | 12 |
| CP22-45 | 94 | 2 | 12 |
| CP22-44 | 95 | 2 | 12 |
| CP22-40 | 91 | 2 | 13 |
| L1-27 | 91 | 2 | 12 |

**Table 5. Composition of light chain CDR regions of antibodies**

| Light chain name | LCDR1 SEQ ID NO: | LCDR2 SEQ ID NO: | LCDR3 SEQ ID NO: |
|---|---|---|---|
| R2-4 | 14 | 19 | 23 |
| R2-6 | 15 | 20 | 24 |
| R2-18 | 16 | 21 | 25 |
| R2-78 | 17 | 22 | 26 |
| R2-85 | 18 | 21 | 25 |
| R2-89 | 14 | 21 | 26 |

**Table 6. Heavy chain variable regions of antibodies**

| Heavy chain name | Amino acid sequence | SEQ ID NO: |
|---|---|---|
| L1-27 | EVQLVESGGGLVQPGGSLRLSCAASGFTFSDSWIHWVRQAPG KGLEWVGWISPYGGSTYYADSVKGRFTISADTSKNTAYLQMN SLRAEDTAVYYCARRHWPGGFDYWGQGTLVTVSS | 27 |
| CP11-36 | EVQLVESGGGLVQPGGSLRLSCAASGFTFSDSWIHWVRQAPG KGLEWVGWISPYGGSTYYADDFRHRFTISADTSKNTAYLQMN SLRAEDTAVYYCARRHWPGGFDYWGQGTLVTVSS | 28 |
| CP11-46 | EVQLVESGGGLVQPGGSLRLSCAASGFTFSDSWIHWVRQAPG KGLEWVGWISPYGGSTYYADSLGDRFTISADTSKNTAYLQMN SLRAEDTAVYYCARRHWPGGFDYWGQGTLVTVSS | 29 |
| CP11-47 | EVQLVESGGGLVQPGGSLRLSCAASGFTFSDSWIHWVRQAPG KGLEWVGWISPYGGSTYYADHLSQRFTISADTSKNTAYLQMN SLRAEDTAVYYCARRHWPGGFDYWGQGTLVTVSS | 30 |
| CP11-55 | EVQLVESGGGLVQPGGSLRLSCAASGFTFSDSWIHWVRQAPG KGLEWVGWISPYGGSTYYADGMRSRFTISADTSKNTAYLQMN SLRAEDTAVYYCARRHWPGGFDYWGQGTLVTVSS | 31 |
| CP11-71 | EVQLVESGGGLVQPGGSLRLSCAASGFTFSDSWIHWVRQAPG KGLEWVGWISPYGGSTYYADSYRSRFTISADTSKNTAYLQMNS LRAEDTAVYYCARRHWPGGFDYWGQGTLVTVSS | 32 |
| CP14-16 | EVQLVESGGGLVQPGGSLRLSCAASGFTFSDSWIHWVRQAPG KGLEWVGWISPYGGSTYYADPYVGRFTISADTSKNTAYLQMN SLRAEDTAVYYCARRHWPGGFDYWGQGTLVTVSS | 33 |
| CP14-80 | EVQLVESGGGLVQPGGSLRLSCAASGFTFSDSWIHWVRQAPG KGLEWVGWISPYGGSTYYADSLKGRFTISADTSKNTAYLQMN SLRAEDTAVYYCARRHWPGGFDYWGQGTLVTVSS | 34 |
| CP21-8 | EVQLVESGGGLVQPGGSLRLSCAASGFTFSDSWIHWVRQAPG KGLEWVGWISPYGGSTYYADSYRARFTISADTSKNTAYLQMN SLRAEDTAVYYCARRHWPGGFDYWGQGTLVTVSS | 35 |
| CP21-47 | EVQLVESGGGLVQPGGSLRLSCAASGFTFSDSWIHWVRQAPG KGLEWVGWISPYGGSTYYADYLHGRFTISADTSKNTAYLQMN SLRAEDTAVYYCARRHWPGGFDYWGQGTLVTVSS | 36 |
| CP21-59 | EVQLVESGGGLVQPGGSLRLSCAASSFSLKDSWIHWVRQAPG KGLEWVGWISPYGGSTYYADSVKGRFTISADTSKNTAYLQMN SLRAEDTAVYYCARRHWPGGFDYWGQGTLVTVSS | 37 |
| CP22-34 | EVQLVESGGGLVQPGGSLRLSCAASGWATRDSWIHWVRQAPG KGLEWVGWISPYGGSTYYADSVKGRFTISADTSKNTAYLQMN SLRAEDTAVYYCARRHWPGGFDYWGQGTLVTVSS | 38 |
| CP22-40 | EVQLVESGGGLVQPGGSLRLSCAASGFTFSDSWIHWVRQAPG KGLEWVGWISPYGGSTYYADSVKGRFTISADTSKNTAYLQMN SLRAEDTAVYYCARRHWPGGLLPWGQGTLVTVSS | 39 |
| CP22-44 | EVQLVESGGGLVQPGGSLRLSCAASGLRPADSWIHWVRQAPG KGLEWVGWISPYGGSTYYADSVKGRFTISADTSKNTAYLQMN SLRAEDTAVYYCARRHWPGGFDYWGQGTLVTVSS | 40 |
| CP22-45 | EVQLVESGGGLVQPGGSLRLSCAASNSHLRDSWIHWVRQAPG | 41 |
| | KGLEWVGWISPYGGSTYYADSVKGRFTISADTSKNTAYLQMN SLRAEDTAVYYCARRHWPGGFDYWGQGTLVTVSS | |

**Table 7. Light chain variable regions of antibodies**

| Light chain name | Amino acid sequence | SEQ ID NO: |
|---|---|---|
| R2-4 | EIVLTQSPSSLSASVGDRVTITCLASQTIGTWLAWYQQKPGKS PQLLIYAASTLQSGVPSRFSGSGSGTDFTLTISSLQPEDVATYY CQQYYSTPRTFGQGTKVEIK | 42 |
| R2-6 | EIVLTQSPSSVSASVGDKLTMTCQASQDIGKHLNWYQQKPGK PPKLLIYGASNLKTGVPSRFSGTGSGTDFTLTISSLQPEDFATY YCQQSYTTPYTFGQGTRLEIK | 43 |
| R2-18 | EIVLTQSPSSVSASVGDRITITCRASQGIGSWLAWYQQKPGKA PSLLIYAASSLQSGVPSRFSGSGSGTDFTLTISSLQTEDVAVYY CQQYFSTPYTFGQGTKVDIK | 44 |
| R2-78 | DIQMTQSPSSFSASTGDRVTITCRASQGISSYLAWYQQKPGKA PKLLIYAASRLQSGVPSRFSGSGSGTDFTLTISSLQAEDVAVYY CQQYYSTPYTFGQGTKVDIK | 45 |
| R2-85 | DIQMTQSPSSLSASVGDRVTIPCRASQGIGKFLAWYQQKPGK APSLLIYAASSLQSGVPSRFSGSGSGTDFTLTISSLQTEDVAVY YCQQYFSTPYTFGQGTKVDIK | 46 |
| R2-89 | DIQMTQSPSSLSASVGDRVTITCLASQTIGTWLAWYQQKPGK SPQLLIYAASSLQSGVPSRFSGSGSGTDFTLTISSLQPEDVATY YCQQYYSTPYTFGQGTKVDIK | 47 |

**Table 8. Constant regions of antibodies**

| Name | Amino acid sequence | SEQ ID NO: |
|---|---|---|
| Heavy chain constant region | | 48 |
| | ASTKGPSVFPLAPSSKSTSGGTAALGCLVKDYFPEPVTVSW | 49 |
| | NSGALTSGVHTFPAVLQSSGLYSLSSVVTVPSSSLGTQTYIC NVNHKPSNTKVDKKVEPKSCDKTHTCPPCPAPELLGGPSV FLFPPKPKDTLMISRTPEVTCVVVDVSHEDPEVKFNWYVD GVEVHNAKTKPREEQYASTYRVVSVLTVLHQDWLNGKEY KCKVSNKALPAPIEKTISKAKGQPREPQVYTLPPSREEMTK NQVSLTCLVKGFYPSDIAVEWESNGQPENNYKTTPPVLDSD GSFFLYSKLTVDKSRWQQGNVFSCSVMHEALHNHYTQKS LSLSPGK | |
| Light chain constant region | | 50 |

**Table 9. Heavy chain sequences of antibodies**

| Heavy chain name | Amino acid sequence | SEQ ID NO: |
|---|---|---|
| L1-27 | EVQLVESGGGLVQPGGSLRLSCAASGFTFSDSWIHWVRQAPG KGLEWVGWISPYGGSTYYADSVKGRFTISADTSKNTAYLQM NSLRAEDTAVYYCARRHWPGGFDYWGQGTLVTVSSASTKGP SVFPLAPSSKSTSGGTAALGCLVKDYFPEPVTVSWNSGALTSG VHTFPAVLQSSGLYSLSSVVTVPSSSLGTQTYICNVNHKPSNT KVDKKVEPKSCDKTHTCPPCPAPELLGGPSVFLFPPKPKDTL MISRTPEVTCVVVDVSHEDPEVKFNWYVDGVEVHNAKTKPR EEQYNSTYRVVSVLTVLHQDWLNGKEYKCKVSNKALPAPIE KTISKAKGQPREPQVYTLPPSRDELTKNQVSLTCLVKGFYPSD IAVEWESNGQPENNYKTTPPVLDSDGSFFLYSKLTVDKSRWQ QGNVFSCSVMHEALHNHYTQKSLSLSPGK | 51 |
| CP11-36 | EVQLVESGGGLVQPGGSLRLSCAASGFTFSDSWIHWVRQAPG KGLEWVGWISPYGGSTYYADDFRHRFTISADTSKNTAYLQM NSLRAEDTAVYYCARRHWPGGFDYWGQGTLVTVSSASTKGP SVFPLAPSSKSTSGGTAALGCLVKDYFPEPVTVSWNSGALTSG VHTFPAVLQSSGLYSLSSVVTVPSSSLGTQTYICNVNHKPSNT KVDKKVEPKSCDKTHTCPPCPAPELLGGPSVFLFPPKPKDTL MISRTPEVTCVVVDVSHEDPEVKFNWYVDGVEVHNAKTKPR | 52 |
| | EEQYNSTYRVVSVLTVLHQDWLNGKEYKCKVSNKALPAPIE KTISKAKGQPREPQVYTLPPSRDELTKNQVSLTCLVKGFYPSD IAVEWESNGQPENNYKTTPPVLDSDGSFFLYSKLTVDKSRWQ QGNVFSCSVMHEALHNHYTQKSLSLSPGK | |
| CP11-46 | EVQLVESGGGLVQPGGSLRLSCAASGFTFSDSWIHWVRQAPG KGLEWVGWISPYGGSTYYADSLGDRFTISADTSKNTAYLQM NSLRAEDTAVYYCARRHWPGGFDYWGQGTLVTVSSASTKGP SVFPLAPSSKSTSGGTAALGCLVKDYFPEPVTVSWNSGALTSG VHTFPAVLQSSGLYSLSSVVTVPSSSLGTQTYICNVNHKPSNT KVDKKVEPKSCDKTHTCPPCPAPELLGGPSVFLFPPKPKDTL MISRTPEVTCVVVDVSHEDPEVKFNWYVDGVEVHNAKTKPR EEQYNSTYRVVSVLTVLHQDWLNGKEYKCKVSNKALPAPIE KTISKAKGQPREPQVYTLPPSRDELTKNQVSLTCLVKGFYPSD IAVEWESNGQPENNYKTTPPVLDSDGSFFLYSKLTVDKSRWQ QGNVFSCSVMHEALHNHYTQKSLSLSPGK | 53 |
| CP11-47 | EVQLVESGGGLVQPGGSLRLSCAASGFTFSDSWIHWVRQAPG KGLEWVGWISPYGGSTYYADHLSQRFTISADTSKNTAYLQM NSLRAEDTAVYYCARRHWPGGFDYWGQGTLVTVSSASTKGP SVFPLAPSSKSTSGGTAALGCLVKDYFPEPVTVSWNSGALTSG VHTFPAVLQSSGLYSLSSVVTVPSSSLGTQTYICNVNHKPSNT KVDKKVEPKSCDKTHTCPPCPAPELLGGPSVFLFPPKPKDTL MISRTPEVTCVVVDVSHEDPEVKFNWYVDGVEVHNAKTKPR EEQYNSTYRVVSVLTVLHQDWLNGKEYKCKVSNKALPAPIE KTISKAKGQPREPQVYTLPPSRDELTKNQVSLTCLVKGFYPSD IAVEWESNGQPENNYKTTPPVLDSDGSFFLYSKLTVDKSRWQ QGNVFSCSVMHEALHNHYTQKSLSLSPGK | 54 |
| CP11-55 | EVQLVESGGGLVQPGGSLRLSCAASGFTFSDSWIHWVRQAPG KGLEWVGWISPYGGSTYYADGMRSRFTISADTSKNTAYLQM NSLRAEDTAVYYCARRHWPGGFDYWGQGTLVTVSSASTKGP SVFPLAPSSKSTSGGTAALGCLVKDYFPEPVTVSWNSGALTSG | 55 |
| | VHTFPAVLQSSGLYSLSSVVTVPSSSLGTQTYICNVNHKPSNT KVDKKVEPKSCDKTHTCPPCPAPELLGGPSVFLFPPKPKDTL MISRTPEVTCVVVDVSHEDPEVKFNWYVDGVEVHNAKTKPR EEQYNSTYRVVSVLTVLHQDWLNGKEYKCKVSNKALPAPIE KTISKAKGQPREPQVYTLPPSRDELTKNQVSLTCLVKGFYPSD IAVEWESNGQPENNYKTTPPVLDSDGSFFLYSKLTVDKSRWQ QGNVFSCSVMHEALHNHYTQKSLSLSPGK | |
| CP11-71 | EVQLVESGGGLVQPGGSLRLSCAASGFTFSDSWIHWVRQAPG KGLEWVGWISPYGGSTYYADSYRSRFTISADTSKNTAYLQMN SLRAEDTAVYYCARRHWPGGFDYWGQGTLVTVSSASTKGPS VFPLAPSSKSTSGGTAALGCLVKDYFPEPVTVSWNSGALTSG VHTFPAVLQSSGLYSLSSVVTVPSSSLGTQTYICNVNHKPSNT KVDKKVEPKSCDKTHTCPPCPAPELLGGPSVFLFPPKPKDTL MISRTPEVTCVVVDVSHEDPEVKFNWYVDGVEVHNAKTKPR EEQYNSTYRVVSVLTVLHQDWLNGKEYKCKVSNKALPAPIE KTISKAKGQPREPQVYTLPPSRDELTKNQVSLTCLVKGFYPSD IAVEWESNGQPENNYKTTPPVLDSDGSFFLYSKLTVDKSRWQ QGNVFSCSVMHEALHNHYTQKSLSLSPGK | 56 |
| CP14-16 | EVQLVESGGGLVQPGGSLRLSCAASGFTFSDSWIHWVRQAPG KGLEWVGWISPYGGSTYYADPYVGRFTISADTSKNTAYLQM NSLRAEDTAVYYCARRHWPGGFDYWGQGTLVTVSSASTKGP SVFPLAPSSKSTSGGTAALGCLVKDYFPEPVTVSWNSGALTSG VHTFPAVLQSSGLYSLSSVVTVPSSSLGTQTYICNVNHKPSNT KVDKKVEPKSCDKTHTCPPCPAPELLGGPSVFLFPPKPKDTL MISRTPEVTCVVVDVSHEDPEVKFNWYVDGVEVHNAKTKPR EEQYNSTYRVVSVLTVLHQDWLNGKEYKCKVSNKALPAPIE KTISKAKGQPREPQVYTLPPSRDELTKNQVSLTCLVKGFYPSD IAVEWESNGQPENNYKTTPPVLDSDGSFFLYSKLTVDKSRWQ QGNVFSCSVMHEALHNHYTQKSLSLSPGK | 57 |
| CP14-80 | EVQLVESGGGLVQPGGSLRLSCAASGFTFSDSWIHWVRQAPG | 58 |
| | KGLEWVGWISPYGGSTYYADSLKGRFTISADTSKNTAYLQM NSLRAEDTAVYYCARRHWPGGFDYWGQGTLVTVSSASTKGP SVFPLAPSSKSTSGGTAALGCLVKDYFPEPVTVSWNSGALTSG VHTFPAVLQSSGLYSLSSVVTVPSSSLGTQTYICNVNHKPSNT KVDKKVEPKSCDKTHTCPPCPAPELLGGPSVFLFPPKPKDTL MISRTPEVTCVVVDVSHEDPEVKFNWYVDGVEVHNAKTKPR EEQYNSTYRVVSVLTVLHQDWLNGKEYKCKVSNKALPAPIE KTISKAKGQPREPQVYTLPPSRDELTKNQVSLTCLVKGFYPSD IAVEWESNGQPENNYKTTPPVLDSDGSFFLYSKLTVDKSRWQ QGNVFSCSVMHEALHNHYTQKSLSLSPGK | |
| CP21-8 | EVQLVESGGGLVQPGGSLRLSCAASGFTFSDSWIHWVRQAPG KGLEWVGWISPYGGSTYYADSYRARFTISADTSKNTAYLQM NSLRAEDTAVYYCARRHWPGGFDYWGQGTLVTVSSASTKGP SVFPLAPSSKSTSGGTAALGCLVKDYFPEPVTVSWNSGALTSG VHTFPAVLQSSGLYSLSSVVTVPSSSLGTQTYICNVNHKPSNT KVDKKVEPKSCDKTHTCPPCPAPELLGGPSVFLFPPKPKDTL MISRTPEVTCVVVDVSHEDPEVKFNWYVDGVEVHNAKTKPR EEQYNSTYRVVSVLTVLHQDWLNGKEYKCKVSNKALPAPIE KTISKAKGQPREPQVYTLPPSRDELTKNQVSLTCLVKGFYPSD IAVEWESNGQPENNYKTTPPVLDSDGSFFLYSKLTVDKSRWQ QGNVFSCSVMHEALHNHYTQKSLSLSPGK | 59 |
| CP21-47 | EVQLVESGGGLVQPGGSLRLSCAASGFTFSDSWIHWVRQAPG KGLEWVGWISPYGGSTYYADYLHGRFTISADTSKNTAYLQM NSLRAEDTAVYYCARRHWPGGFDYWGQGTLVTVSSASTKGP SVFPLAPSSKSTSGGTAALGCLVKDYFPEPVTVSWNSGALTSG VHTFPAVLQSSGLYSLSSVVTVPSSSLGTQTYICNVNHKPSNT KVDKKVEPKSCDKTHTCPPCPAPELLGGPSVFLFPPKPKDTL MISRTPEVTCVVVDVSHEDPEVKFNWYVDGVEVHNAKTKPR EEQYNSTYRVVSVLTVLHQDWLNGKEYKCKVSNKALPAPIE KTISKAKGQPREPQVYTLPPSRDELTKNQVSLTCLVKGFYPSD IAVEWESNGQPENNYKTTPPVLDSDGSFFLYSKLTVDKSRWQ | 60 |
| | QGNVFSCSVMHEALHNHYTQKSLSLSPGK | |
| CP21-59 | EVQLVESGGGLVQPGGSLRLSCAASSFSLKDSWIHWVRQAPG KGLEWVGWISPYGGSTYYADSVKGRFTISADTSKNTAYLQM NSLRAEDTAVYYCARRHWPGGFDYWGQGTLVTVSSASTKGP SVFPLAPSSKSTSGGTAALGCLVKDYFPEPVTVSWNSGALTSG VHTFPAVLQSSGLYSLSSVVTVPSSSLGTQTYICNVNHKPSNT KVDKKVEPKSCDKTHTCPPCPAPELLGGPSVFLFPPKPKDTL MISRTPEVTCVVVDVSHEDPEVKFNWYVDGVEVHNAKTKPR EEQYNSTYRVVSVLTVLHQDWLNGKEYKCKVSNKALPAPIE KTISKAKGQPREPQVYTLPPSRDELTKNQVSLTCLVKGFYPSD IAVEWESNGQPENNYKTTPPVLDSDGSFFLYSKLTVDKSRWQ QGNVFSCSVMHEALHNHYTQKSLSLSPGK | 61 |
| CP22-34 | EVQLVESGGGLVQPGGSLRLSCAASGWATRDSWIHWVRQAP GKGLEWVGWISPYGGSTYYADSVKGRFTISADTSKNTAYLQ MNSLRAEDTAVYYCARRHWPGGFDYWGQGTLVTVSSASTK GPSVFPLAPSSKSTSGGTAALGCLVKDYFPEPVTVSWNSGALT SGVHTFPAVLQSSGLYSLSSVVTVPSSSLGTQTYICNVNHKPS NTKVDKKVEPKSCDKTHTCPPCPAPELLGGPSVFLFPPKPKDT LMISRTPEVTCVVVDVSHEDPEVKFNWYVDGVEVHNAKTKP REEQYNSTYRVVSVLTVLHQDWLNGKEYKCKVSNKALPAPI EKTISKAKGQPREPQVYTLPPSRDELTKNQVSLTCLVKGFYPS DIAVEWESNGQPENNYKTTPPVLDSDGSFFLYSKLTVDKSRW QQGNVFSCSVMHEALHNHYTQKSLSLSPGK | 62 |
| CP22-40 | EVQLVESGGGLVQPGGSLRLSCAASGFTFSDSWIHWVRQAPG KGLEWVGWISPYGGSTYYADSVKGRFTISADTSKNTAYLQM NSLRAEDTAVYYCARRHWPGGLLPWGQGTLVTVSSASTKGP SVFPLAPSSKSTSGGTAALGCLVKDYFPEPVTVSWNSGALTSG VHTFPAVLQSSGLYSLSSVVTVPSSSLGTQTYICNVNHKPSNT KVDKKVEPKSCDKTHTCPPCPAPELLGGPSVFLFPPKPKDTL MISRTPEVTCVVVDVSHEDPEVKFNWYVDGVEVHNAKTKPR | 63 |
| | EEQYNSTYRVVSVLTVLHQDWLNGKEYKCKVSNKALPAPIE KTISKAKGQPREPQVYTLPPSRDELTKNQVSLTCLVKGFYPSD IAVEWESNGQPENNYKTTPPVLDSDGSFFLYSKLTVDKSRWQ QGNVFSCSVMHEALHNHYTQKSLSLSPGK | |
| CP22-44 | EVQLVESGGGLVQPGGSLRLSCAASGLRPADSWIHWVRQAP GKGLEWVGWISPYGGSTYYADSVKGRFTISADTSKNTAYLQ MNSLRAEDTAVYYCARRHWPGGFDYWGQGTLVTVSSASTK GPSVFPLAPSSKSTSGGTAALGCLVKDYFPEPVTVSWNSGALT SGVHTFPAVLQSSGLYSLSSVVTVPSSSLGTQTYICNVNHKPS NTKVDKKVEPKSCDKTHTCPPCPAPELLGGPSVFLFPPKPKDT LMISRTPEVTCVVVDVSHEDPEVKFNWYVDGVEVHNAKTKP REEQYNSTYRVVSVLTVLHQDWLNGKEYKCKVSNKALPAPI EKTISKAKGQPREPQVYTLPPSRDELTKNQVSLTCLVKGFYPS DIAVEWESNGQPENNYKTTPPVLDSDGSFFLYSKLTVDKSRW QQGNVFSCSVMHEALHNHYTQKSLSLSPGK | 64 |
| CP22-45 | EVQLVESGGGLVQPGGSLRLSCAASNSHLRDSWIHWVRQAP GKGLEWVGWISPYGGSTYYADSVKGRFTISADTSKNTAYLQ MNSLRAEDTAVYYCARRHWPGGFDYWGQGTLVTVSSASTK GPSVFPLAPSSKSTSGGTAALGCLVKDYFPEPVTVSWNSGALT SGVHTFPAVLQSSGLYSLSSVVTVPSSSLGTQTYICNVNHKPS NTKVDKKVEPKSCDKTHTCPPCPAPELLGGPSVFLFPPKPKDT LMISRTPEVTCVVVDVSHEDPEVKFNWYVDGVEVHNAKTKP REEQYNSTYRVVSVLTVLHQDWLNGKEYKCKVSNKALPAPI EKTISKAKGQPREPQVYTLPPSRDELTKNQVSLTCLVKGFYPS DIAVEWESNGQPENNYKTTPPVLDSDGSFFLYSKLTVDKSRW QQGNVFSCSVMHEALHNHYTQKSLSLSPGK | 65 |

**Table 10. Light chain sequences of antibodies**

| Light chain name | Amino acid sequence | SEQ ID NO: |
|---|---|---|
| R2-4 | EIVLTQSPSSLSASVGDRVTITCLASQTIGTWLAWYQQKPGKSP QLLIYAASTLQSGVPSRFSGSGSGTDFTLTISSLQPEDVATYYCQ QYYSTPRTFGQGTKVEIKRTVAAPSVFIFPPSDEQLKSGTASVVC LLNNFYPREAKVQWKVDNALQSGNSQESVTEQDSKDSTYSLS STLTLSKADYEKHKVYACEVTHQGLSSPVTKSFNRGEC | 66 |
| R2-6 | EIVLTQSPSSVSASVGDKLTMTCQASQDIGKHLNWYQQKPGKP PKLLIYGASNLKTGVPSRFSGTGSGTDFTLTISSLQPEDFATYYC QQSYTTPYTFGQGTRLEIKRTVAAPSVFIFPPSDEQLKSGTASVV CLLNNFYPREAKVQWKVDNALQSGNSQESVTEQDSKDSTYSL SSTLTLSKADYEKHKVYACEVTHQGLSSPVTKSFNRGEC | 67 |
| R2-18 | EIVLTQSPSSVSASVGDRITITCRASQGIGSWLAWYQQKPGKAP SLLIYAASSLQSGVPSRFSGSGSGTDFTLTISSLQTEDVAVYYCQ QYFSTPYTFGQGTKVDIKRTVAAPSVFIFPPSDEQLKSGTASVV CLLNNFYPREAKVQWKVDNALQSGNSQESVTEQDSKDSTYSL SSTLTLSKADYEKHKVYACEVTHQGLSSPVTKSFNRGEC | 68 |
| R2-78 | DIQMTQSPSSFSASTGDRVTITCRASQGISSYLAWYQQKPGKAP KLLIYAASRLQSGVPSRFSGSGSGTDFTLTISSLQAEDVAVYYCQ QYYSTPYTFGQGTKVDIKRTVAAPSVFIFPPSDEQLKSGTASVV CLLNNFYPREAKVQWKVDNALQSGNSQESVTEQDSKDSTYSL SSTLTLSKADYEKHKVYACEVTHQGLSSPVTKSFNRGEC | 69 |
| R2-85 | DIQMTQSPSSLSASVGDRVTIPCRASQGIGKFLAWYQQKPGKA PSLLIYAASSLQSGVPSRFSGSGSGTDFTLTISSLQTEDVAVYYC QQYFSTPYTFGQGTKVDIKRTVAAPSVFIFPPSDEQLKSGTASV VCLLNNFYPREAKVQWKVDNALQSGNSQESVTEQDSKDSTYS LSSTLTLSKADYEKHKVYACEVTHQGLSSPVTKSFNRGEC | 70 |
| R2-89 | DIQMTQSPSSLSASVGDRVTITCLASQTIGTWLAWYQQKPGKS PQLLIYAASSLQSGVPSRFSGSGSGTDFTLTISSLQPEDVATYYC QQYYSTPYTFGQGTKVDIKRTVAAPSVFIFPPSDEQLKSGTASV VCLLNNFYPREAKVQWKVDNALQSGNSQESVTEQDSKDSTYS LSSTLTLSKADYEKHKVYACEVTHQGLSSPVTKSFNRGEC | 71 |

### 2) Preparation of fusion protein A

The schematic structural diagram of fusion protein A is shown in FIG. 1, which consists of 4 polypeptides: a first polypeptide (set forth in SEQ ID NO: 83), a second polypeptide (set forth in SEQ ID NO: 84), and two third polypeptides (set forth in SEQ ID NO: 66) with identical sequences, wherein the first polypeptide comprises from N-terminus to C-terminus: an anti-PD-L1 heavy chain a (set forth in SEQ ID NO: 79), a linker (set forth in SEQ ID NO: 85), and IL-15Rα sushi domain (set forth in SEQ ID NO: 81); the second polypeptide comprises from N-terminus to C-terminus: an anti-PD-L1 heavy chain b (set forth in SEQ ID NO: 80), a linker (set forth in SEQ ID NO: 85), and IL-15 (set forth in SEQ ID NO: 82); the third polypeptide is an anti-PD-L1 light chain. The first polypeptide has a nucleic acid sequence set forth in SEQ ID NO: 72, the second polypeptide has a nucleic acid sequence set forth in SEQ ID NO: 73, and the third polypeptide has a nucleic acid sequence set forth in SEQ ID NO: 74. The related amino acid sequences of fusion protein A are shown in Table 11, and the related nucleic acid sequences are shown in Table 12.

The DNA sequences encoding the first polypeptide, the second polypeptide, and the third polypeptide of fusion protein A were separately cloned into expression vectors, and then transiently transfected into HEK293F cells. Fusion protein A was obtained by cell culture and purification.

**Table 11. Related amino acid sequences of fusion protein A**

| Name | Amino acid sequence | SEQ ID NO: |
|---|---|---|
| HCDR1 | DSWIH | 1 |
| HCDR2 | WISPYGGSTYYADSYRS | 7 |
| HCDR3 | RHWPGGFDY | 12 |
| LCDR1 | LASQTIGTWLA | 14 |
| LCDR2 | AASTLQS | 19 |
| LCDR3 | QQYYSTPRT | 23 |
| VH | EVQLVESGGGLVQPGGSLRLSCAASGFTFSDSWI HWVRQAPGKGLEWVGWISPYGGSTYYADSYRS RFTISADTSKNTAYLQMNSLRAEDTAVYYCARRH WPGGFDYWGQGTLVTVSS | 32 |
| VL | EIVLTQSPSSLSASVGDRVTITCLASQTIGTWLAW YQQKPGKSPQLLIYAASTLQSGVPSRFSGSGSGT DFTLTISSLQPEDVATYYCQQYYSTPRTFGQGTKV EIK | 42 |
| CHa | ASTKGPSVFPLAPSSKSTSGGTAALGCLVKDYFPE PVTVSWNSGALTSGVHTFPAVLQSSGLYSLSSVV TVPSSSLGTQTYICNVNHKPSNTKVDKKVEPKSC | 77 |
| | DKTHTCPPCPAPELLGGPSVFLFPPKPKDTLMISR TPEVTCVVVDVSHEDPEVKFNWYVDGVEVHNA KTKPREEQYNSTYRVVSVLTVLHQDWLNGKEYK CKVSNKALPAPIEKTISKAKGQPREPQVYTLPPCR DELTKNQVSLWCLVKGFYPSDIAVEWESNGQPEN NYKTTPPVLDSDGSFFLYSKLTVDKSRWQQGNVF SCSVMHEALHNHYTQKSLSLSPGA | |
| CHb | ASTKGPSVFPLAPSSKSTSGGTAALGCLVKDYFPE PVTVSWNSGALTSGVHTFPAVLQSSGLYSLSSVV TVPSSSLGTQTYICNVNHKPSNTKVDKKVEPKSC DKTHTCPPCPAPELLGGPSVFLFPPKPKDTLMISR TPEVTCVVVDVSHEDPEVKFNWYVDGVEVHNA KTKPREEQYNSTYRVVSVLTVLHQDWLNGKEYK CKVSNKALPAPIEKTISKAKGQPREPQVCTLPPSR DELTKNQVSLSCAVKGFYPSDIAVEWESNGQPEN NYKTTPPVLDSDGSFFLVSKLTVDKSRWQQGNVF SCSVMHEALHNHYTQKSLSLSPGA | 78 |
| CL | | 50 |
| Anti-PD-L1 heavy chain a | EVQLVESGGGLVQPGGSLRLSCAASGFTFSDSWI HWVRQAPGKGLEWVGWISPYGGSTYYADSYRS RFTISADTSKNTAYLQMNSLRAEDTAVYYCARRH WPGGFDYWGQGTLVTVSSASTKGPSVFPLAPSS KSTSGGTAALGCLVKDYFPEPVTVSWNSGALTSG VHTFPAVLQSSGLYSLSSVVTVPSSSLGTQTYICN VNHKPSNTKVDKKVEPKSCDKTHTCPPCPAPELL GGPSVFLFPPKPKDTLMISRTPEVTCVVVDVSHE DPEVKFNWYVDGVEVHNAKTKPREEQYNSTYR VVSVLTVLHQDWLNGKEYKCKVSNKALPAPIEK TISKAKGQPREPQVYTLPPCRDELTKNQVSLWCL VKGFYPSDIAVEWESNGQPENNYKTTPPVLDSDG SFFLYSKLTVDKSRWQQGNVFSCSVMHEALHNH YTQKSLSLSPGA | 79 |
| Anti-PD-L1 heavy chain b | EVQLVESGGGLVQPGGSLRLSCAASGFTFSDSWI HWVRQAPGKGLEWVGWISPYGGSTYYADSYRS RFTISADTSKNTAYLQMNSLRAEDTAVYYCARRH WPGGFDYWGQGTLVTVSSASTKGPSVFPLAPSS KSTSGGTAALGCLVKDYFPEPVTVSWNSGALTSG VHTFPAVLQSSGLYSLSSVVTVPSSSLGTQTYICN VNHKPSNTKVDKKVEPKSCDKTHTCPPCPAPELL GGPSVFLFPPKPKDTLMISRTPEVTCVVVDVSHE DPEVKFNWYVDGVEVHNAKTKPREEQYNSTYR VVSVLTVLHQDWLNGKEYKCKVSNKALPAPIEK TISKAKGQPREPQVCTLPPSRDELTKNQVSLSCAV KGFYPSDIAVEWESNGQPENNYKTTPPVLDSDGS FFLVSKLTVDKSRWQQGNVFSCSVMHEALHNHY TQKSLSLSPGA | 80 |
| Anti-PD-L1 light chain/third polypeptide | EIVLTQSPSSLSASVGDRVTITCLASQTIGTWLAW YQQKPGKSPQLLIYAASTLQSGVPSRFSGSGSGT DFTLTISSLQPEDVATYYCQQYYSTPRTFGQGTKV EIKRTVAAPSVFIFPPSDEQLKSGTASVVCLLNNF YPREAKVQWKVDNALQSGNSQESVTEQDSKDS TYSLSSTLTLSKADYEKHKVYACEVTHQGLSSPV TKSFNRGEC | 66 |
| Linker | GGGGSGGGGSGGGGS | 85 |
| IL-15Rα sushi domain | ITCPPPMSVEHADIWVKSYSLYSRERYICNSGFKR KAGTSSLTECVLNKATNVAHWTTPSLKCIR | 81 |
| IL-15 | NWVNVISDLKKIEDLIQSMHIDATLYTESDVHPSC KVTAMKCFLLELQVISLESGDASIHDTVENLIILA NNSLSSNGNVTESGCKECEELEEKNIKEFLQSFV HIVQMFINTS | 82 |
| First polypeptide | EVQLVESGGGLVQPGGSLRLSCAASGFTFSDSWI HWVRQAPGKGLEWVGWISPYGGSTYYADSYRS RFTISADTSKNTAYLQMNSLRAEDTAVYYCARRH WPGGFDYWGQGTLVTVSSASTKGPSVFPLAPSS KSTSGGTAALGCLVKDYFPEPVTVSWNSGALTSG VHTFPAVLQSSGLYSLSSVVTVPSSSLGTQTYICN VNHKPSNTKVDKKVEPKSCDKTHTCPPCPAPELL | 83 |
| | GGPSVFLFPPKPKDTLMISRTPEVTCVVVDVSHE DPEVKFNWYVDGVEVHNAKTKPREEQYNSTYR VVSVLTVLHQDWLNGKEYKCKVSNKALPAPIEK TISKAKGQPREPQVYTLPPCRDELTKNQVSLWCL VKGFYPSDIAVEWESNGQPENNYKTTPPVLDSDG SFFLYSKLTVDKSRWQQGNVFSCSVMHEALHNH YTQKSLSLSPGAGGGGSGGGGSGGGGSITCPPPM SVEHADIWVKSYSLYSRERYICNSGFKRKAGTSS LTECVLNKATNVAHWTTPSLKCIR | |
| Second polypeptide | EVQLVESGGGLVQPGGSLRLSCAASGFTFSDSWI HWVRQAPGKGLEWVGWISPYGGSTYYADSYRS RFTISADTSKNTAYLQMNSLRAEDTAVYYCARRH WPGGFDYWGQGTLVTVSSASTKGPSVFPLAPSS KSTSGGTAALGCLVKDYFPEPVTVSWNSGALTSG VHTFPAVLQSSGLYSLSSVVTVPSSSLGTQTYICN VNHKPSNTKVDKKVEPKSCDKTHTCPPCPAPELL GGPSVFLFPPKPKDTLMISRTPEVTCVVVDVSHE DPEVKFNWYVDGVEVHNAKTKPREEQYNSTYR VVSVLTVLHQDWLNGKEYKCKVSNKALPAPIEK TISKAKGQPREPQVCTLPPSRDELTKNQVSLSCAV KGFYPSDIAVEWESNGQPENNYKTTPPVLDSDGS FFLVSKLTVDKSRWQQGNVFSCSVMHEALHNHY TQKSLSLSPGAGGGGSGGGGSGGGGSNWVNVIS DLKKIEDLIQSMHIDATLYTESDVHPSCKVTAMK CFLLELQVISLESGDASIHDTVENLIILANNSLSSN GNVTESGCKECEELEEKNIKEFLQSFVHIVQMFIN TS | 84 |

**Table 12. Related nucleic acid sequences of fusion protein A**

| Name | Nucleic acid sequence | SEQ ID NO: |
|---|---|---|
| First polypeptide | GAGGTGCAGCTGGTGGAATCCGGCGGAGGGCTGGTG CAGCCAGGTGGCTCTCTGAGACTGAGTTGCGCCGCTT CAGGATTCACCTTTTCCGACTCTTGGATTCACTGGGTG CGCCAGGCCCCCGGAAAGGGACTGGAGTGGGTCGGT TGGATTTCTCCTTACGGAGGGAGTACATACTATGCCGA CTCTTATAGGTCGCGATTCACTATCTCCGCTGATACTAG CAAAAACACCGCATATCTGCAGATGAACTCCCTGAGG | 72 |
| | GCAGAAGACACAGCCGTCTACTATTGTGCAAGGCGGC ATTGGCCCGGTGGCTTTGATTACTGGGGTCAGGGTAC CCTGGTGACCGTGTCCTCCGCCTCCACCAAGGGCCCC TCCGTGTTCCCTCTGGCCCCCTCTTCCAAGAGCACCA GCGGCGGCACCGCCGCTCTGGGATGTCTGGTGAAGG ATTACTTTCCCGAGCCTGTGACCGTGAGCTGGAACTC CGGCGCCCTGACCTCCGGCGTGCACACATTCCCTGCC GTGCTGCAGAGCAGCGGCCTGTACTCCCTGAGCAGC GTGGTGACCGTGCCTAGCTCCAGCCTGGGCACCCAGA CCTACATCTGTAACGTGAACCACAAGCCTTCCAACAC CAAGGTGGACAAGAAGGTGGAGCCTAAGAGCTGTGA CAAGACCCACACCTGCCCTCCTTGTCCTGCCCCTGAG CTGCTGGGCGGCCCATCTGTGTTCCTGTTCCCTCCCA AGCCCAAGGACACCCTGATGATCAGCAGAACCCCCG AGGTGACCTGTGTGGTGGTGGATGTGTCCCACGAGGA CCCTGAGGTGAAGTTTAACTGGTACGTGGATGGCGTG GAGGTGCACAATGCCAAGACCAAGCCCAGAGAGGAG CAGTACAATTCCACCTACAGGGTGGTGTCCGTGCTGA CCGTGCTGCACCAGGACTGGCTGAATGGCAAGGAGT ACAAGTGTAAGGTGAGCAACAAGGCCCTGCCCGCCC CTATCGAGAAGACCATCAGCAAGGCCAAGGGCCAGC CTAGGGAGCCTCAGGTGTACACCCTGCCCCCCTGCAG AGACGAGCTGACCAAGAATCAGGTGAGCCTGTGGTG CCTGGTGAAGGGCTTCTACCCCAGCGACATCGCCGTG GAGTGGGAGTCCAATGGCCAGCCTGAGAACAATTAC AAGACCACCCCCCCTGTGCTGGATAGCGATGGCAGCT TCTTTCTGTACAGCAAGCTGACCGTGGATAAGTCCAG GTGGCAGCAGGGCAATGTGTTTAGCTGTAGCGTGATG CACGAGGCCCTGCACAATCACTACACCCAGAAGAGC CTGAGCCTGTCCCCCGGCGCTGGCGGAGGAGGAAGC GGAGGCGGAGGTAGCGGCGGAGGAGGTAGCATCACC TGCCCCCCTCCCATGAGCGTGGAGCACGCCGACATTT GGGTGAAGAGCTACAGCCTGTACAGCCGGGAGCGCT ACATTTGCAACAGCGGCTTCAAGCGGAAGGCCGGCA CATCTTCTCTCACCGAGTGCGTGCTGAACAAGGCCAC CAACGTGGCCCATTGGACCACACCCAGCCTGAAGTGT ATCCGC | |
| Second polypeptide | GAGGTGCAGCTGGTGGAATCCGGCGGAGGGCTGGTG CAGCCAGGTGGCTCTCTGAGACTGAGTTGCGCCGCTT CAGGATTCACCTTTTCCGACTCTTGGATTCACTGGGTG CGCCAGGCCCCCGGAAAGGGACTGGAGTGGGTCGGT TGGATTTCTCCTTACGGAGGGAGTACATACTATGCCGA CTCTTATAGGTCGCGATTCACTATCTCCGCTGATACTAG CAAAAACACCGCATATCTGCAGATGAACTCCCTGAGG GCAGAAGACACAGCCGTCTACTATTGTGCAAGGCGGC ATTGGCCCGGTGGCTTTGATTACTGGGGTCAGGGTAC CCTGGTGACCGTGTCCTCCGCCTCCACCAAGGGCCCC | 73 |
| | TCCGTGTTCCCTCTGGCCCCCTCTTCCAAGAGCACCA GCGGCGGCACCGCCGCTCTGGGATGTCTGGTGAAGG ATTACTTTCCCGAGCCTGTGACCGTGAGCTGGAACTC CGGCGCCCTGACCTCCGGCGTGCACACATTCCCTGCC GTGCTGCAGAGCAGCGGCCTGTACTCCCTGAGCAGC GTGGTGACCGTGCCTAGCTCCAGCCTGGGCACCCAGA CCTACATCTGTAACGTGAACCACAAGCCTTCCAACAC CAAGGTGGACAAGAAGGTGGAGCCTAAGAGCTGTGA CAAGACCCACACCTGCCCTCCTTGTCCTGCCCCTGAG CTGCTGGGCGGCCCATCTGTGTTCCTGTTCCCTCCCA AGCCCAAGGACACCCTGATGATCAGCAGAACCCCCG AGGTGACCTGTGTGGTGGTGGATGTGTCCCACGAGGA CCCTGAGGTGAAGTTTAACTGGTACGTGGATGGCGTG GAGGTGCACAATGCCAAGACCAAGCCCAGAGAGGAG CAGTACAACAGCACCTACAGAGTGGTGTCCGTGCTGA CCGTGCTGCACCAGGATTGGCTGAACGGCAAGGAGT ACAAGTGCAAGGTGTCCAATAAGGCCCTGCCCGCCCC CATCGAGAAGACCATCTCCAAGGCCAAGGGCCAGCC CAGAGAGCCTCAGGTGTGTACCCTGCCTCCCTCCAGG GATGAGCTGACCAAGAACCAGGTGTCCCTGAGCTGC GCCGTGAAGGGCTTCTACCCCAGCGATATCGCCGTGG AGTGGGAGTCCAACGGCCAGCCCGAGAATAATTACA AGACCACCCCCCCCGTGCTGGACAGCGACGGATCTTT CTTTCTGGTGTCCAAGCTGACCGTGGATAAGTCCAGG TGGCAGCAGGGCAACGTGTTTAGCTGTAGCGTGATGC ACGAGGCCCTGCACAACCACTACACCCAGAAGTCCC TGTCCCTGAGCCCCGGCGCTGGCGGAGGAGGAAGCG GAGGCGGAGGTAGCGGCGGAGGAGGTAGCAATTGGG TGAACGTGATCAGCGACCTGAAGAAGATCGAGGACC TGATCCAGAGCATGCACATCGACGCCACCCTGTACAC CGAGTCAGACGTGCACCCCTCTTGCAAGGTCACCGCC ATGAAGTGCTTCCTGCTGGAGCTGCAGGTCATCAGCC TGGAAAGCGGAGACGCTAGCATCCACGACACCGTGG AGAACCTGATCATCCTGGCCAACAACAGCCTGAGCA GCAACGGCAACGTGACAGAGAGCGGTTGCAAGGAGT GCGAGGAGCTGGAGGAGAAGAACATCAAGGAGTTCC TGCAGTCCTTCGTGCACATCGTGCAGATGTTCATCAAC ACCAGC | |
| Third polypeptide | GAAATTGTGCTGACTCAGTCTCCATCCTCCCTGTCTGC ATCTGTAGGAGACAGAGTCACCATCACTTGCCTGGCA AGTCAGACCATTGGTACATGGTTAGCATGGTATCAGCA GAAACCAGGGAAATCTCCTCAGCTCCTGATCTATGCT GCATCCACTTTGCAATCAGGGGTCCCATCTCGGTTCA GTGGCAGTGGATCTGGGACAGATTTCACTCTCACCAT CAGCAGCCTGCAGCCTGAAGATGTTGCAACTTATTAC TGTCAGCAATATTATAGTACTCCTCGGACGTTCGGCCA AGGGACCAAGGTGGAGATTAAACGTACGGTGGCTGC | 74 |
| | ACCATCTGTCTTCATCTTCCCGCCATCTGATGAGCAGT TGAAATCTGGAACTGCCTCTGTTGTGTGCCTGCTGAA TAACTTCTATCCCAGAGAGGCCAAAGTACAGTGGAAG GTGGATAACGCCCTCCAATCGGGTAACTCCCAGGAGA GTGTCACAGAGCAGGACAGCAAGGACAGCACCTACA GCCTCAGCAGCACCCTGACGCTGAGCAAAGCAGACT ACGAGAAACACAAAGTCTACGCCTGCGAAGTCACCC ATCAGGGCCTGAGCTCGCCCGTCACAAAGAGCTTCA ACAGGGGAGAGTGT | |

### Example 2: Determination of Binding of Fusion Protein A to CTLL-2 Cells and Activity Assay

The binding of fusion protein A to CTLL-2 cells (mouse cytotoxic T lymphocyte cell line) endogenously expressing IL-15Rα, IL-2Rβ, and IL-2Rγ was assessed using an assay based on fluorescence-activated cell sorting (FACS). CTLL-2 cells were resuspended in PBS buffer to obtain a single-cell suspension, and the suspension was mixed with 100 µL of fusion protein A samples at different concentrations (starting from 100 nM, 2-fold serial dilution, with 10 concentrations) in equal volume (all in a 96-well plate) at 500,000 cells/well. The mixtures were equilibrated at 4 °C for 60 min and washed with PBS buffer. A phycoerythrin (PE)-conjugated goat-anti-human IgG Fc antibody (Invitrogen, Cat. No. 12-4998-82) was then added as a secondary antibody, and the mixtures were equilibrated at 4 °C in the dark for 30 min. The cells were washed again with PBS buffer and analyzed by flow cytometry. Data were analyzed using non-linear regression and GraphPad PRISM 8 (GraphPad Software, San Diego, CA). As shown in FIG. 2, the FACS binding assay showed that fusion protein A was able to significantly bind to CTLL-2 cells.

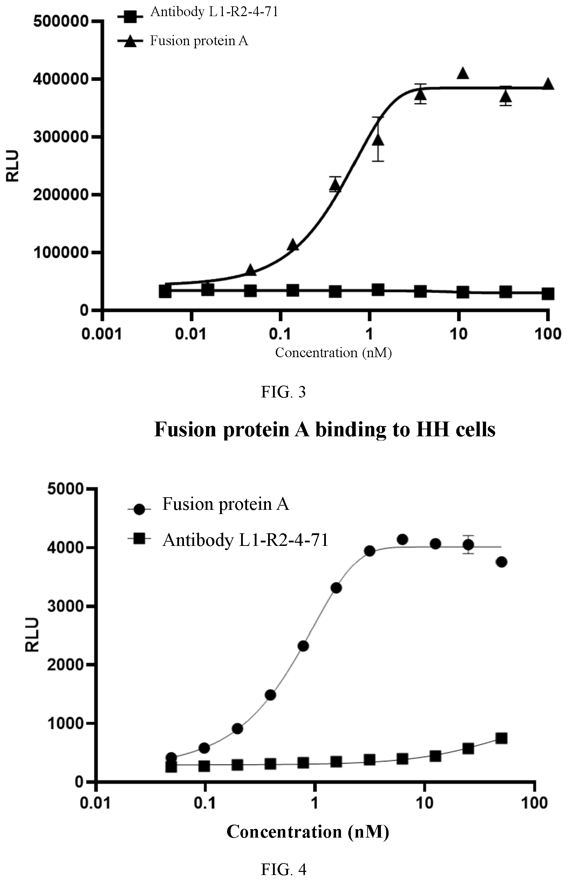

The activity of fusion protein A was assessed in a cell proliferation assay using CTLL-2 cells. CTLL-2 cells were maintained in RPMI-1640 culture medium supplemented with 2 mM L-glutamine, 1 mM sodium pyruvate, 10% fetal bovine serum (FBS), and 10 ng/mL IL-2 at 37 °C with 5% CO₂. The cells were subjected to suspension culture until they reached a cell density of 5 × 10⁵ cells per milliliter prior to aliquoting. For activity analysis, 2-3 days after the last aliquoting, the cells were washed with RPMI-1640 culture medium and resuspended in RPMI-1640 culture medium containing 15% FBS, and the cells were plated on a 96-well white plate at a density of 20 thousand cells/well (50 µL) supplemented with PBS at edge wells. Fusion protein A was diluted as follows: The diluent was RPMI-1640 culture medium containing 15% FBS. The antibody L1-R2-4-71 and fusion protein A samples were 1:3 diluted from 200 nM and added to the above white plate with the cells plated at 50 µL/well. The plate was incubated in an incubator at 37 °C with 5% CO₂ for 24 h. During the assay, the CellCounting-Lite 2.0 Luminescent Cell Viability Assay reagent was taken out from a refrigerator at -20 °C and equilibrated to room temperature in advance. The cell culture plate was taken out from the incubator and equilibrated at room temperature (25±3 °C) for 5-10 min. 50 µL of CellCounting-Lite 2.0 Luminescent Cell Viability Assay reagent (Vazyme, Cat. No. DD1101-02) was added to each well, and the plate was incubated at room temperature in the dark for 5-30 min. Relative light units (RLU) were read using the Luminescence detection module on the SpectraMax multi-functional microplate reader. Data were analyzed using non-linear regression and GraphPad PRISM 8 (GraphPad Software, San Diego, CA). As shown in FIG. 3, the CTLL-2 cell proliferation assay showed that fusion protein A could significantly activate the proliferation activity of CTLL-2 cells, with an EC₅₀ value of 0.417 nM.

### Example 3: Determination of Binding of Fusion Protein A to HH Cells and Activity Assay

The binding of fusion protein A to HH cells (human cutaneous T-cell lymphoma cells; ATCC CRL-2105) endogenously expressing IL-2Rβ and IL-2Rγ was assessed using an assay based on fluorescence-activated cell sorting (FACS). HH cells were resuspended in PBS buffer to obtain a single-cell suspension, and the suspension was mixed with 100 µL of antibody L1-R2-4-71 or fusion protein A samples at different concentrations (starting at a concentration of 100 nM, 2-fold dilution, with 11 concentration gradients) in equal volume (all in a 96-well plate) at 500,000 cells/well. The mixtures were equilibrated at 4 °C for 60 min and washed with PBS buffer. A phycoerythrin (PE)-conjugated goat-anti-human IgG Fc antibody (Invitrogen, Cat. No. 12-4998-82) was then added as a secondary antibody, and the mixtures were equilibrated at 4 °C in the dark for 30 min. The cells were washed again with PBS buffer and analyzed by flow cytometry. Data were analyzed using non-linear regression and GraphPad PRISM 8 (GraphPad Software, San Diego, CA). As shown in FIG. 4, the FACS binding assay showed that fusion protein A was able to significantly bind to HH cells.

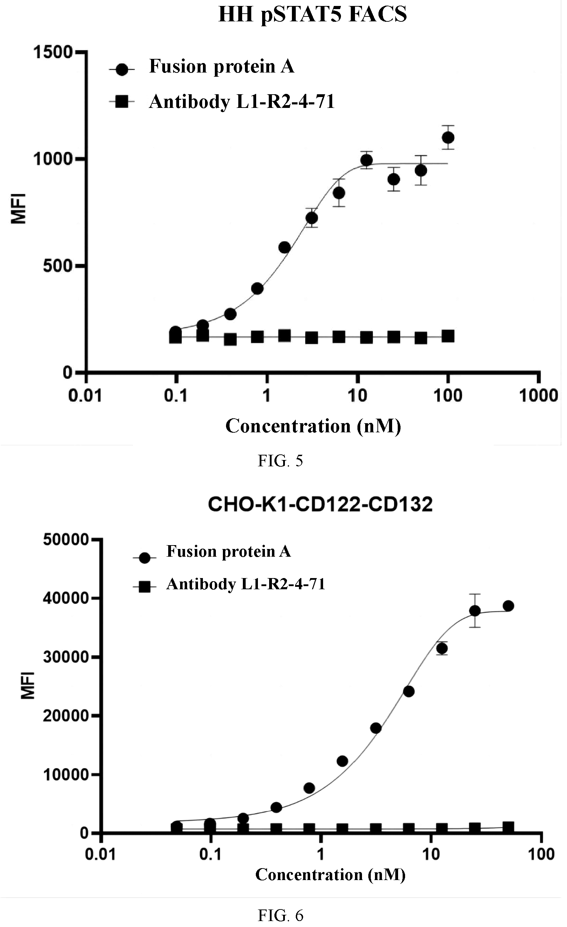

After binding to IL-15Rα, IL-15 would perform trans-acting to bind to IL-2Rβ and IL-2Rγ, activating the downstream signaling pathway and resulting in STAT5 phosphorylation. STAT5 phosphorylation was observed after co-incubation of fusion protein A samples at different concentrations with HH cells to evaluate the ability of fusion protein A to activate HH cell activity. HH cells in the logarithmic growth phase were collected, washed with PBS, and then resuspended in a preheated RPMI-1640 culture medium (2 million cells/100 µL), and the mixture was incubated at 37 °C for 30 min. In the meantime, antibody L1-R2-4-71 or fusion protein A samples were diluted with RPMI-1640 culture medium containing 10% FBS (2-fold dilution starting from 200 nM, with 11 concentrations, 100 µL each). 100 µL of each of the above diluted fusion protein A samples was well mixed with an equal volume of cells, and the mixture was incubated at 37 °C for 15 min. Then the mixture was immediately placed on ice, and an equal volume of 4% paraformaldehyde (FPA) solution (with a final concentration of 2%) was added to immobilize the cells. The mixture was placed on ice for 30 min. Washing was then performed with pre-cooled PBS buffer. The supernatant was discarded, and 1 mL of pre-cooled 90% methanol was added. The mixture was placed on ice for 30 min, then washed with pre-cooled PBS buffer, and resuspended in PBS buffer, and then PE anti-STAT5 Phospho (Tyr694) Antibody (BioLegend, Cat. No. 936904) was added. The mixture was incubated at room temperature in the dark for 40 min, washed, and then assayed using an instrument (Beckman CytoFlex). The mean fluorescent intensity (MFI) value was counted to evaluate the phosphorylation level of STAT5. Data were analyzed using non-linear regression and GraphPad PRISM 8 (GraphPad Software, San Diego, CA). As shown in FIG. 5, the STAT5 phosphorylation level analysis showed that fusion protein A could significantly activate the activity of HH cells, with an EC₅₀ value of 1.609 nM.

### Example 4: Determination of Binding of Fusion Protein A to CHO-K1-CD122-CD132 Cells

The binding of fusion protein A to CHO-K1 cells (i.e., CHO-K1-CD122-CD132 cells) exogenously expressing IL-2Rβ (CD122) and IL-2Rγ (CD132) was assessed using an assay based on fluorescence-activated cell sorting (FACS). CHO-K1-CD122-CD132 cells were resuspended in PBS buffer to obtain a single-cell suspension, and the suspension was mixed with 100 µL of fusion protein A or antibody L1-R2-4-71 samples at different concentrations (starting from 100 nM, 2-fold serial dilution) in equal volume (all in a 96-well plate) at 500,000 cells/well. The mixtures were equilibrated at 4 °C for 60 min and washed with PBS buffer. A phycoerythrin (PE)-conjugated goat-anti-human IgG Fc antibody (Invitrogen, Cat. No. 12-4998-82) was then added as a secondary antibody, and the mixtures were equilibrated at 4 °C in the dark for 30 min. The cells were washed again with PBS buffer and analyzed by flow cytometry. Data were analyzed using non-linear regression and GraphPad PRISM 8 (GraphPad Software, San Diego, CA). As shown in FIG. 6, the FACS binding assay showed that fusion protein A was able to significantly bind to CHO-K1-CD122-CD132 cells.

Construction method for the CHO-K1-CD122-CD132 cells: A vector connected with a CD122 gene sequence (NCBI Reference Sequence: NM_000878.5) was linearized and then electroporated into CHO-K1, and after culturing, a stable CHO-CD122 cell strain was obtained by screening; on the basis of the CHO-CD122 cell strain, a lentivirus containing a CD132 gene sequence (NCBI Reference Sequence: NM_000206.3) was used for further infection, and after culturing, a stable CHO-K1-CD122-CD132 cell strain was obtained by screening.

### Example 5: Experiment on In Vitro Activation of PBMC by Fusion Protein A

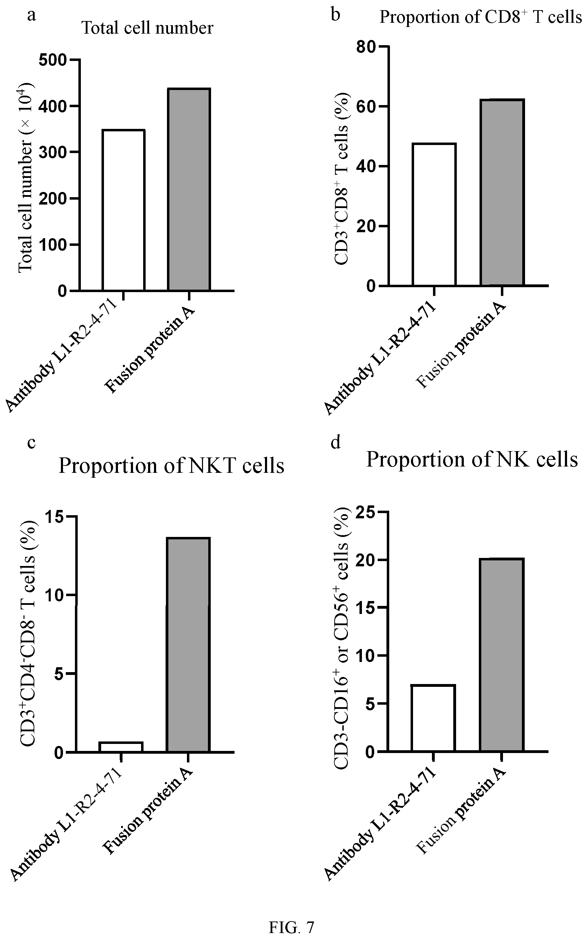

This example outlines the effect of fusion protein A samples in inducing the selective activation and expansion of effector lymphocytes in human peripheral blood. A 6-well cell culture plate was coated the night before with 500 µL of 200 ng/mL anti-CD3 antibody (Novoprotein, GMP-A018). The supernatant was discarded the next morning, and then PBMCs resuspended in RPMI-1640 culture medium containing 15% FBS were added at 1 × 10⁶ cells/3 mL/well. Fusion protein A or antibody L1-R2-4-71 was separately added at final concentrations of 0.5 nM and 20 nM, respectively. The mixtures were cultured at 37 °C with 5% CO₂ for 7 days. The PBMCs were transferred to a new 6-well cell culture plate (anti-CD3 antibody was removed), supplemented with fusion protein A or antibody L1-R2-4-71, and cultured for another 5 days. The proliferation of CD8⁺ T, NK, and NKT cells was detected by flow cytometry. The CD8⁺ T was CD3CD8 double positive T cells (i.e., CD3⁺CD8⁺ T cells), the NK cells were CD16 or CD56 positive cells, and the NKT cells were CD3 positive CD4CD8 double negative T cells (i.e., CD3⁺CD4⁻CD8⁻ T cells). The sources of the antibodies used for detection were as follows: CD3 antibody (elabscience, Cat. No. FW2689), CD4 antibody (elabscience, Cat. No. FW0218), CD8 antibody (elabscience, Cat. No. FW0931), CD16 antibody (BioLegend, Cat. No. 302038), and CD56 antibody (BioLegend, Cat. No. 302630). The results are shown in FIGs. 7a-d. Compared with the antibody L1-R2-4-71, fusion protein A could significantly promote the expansion of CD8⁺ T, NK, and NKT cells.

### Example 6: Cytokine Release Experiment for Fusion Protein A

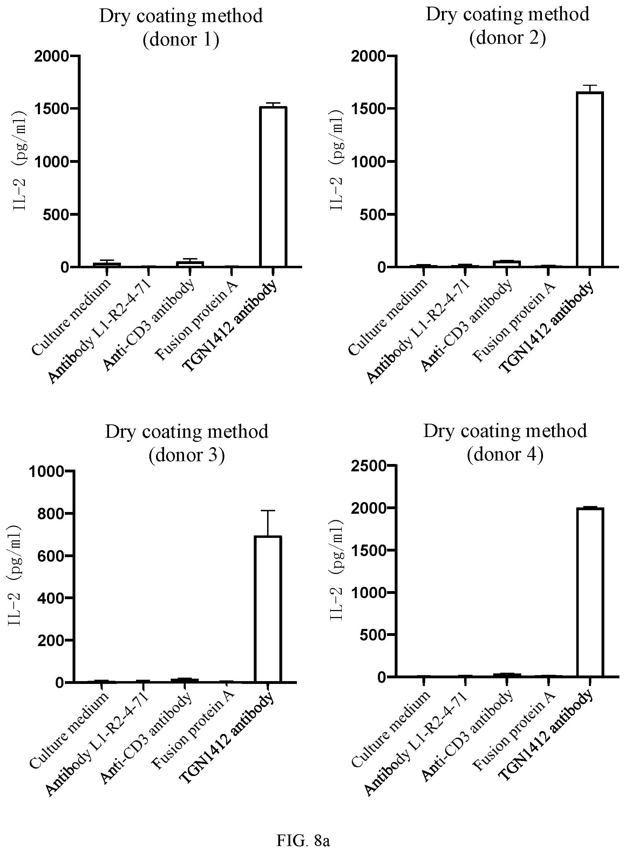

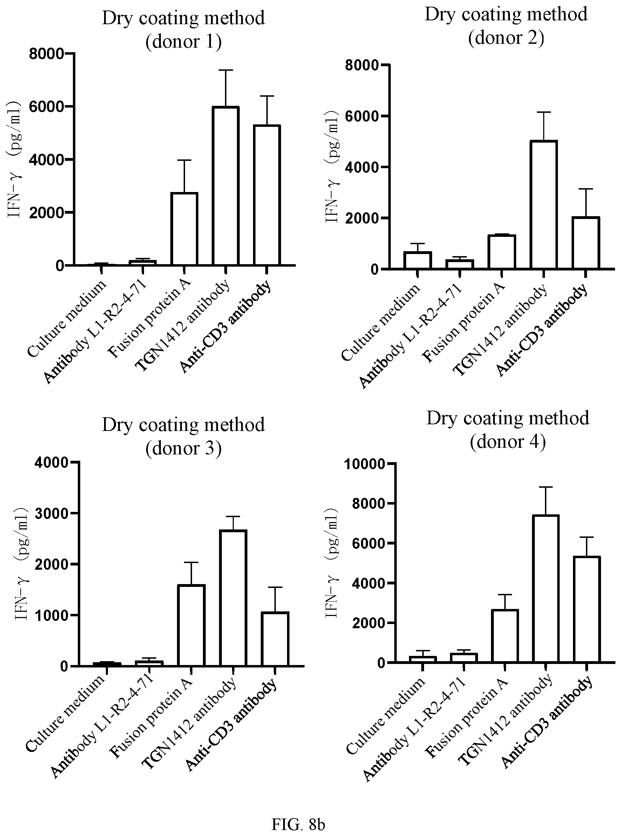

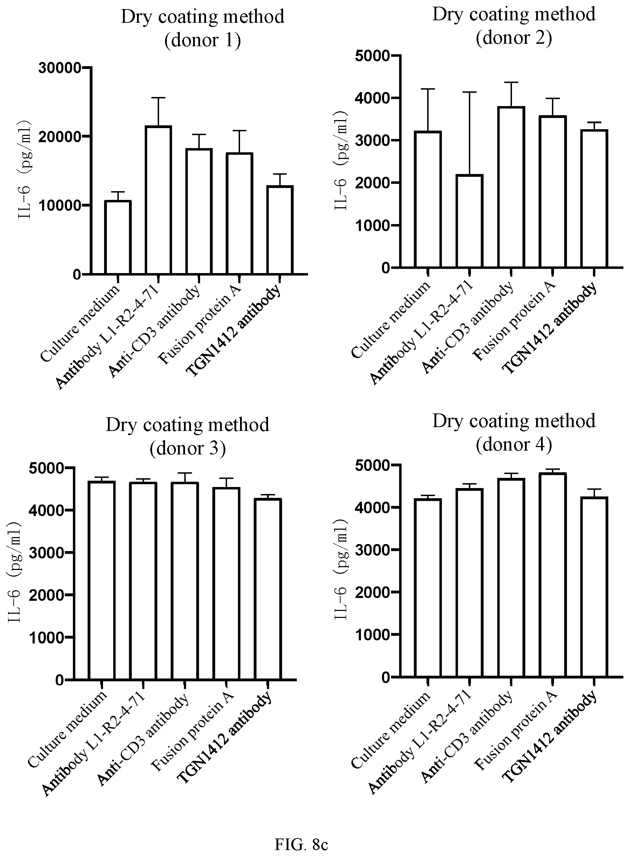

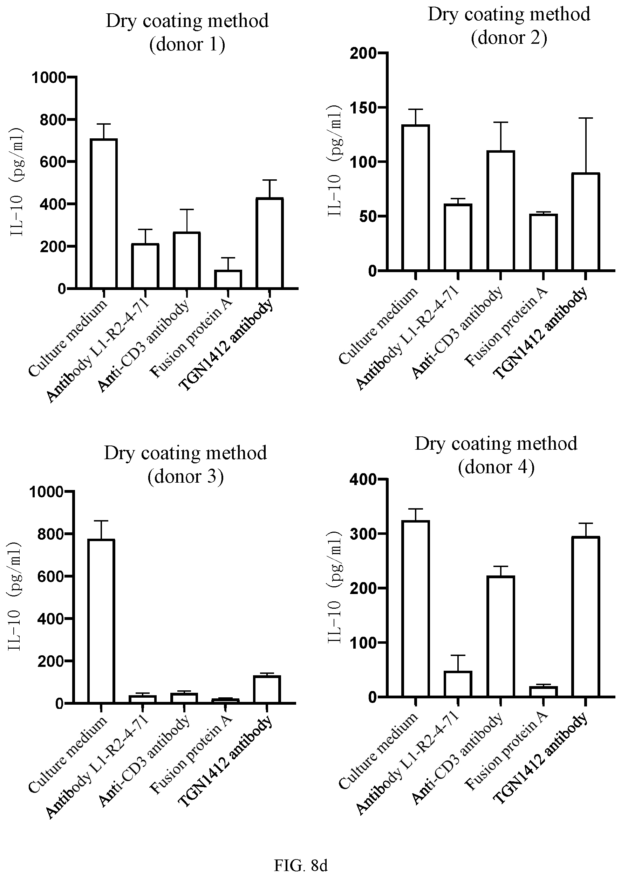

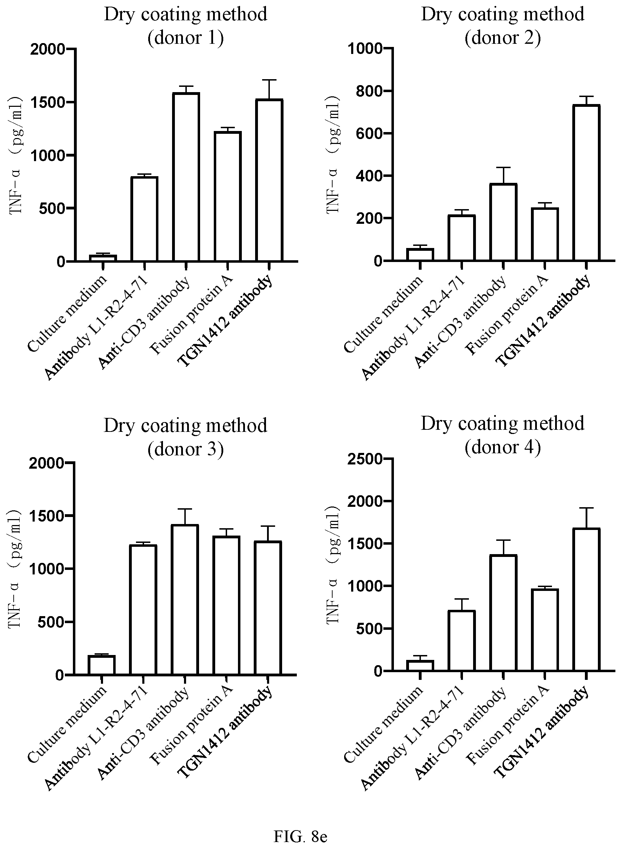

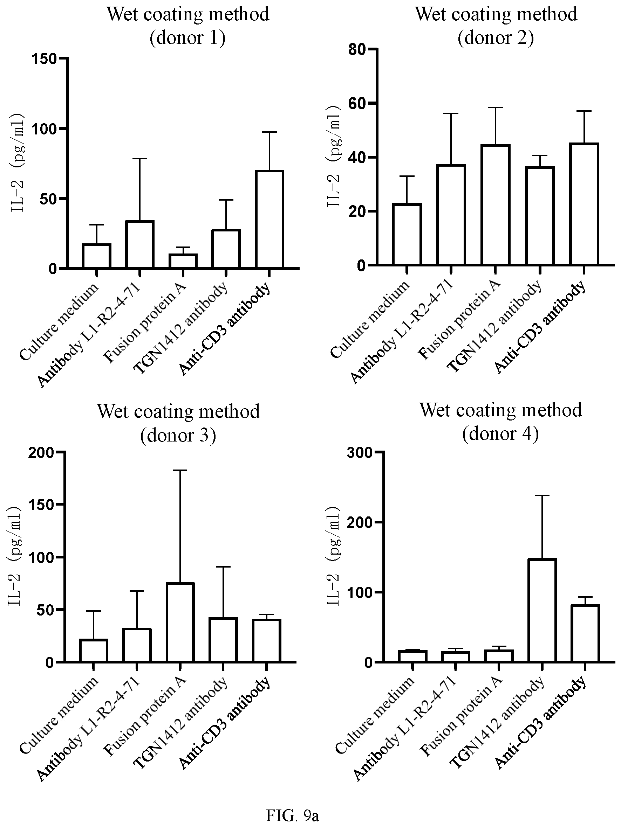

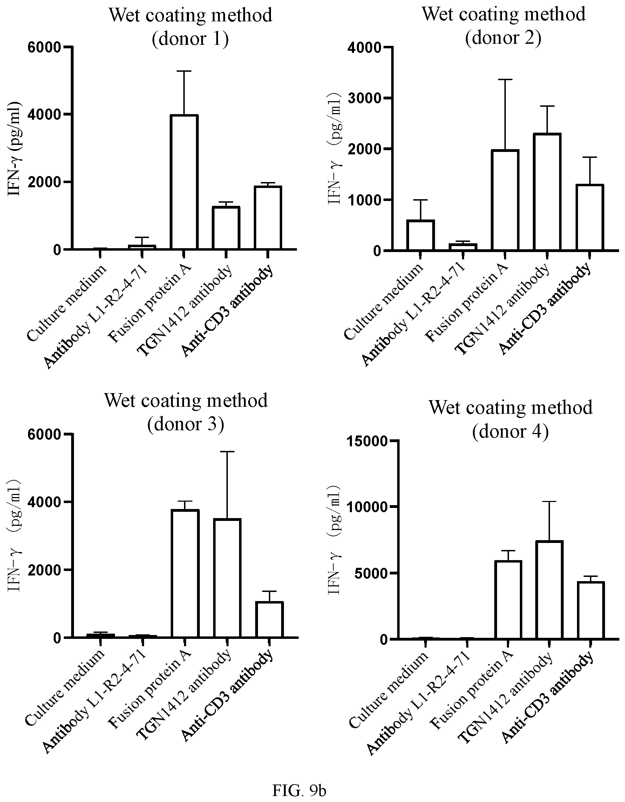

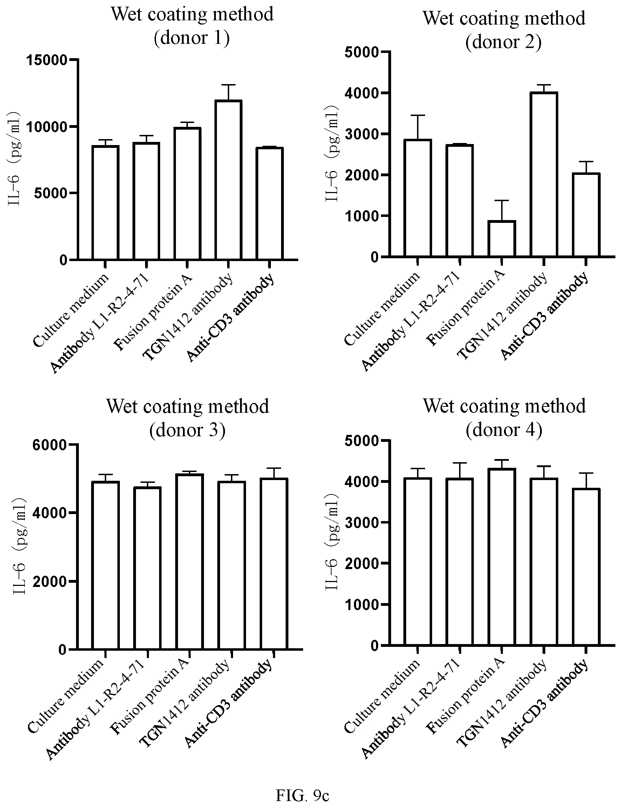

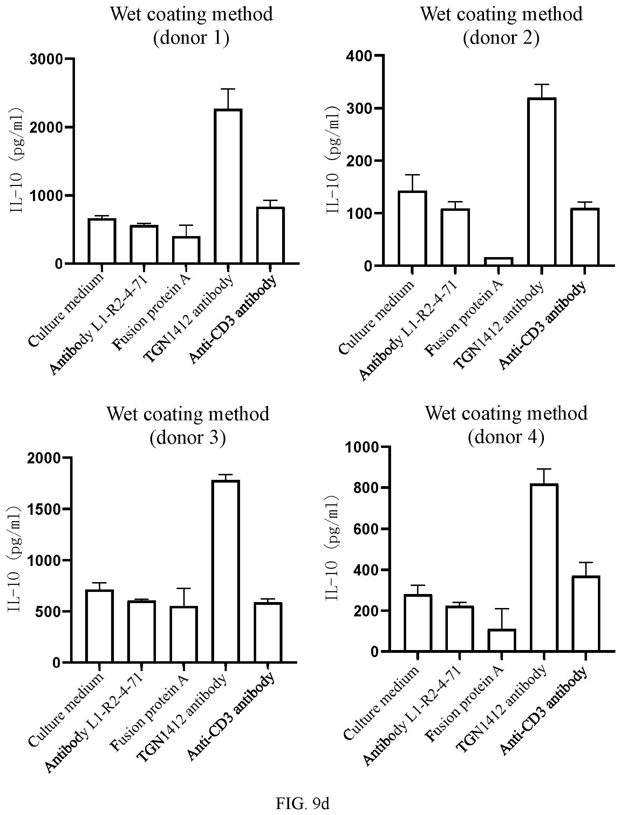

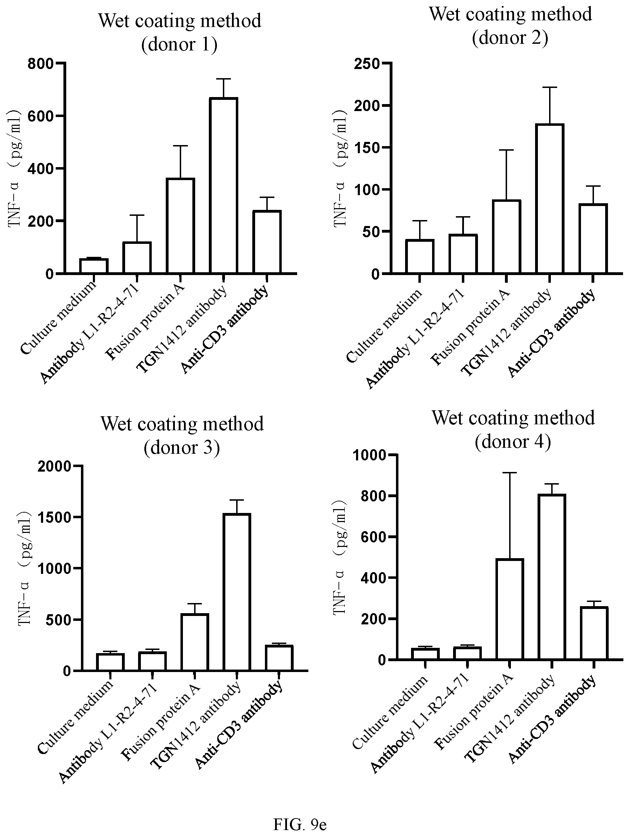

In this example, liquid phase and solid phase incubation systems were used to evaluate cytokine release caused by different test samples. The dry coating method, namely a solid-phase incubation system. A 96-well cell culture plate was coated with 25 µg/mL fusion protein A sample at a volume of 40 µL/well. After the plate was blow-dried in a superclean bench overnight, 1 × 10⁵ PBMC cells (Leide Biosciences, Guangzhou) were added to each well at a volume of 200 µL. The wet coating method, namely a liquid-phase incubation system. 25 µg/mL fusion protein A sample was added to a 96-well cell culture plate at 100 µL/well, and 1 × 10⁵ PBMC cells were added to each well at a volume of 100 µL. Meanwhile, an anti-CD3 antibody (Novoprotein, GMP-A018) and a TGN1412 antibody (cd28 agonist antibody; sequence derived from patent US8709414B2) were selected as positive control antibodies. After three days of incubation, the supernatant was collected and assayed for IL-2, IFN-γ, IL-10, IL-6, and TNF-α (MABTECH, ELISABATIC kit). As shown in FIGs. 8a-8e and FIGs. 9a-9e, the TGN1412 antibody could significantly activate PBMCs in both liquid-phase and solid-phase incubation systems, showing that the release amount of IL-2, IL-10, IFN-γ, and TNF-α was much higher than that of the other test samples. The anti-CD3 antibody, as a control antibody, could also activate PBMCs to different degrees. In the solid-phase incubation system, fusion protein A could not activate PBMCs to release IL-2, and had a comparable effect to the antibody L1-R2-4-71 on the release of other cytokines.

### Example 7: In Vivo Anti-Tumor Efficacy of Fusion Protein A

This example describes an *in vivo* experiment evaluating the functional blocking of PD-L1 and the functional activation of IL-15R by fusion protein A. Since the PD-L1 monoclonal antibody also binds to mouse PD-L1 and IL-15 can also recognize receptors of mice, the efficacy of different test samples in mouse tumor xenograft models can be directly evaluated *in vivo* using wild-type mice.

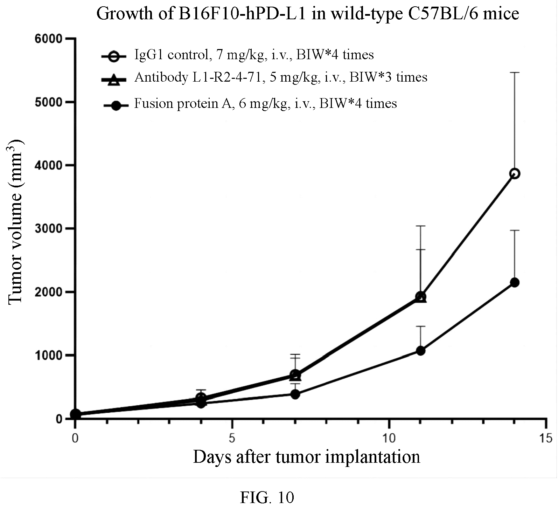

Tumor-bearing mouse models were prepared by implanting tumor cells into C57BL/6 mice. A murine melanoma cell line B16F10 (i.e. B16F10-hPD-L1; Model Organisms Center, Inc.) stably expressing human PD-L1 was used in this assay. B16F10-hPD-L1 (1 × 10⁶) was injected subcutaneously in 8-week-old C57BL/6 mice. When the mean tumor volume reached about 75 mm³, the mice were randomly grouped, with 10 in each group, according to tumor volume. Day 6 after tumor implantation was the day of grouping. The day of grouping was defined as D0. The administration was started on D0, the day of grouping. The administration was performed twice a week, and the tumor volume was measured twice. The mice were administered IgG1 control (Sino Biological, HG1K), antibody L1-R2-4-71, and fusion protein A by intravenous injection. The efficacy of the different test samples was assessed by assessing the inhibition of tumor size. The tumor volume inhibition rate (TGI) was calculated as follows:$TGI = \left[1-\left(TVt-TVinitial\right)/\left(CVt-CVinitial\right)\right] \times 100 % ,$ wherein TVt represents the tumor volume of the treatment group at each measurement; TVinitial represents the tumor volume of the treatment group at the time of grouping and administration; CVt represents the tumor volume of the control group at each measurement; CVinitial represents the tumor volume of the control group at the time of grouping and administration. It can be seen from FIG. 10 that the model was insensitive to the PD-L1 monoclonal antibody, the antibody L1-R2-4-71 had no significant efficacy, and fusion protein A had a relatively significant inhibitory effect on tumors.

## Claims

1. A fusion protein, comprising:
i. an anti-PD-L1 antibody or an antigen-binding fragment, wherein the anti-PD-L1 antibody or the antigen-binding fragment comprises HCDR1 set forth in any one of SEQ ID NOs: 1 and 91-95, HCDR2 set forth in any one of SEQ ID NOs: 2-11, HCDR3 set forth in SEQ ID NO: 12 or 13, LCDR1 set forth in any one of SEQ ID NOs: 14-18, LCDR2 set forth in any one of SEQ ID NOs: 19-22, and LCDR3 set forth in any one of SEQ ID NOs: 23-26;
ii. IL-15 or a fragment thereof; and
iii. IL-15Rα or a sushi domain thereof.

2. The fusion protein according to claim 1, wherein the anti-PD-L1 antibody or the antigen-binding fragment comprises HCDR1 set forth in SEQ ID NO: 1, HCDR2 set forth in SEQ ID NO: 2, HCDR3 set forth in SEQ ID NO: 12, LCDR1 set forth in SEQ ID NO: 14, LCDR2 set forth in SEQ ID NO: 19, and LCDR3 set forth in SEQ ID NO: 23; or
the anti-PD-L1 antibody or the antigen-binding fragment comprises HCDR1 set forth in SEQ ID NO: 1, HCDR2 set forth in SEQ ID NO: 2, HCDR3 set forth in SEQ ID NO: 12, LCDR1 set forth in SEQ ID NO: 15, LCDR2 set forth in SEQ ID NO: 20, and LCDR3 set forth in SEQ ID NO: 24; or
the anti-PD-L1 antibody or the antigen-binding fragment comprises HCDR1 set forth in SEQ ID NO: 1, HCDR2 set forth in SEQ ID NO: 2, HCDR3 set forth in SEQ ID NO: 12, LCDR1 set forth in SEQ ID NO: 16, LCDR2 set forth in SEQ ID NO: 21, and LCDR3 set forth in SEQ ID NO: 25; or
the anti-PD-L1 antibody or the antigen-binding fragment comprises HCDR1 set forth in SEQ ID NO: 1, HCDR2 set forth in SEQ ID NO: 2, HCDR3 set forth in SEQ ID NO: 12, LCDR1 set forth in SEQ ID NO: 17, LCDR2 set forth in SEQ ID NO: 22, and LCDR3 set forth in SEQ ID NO: 26; or
the anti-PD-L1 antibody or the antigen-binding fragment comprises HCDR1 set forth in SEQ ID NO: 1, HCDR2 set forth in SEQ ID NO: 2, HCDR3 set forth in SEQ ID NO: 12, LCDR1 set forth in SEQ ID NO: 18, LCDR2 set forth in SEQ ID NO: 21, and LCDR3 set forth in SEQ ID NO: 25; or
the anti-PD-L1 antibody or the antigen-binding fragment comprises HCDR1 set forth in SEQ ID NO: 1, HCDR2 set forth in SEQ ID NO: 2, HCDR3 set forth in SEQ ID NO: 12, LCDR1 set forth in SEQ ID NO: 14, LCDR2 set forth in SEQ ID NO: 21, and LCDR3 set forth in SEQ ID NO: 26; or
the anti-PD-L1 antibody or the antigen-binding fragment comprises HCDR1 set forth in SEQ ID NO: 1, HCDR2 set forth in SEQ ID NO: 3, HCDR3 set forth in SEQ ID NO: 12, LCDR1 set forth in SEQ ID NO: 14, LCDR2 set forth in SEQ ID NO: 19, and LCDR3 set forth in SEQ ID NO: 23; or
the anti-PD-L1 antibody or the antigen-binding fragment comprises HCDR1 set forth in SEQ ID NO: 1, HCDR2 set forth in SEQ ID NO: 4, HCDR3 set forth in SEQ ID NO: 12, LCDR1 set forth in SEQ ID NO: 14, LCDR2 set forth in SEQ ID NO: 19, and LCDR3 set forth in SEQ ID NO: 23; or
the anti-PD-L1 antibody or the antigen-binding fragment comprises HCDR1 set forth in SEQ ID NO: 1, HCDR2 set forth in SEQ ID NO: 5, HCDR3 set forth in SEQ ID NO: 12, LCDR1 set forth in SEQ ID NO: 14, LCDR2 set forth in SEQ ID NO: 19, and LCDR3 set forth in SEQ ID NO: 23; or
the anti-PD-L1 antibody or the antigen-binding fragment comprises HCDR1 set forth in SEQ ID NO: 1, HCDR2 set forth in SEQ ID NO: 6, HCDR3 set forth in SEQ ID NO: 12, LCDR1 set forth in SEQ ID NO: 14, LCDR2 set forth in SEQ ID NO: 19, and LCDR3 set forth in SEQ ID NO: 23; or
the anti-PD-L1 antibody or the antigen-binding fragment comprises HCDR1 set forth in SEQ ID NO: 1, HCDR2 set forth in SEQ ID NO: 7, HCDR3 set forth in SEQ ID NO: 12, LCDR1 set forth in SEQ ID NO: 14, LCDR2 set forth in SEQ ID NO: 19, and LCDR3 set forth in SEQ ID NO: 23; or
the anti-PD-L1 antibody or the antigen-binding fragment comprises HCDR1 set forth in SEQ ID NO: 91, HCDR2 set forth in SEQ ID NO: 2, HCDR3 set forth in SEQ ID NO: 12, LCDR1 set forth in SEQ ID NO: 14, LCDR2 set forth in SEQ ID NO: 19, and LCDR3 set forth in SEQ ID NO: 23; or
the anti-PD-L1 antibody or the antigen-binding fragment comprises HCDR1 set forth in SEQ ID NO: 91, HCDR2 set forth in SEQ ID NO: 2, HCDR3 set forth in SEQ ID NO: 12, LCDR1 set forth in SEQ ID NO: 15, LCDR2 set forth in SEQ ID NO: 20, and LCDR3 set forth in SEQ ID NO: 24; or
the anti-PD-L1 antibody or the antigen-binding fragment comprises HCDR1 set forth in SEQ ID NO: 91, HCDR2 set forth in SEQ ID NO: 2, HCDR3 set forth in SEQ ID NO: 12, LCDR1 set forth in SEQ ID NO: 16, LCDR2 set forth in SEQ ID NO: 21, and LCDR3 set forth in SEQ ID NO: 25; or
the anti-PD-L1 antibody or the antigen-binding fragment comprises HCDR1 set forth in SEQ ID NO: 91, HCDR2 set forth in SEQ ID NO: 2, HCDR3 set forth in SEQ ID NO: 12, LCDR1 set forth in SEQ ID NO: 17, LCDR2 set forth in SEQ ID NO: 22, and LCDR3 set forth in SEQ ID NO: 26; or
the anti-PD-L1 antibody or the antigen-binding fragment comprises HCDR1 set forth in SEQ ID NO: 91, HCDR2 set forth in SEQ ID NO: 2, HCDR3 set forth in SEQ ID NO: 12, LCDR1 set forth in SEQ ID NO: 18, LCDR2 set forth in SEQ ID NO: 21, and LCDR3 set forth in SEQ ID NO: 25; or
the anti-PD-L1 antibody or the antigen-binding fragment comprises HCDR1 set forth in SEQ ID NO: 91, HCDR2 set forth in SEQ ID NO: 2, HCDR3 set forth in SEQ ID NO: 12, LCDR1 set forth in SEQ ID NO: 14, LCDR2 set forth in SEQ ID NO: 21, and LCDR3 set forth in SEQ ID NO: 26; or
the anti-PD-L1 antibody or the antigen-binding fragment comprises HCDR1 set forth in SEQ ID NO: 91, HCDR2 set forth in SEQ ID NO: 3, HCDR3 set forth in SEQ ID NO: 12, LCDR1 set forth in SEQ ID NO: 14, LCDR2 set forth in SEQ ID NO: 19, and LCDR3 set forth in SEQ ID NO: 23; or
the anti-PD-L1 antibody or the antigen-binding fragment comprises HCDR1 set forth in SEQ ID NO: 91, HCDR2 set forth in SEQ ID NO: 4, HCDR3 set forth in SEQ ID NO: 12, LCDR1 set forth in SEQ ID NO: 14, LCDR2 set forth in SEQ ID NO: 19, and LCDR3 set forth in SEQ ID NO: 23; or
the anti-PD-L1 antibody or the antigen-binding fragment comprises HCDR1 set forth in SEQ ID NO: 91, HCDR2 set forth in SEQ ID NO: 5, HCDR3 set forth in SEQ ID NO: 12, LCDR1 set forth in SEQ ID NO: 14, LCDR2 set forth in SEQ ID NO: 19, and LCDR3 set forth in SEQ ID NO: 23; or
the anti-PD-L1 antibody or the antigen-binding fragment comprises HCDR1 set forth in SEQ ID NO: 91, HCDR2 set forth in SEQ ID NO: 6, HCDR3 set forth in SEQ ID NO: 12, LCDR1 set forth in SEQ ID NO: 14, LCDR2 set forth in SEQ ID NO: 19, and LCDR3 set forth in SEQ ID NO: 23; or
the anti-PD-L1 antibody or the antigen-binding fragment comprises HCDR1 set forth in SEQ ID NO: 91, HCDR2 set forth in SEQ ID NO: 7, HCDR3 set forth in SEQ ID NO: 12, LCDR1 set forth in SEQ ID NO: 14, LCDR2 set forth in SEQ ID NO: 19, and LCDR3 set forth in SEQ ID NO: 23.

3. The fusion protein according to claim 1 or 2, wherein the anti-PD-L1 antibody or the antigen-binding fragment comprises a heavy chain variable region and a light chain variable region, wherein
the heavy chain variable region comprises an amino acid sequence set forth in any one of SEQ ID NOs: 27-41, or an amino acid sequence having at least 90% identity to a sequence set forth in any one of SEQ ID NOs: 27-41, or an amino acid sequence having one or more conservative amino acid substitutions as compared to a sequence set forth in any one of SEQ ID NOs: 27-41; and/or
the light chain variable region comprises an amino acid sequence set forth in any one of SEQ ID NOs: 42-47, or an amino acid sequence having at least 90% identity to a sequence set forth in any one of SEQ ID NOs: 42-47, or an amino acid sequence having one or more conservative amino acid substitutions as compared to a sequence set forth in any one of SEQ ID NOs: 42-47.

4. The fusion protein according to claim 3, wherein the heavy chain variable region comprises an amino acid sequence set forth in any one of SEQ ID NOs: 27-41, and the light chain variable region comprises an amino acid sequence set forth in any one of SEQ ID NOs: 42-47; or
the heavy chain variable region comprises the amino acid sequence set forth in SEQ ID NO: 27, and the light chain variable region comprises the amino acid sequence set forth in SEQ ID NO: 42; or
the heavy chain variable region comprises the amino acid sequence set forth in SEQ ID NO: 27, and the light chain variable region comprises the amino acid sequence set forth in SEQ ID NO: 43; or
the heavy chain variable region comprises the amino acid sequence set forth in SEQ ID NO: 27, and the light chain variable region comprises the amino acid sequence set forth in SEQ ID NO: 44; or
the heavy chain variable region comprises the amino acid sequence set forth in SEQ ID NO: 27, and the light chain variable region comprises the amino acid sequence set forth in SEQ ID NO: 45; or
the heavy chain variable region comprises the amino acid sequence set forth in SEQ ID NO: 27, and the light chain variable region comprises the amino acid sequence set forth in SEQ ID NO: 46; or
the heavy chain variable region comprises the amino acid sequence set forth in SEQ ID NO: 27, and the light chain variable region comprises the amino acid sequence set forth in SEQ ID NO: 47; or
the heavy chain variable region comprises the amino acid sequence set forth in SEQ ID NO: 28, and the light chain variable region comprises the amino acid sequence set forth in SEQ ID NO: 42; or
the heavy chain variable region comprises the amino acid sequence set forth in SEQ ID NO: 29, and the light chain variable region comprises the amino acid sequence set forth in SEQ ID NO: 42; or
the heavy chain variable region comprises the amino acid sequence set forth in SEQ ID NO: 30, and the light chain variable region comprises the amino acid sequence set forth in SEQ ID NO: 42; or
the heavy chain variable region comprises the amino acid sequence set forth in SEQ ID NO: 31, and the light chain variable region comprises the amino acid sequence set forth in SEQ ID NO: 42; or
the heavy chain variable region comprises the amino acid sequence set forth in SEQ ID NO: 32, and the light chain variable region comprises the amino acid sequence set forth in SEQ ID NO: 42.

5. The fusion protein according to any one of claims 1-4, wherein the anti-PD-L1 antibody or the antigen-binding fragment further comprises a heavy chain constant region and a light chain constant region.

6. The fusion protein according to claim 5, wherein the heavy chain constant region comprises an amino acid sequence set forth in SEQ ID NO: 48 or 49, or an amino acid sequence having at least 90% identity to a sequence set forth in SEQ ID NO: 48 or 49, or an amino acid sequence having one or more conservative amino acid substitutions as compared to a sequence set forth in SEQ ID NO: 48 or 49; and/or
the light chain constant region comprises the amino acid sequence set forth in SEQ ID NO: 50, or an amino acid sequence having at least 90% identity to the sequence set forth in SEQ ID NO: 50, or an amino acid sequence having one or more conservative amino acid substitutions as compared to the sequence set forth in SEQ ID NO: 50.

7. The fusion protein according to claim 5, wherein the heavy chain constant region comprises the amino acid sequence set forth in SEQ ID NO: 48, and the light chain constant region comprises the amino acid sequence set forth in SEQ ID NO: 50; or
the heavy chain constant region comprises the amino acid sequence set forth in SEQ ID NO: 49, and the light chain constant region comprises the amino acid sequence set forth in SEQ ID NO: 50.

8. The fusion protein according to any one of claims 1-6, wherein the anti-PD-L1 antibody or the antigen-binding fragment further comprises a heavy chain constant region a, a heavy chain constant region b, and a light chain constant region; the heavy chain constant region a and/or the heavy chain constant region b comprise(s) an amino acid mutation selected from Y349C, S354C, T366W, T366S, L368A, and Y407V, wherein the amino acid position is Eu numbered.

9. The fusion protein according to claim 8, wherein the heavy chain constant region a comprises an amino acid mutation selected from S354C and T366W.

10. The fusion protein according to claim 8 or 9, wherein the heavy chain constant region b comprises an amino acid mutation selected from Y349C, T366S, L368A, and Y407V.

11. The fusion protein according to any one of claims 8-10, wherein the heavy chain constant region a comprises an amino acid mutation selected from S354C and T366W, and the heavy chain constant region b comprises an amino acid mutation selected from Y349C, T366S, L368A, and Y407V.

12. The fusion protein according to any one of claims 1-6 and 8-11, wherein the heavy chain constant region comprises an amino acid mutation K447A, wherein the amino acid position is Eu numbered.

13. The fusion protein according to any one of claims 8-12, wherein
the heavy chain constant region a comprises the amino acid sequence set forth in SEQ ID NO: 77, or an amino acid sequence having at least 90% identity to the sequence set forth in SEQ ID NO: 77, or an amino acid sequence having one or more conservative amino acid substitutions as compared to the sequence set forth in SEQ ID NO: 77.

14. The fusion protein according to any one of claims 8-13, wherein
the heavy chain constant region b comprises the amino acid sequence set forth in SEQ ID NO: 78, or an amino acid sequence having at least 90% identity to the sequence set forth in SEQ ID NO: 78, or an amino acid sequence having one or more conservative amino acid substitutions as compared to the sequence set forth in SEQ ID NO: 78.

15. The fusion protein according to any one of claims 8-14, wherein the heavy chain constant region a comprises the amino acid sequence set forth in SEQ ID NO: 77, or an amino acid sequence having at least 90% identity to the sequence set forth in SEQ ID NO: 77, or an amino acid sequence having one or more conservative amino acid substitutions as compared to the sequence set forth in SEQ ID NO: 77, and the heavy chain constant region b comprises the amino acid sequence set forth in SEQ ID NO: 78, or an amino acid sequence having at least 90% identity to the sequence set forth in SEQ ID NO: 78, or an amino acid sequence having one or more conservative amino acid substitutions as compared to the sequence set forth in SEQ ID NO: 78.

16. The fusion protein according to any one of claims 8-15, wherein
the light chain constant region comprises the amino acid sequence set forth in SEQ ID NO: 50, or an amino acid sequence having at least 90% identity to the sequence set forth in SEQ ID NO: 50, or an amino acid sequence having one or more conservative amino acid substitutions as compared to the sequence set forth in SEQ ID NO: 50.

17. The fusion protein according to any one of claims 8-16, wherein the heavy chain constant region a comprises the amino acid sequence set forth in SEQ ID NO: 77, the heavy chain constant region b comprises the amino acid sequence set forth in SEQ ID NO: 78, and the light chain constant region comprises the amino acid sequence set forth in SEQ ID NO: 50.

18. The fusion protein according to any one of claims 1-7, wherein the anti-PD-L1 antibody comprises a heavy chain and a light chain, wherein
the heavy chain comprises an amino acid sequence set forth in any one of SEQ ID NOs: 51-65, or an amino acid sequence having at least 90% identity to a sequence set forth in any one of SEQ ID NOs: 51-65, or an amino acid sequence having one or more conservative amino acid substitutions as compared to a sequence set forth in any one of SEQ ID NOs: 51-65; and/or
the light chain comprises an amino acid sequence set forth in any one of SEQ ID NOs: 66-71, or an amino acid sequence having at least 90% identity to a sequence set forth in any one of SEQ ID NOs: 66-71, or an amino acid sequence having one or more conservative amino acid substitutions as compared to a sequence set forth in any one of SEQ ID NOs: 66-71.

19. The fusion protein according to claim 18, wherein the heavy chain comprises the amino acid sequence set forth in SEQ ID NO: 51, and the light chain comprises the amino acid sequence set forth in SEQ ID NO: 66; or
the heavy chain comprises the amino acid sequence set forth in SEQ ID NO: 51, and the light chain comprises the amino acid sequence set forth in SEQ ID NO: 67; or
the heavy chain comprises the amino acid sequence set forth in SEQ ID NO: 51, and the light chain comprises the amino acid sequence set forth in SEQ ID NO: 68; or
the heavy chain comprises the amino acid sequence set forth in SEQ ID NO: 51, and the light chain comprises the amino acid sequence set forth in SEQ ID NO: 69; or
the heavy chain comprises the amino acid sequence set forth in SEQ ID NO: 51, and the light chain comprises the amino acid sequence set forth in SEQ ID NO: 70; or
the heavy chain comprises the amino acid sequence set forth in SEQ ID NO: 51, and the light chain comprises the amino acid sequence set forth in SEQ ID NO: 71; or
the heavy chain comprises the amino acid sequence set forth in SEQ ID NO: 52, and the light chain comprises the amino acid sequence set forth in SEQ ID NO: 66; or
the heavy chain comprises the amino acid sequence set forth in SEQ ID NO: 53, and the light chain comprises the amino acid sequence set forth in SEQ ID NO: 66; or
the heavy chain comprises the amino acid sequence set forth in SEQ ID NO: 54, and the light chain comprises the amino acid sequence set forth in SEQ ID NO: 66; or
the heavy chain comprises the amino acid sequence set forth in SEQ ID NO: 55, and the light chain comprises the amino acid sequence set forth in SEQ ID NO: 66; or
the heavy chain comprises the amino acid sequence set forth in SEQ ID NO: 56, and the light chain comprises the amino acid sequence set forth in SEQ ID NO: 66.

20. The fusion protein according to any one of claims 1-5 and 8-17, wherein the anti-PD-L1 antibody comprises a heavy chain a, a heavy chain b, and a light chain, wherein
the heavy chain a comprises the amino acid sequence set forth in SEQ ID NO: 79, or an amino acid sequence having at least 90% identity to the sequence set forth in SEQ ID NO: 79, or an amino acid sequence having one or more conservative amino acid substitutions as compared to the sequence set forth in SEQ ID NO: 79; and/or
the heavy chain b comprises the amino acid sequence set forth in SEQ ID NO: 80, or an amino acid sequence having at least 90% identity to the sequence set forth in SEQ ID NO: 80, or an amino acid sequence having one or more conservative amino acid substitutions as compared to the sequence set forth in SEQ ID NO: 80; and/or
the light chain comprises the amino acid sequence set forth in SEQ ID NO: 66, or an amino acid sequence having at least 90% identity to the sequence set forth in SEQ ID NO: 66, or an amino acid sequence having one or more conservative amino acid substitutions as compared to the sequence set forth in SEQ ID NO: 66.

21. The fusion protein according to claim 20, wherein the heavy chain a comprises the amino acid sequence set forth in SEQ ID NO: 79, the heavy chain b comprises the amino acid sequence set forth in SEQ ID NO: 80, and the light chain comprises the amino acid sequence set forth in SEQ ID NO: 66.

22. The fusion protein according to any one of claims 1-21, wherein the IL-15 comprises the amino acid sequence set forth in SEQ ID NO: 82, or an amino acid sequence having at least 90% identity to the sequence set forth in SEQ ID NO: 82, or an amino acid sequence having one or more conservative amino acid substitutions as compared to the sequence set forth in SEQ ID NO: 82.

23. The fusion protein according to any one of claims 1-22, wherein the IL-15 comprises the amino acid sequence set forth in SEQ ID NO: 82.

24. The fusion protein according to any one of claims 1-23, wherein the IL-15Rα or the sushi domain thereof comprises the amino acid sequence set forth in SEQ ID NO: 81, or an amino acid sequence having at least 90% identity to the sequence set forth in SEQ ID NO: 81, or an amino acid sequence having one or more conservative amino acid substitutions as compared to the sequence set forth in SEQ ID NO: 81.

25. The fusion protein according to any one of claims 1-24, wherein the IL-15Rα or the sushi domain thereof comprises the amino acid sequence set forth in SEQ ID NO: 81.

26. The fusion protein according to any one of claims 1-25, wherein the anti-PD-L1 antibody or the antigen-binding fragment is linked to the IL-15 or the fragment thereof and the IL-15Rα or the sushi domain thereof via a linker.

27. The fusion protein according to claim 26, wherein the C-terminus of one heavy chain of the anti-PD-L1 antibody is linked to the IL-15 or the fragment thereof via a linker, and the C-terminus of the other heavy chain of the anti-PD-L1 antibody is linked to the IL-15Rα or the sushi domain thereof via a linker.

28. The fusion protein according to claim 26 or 27, wherein the linker is a GS linker.

29. The fusion protein according to claim 28, wherein the linker is independently selected from GS, GGS, GGGS, GGGGS, SGGGS, GGSS, (GGGGS)₂, (GGGGS)₃, or any combination thereof.

30. The fusion protein according to claim 28, wherein the linker is (GₘS)ₙ, wherein each m is independently 1, 2, 3, 4, 5, or 6, and n is 1, 2, 3, 4, or 5.

31. A fusion protein, comprising a first polypeptide, a second polypeptide, and a third polypeptide, wherein
the first polypeptide comprises the amino acid sequence set forth in SEQ ID NO: 83, or an amino acid sequence having at least 90% identity to the sequence set forth in SEQ ID NO: 83, or an amino acid sequence having one or more conservative amino acid substitutions as compared to the sequence set forth in SEQ ID NO: 83; and/or
the second polypeptide comprises the amino acid sequence set forth in SEQ ID NO: 84, or an amino acid sequence having at least 90% identity to the sequence set forth in SEQ ID NO: 84, or an amino acid sequence having one or more conservative amino acid substitutions as compared to the sequence set forth in SEQ ID NO: 84; and/or
the third polypeptide comprises the amino acid sequence set forth in SEQ ID NO: 66, or an amino acid sequence having at least 90% identity to the sequence set forth in SEQ ID NO: 66, or an amino acid sequence having one or more conservative amino acid substitutions as compared to the sequence set forth in SEQ ID NO: 66.

32. A fusion protein, comprising a first polypeptide, a second polypeptide, and a third polypeptide, wherein the first polypeptide comprises the amino acid sequence set forth in SEQ ID NO: 83, the second polypeptide comprises the amino acid sequence set forth in SEQ ID NO: 84, and the third polypeptide comprises the amino acid sequence set forth in SEQ ID NO: 66.

33. A biomaterial, being
(1) a polynucleotide encoding the fusion protein according to any one of claims 1-32 or a portion thereof;
(2) a vector comprising a polynucleotide encoding the fusion protein according to any one of claims 1-32 or a portion thereof; or
(3) a cell comprising a polynucleotide encoding the fusion protein according to any one of claims 1-32 or a portion thereof.

34. A pharmaceutical composition, comprising the fusion protein according to any one of claims 1-32, or further comprising a pharmaceutically acceptable excipient.

35. A method for preventing, treating, or ameliorating a disease, comprising administering to a patient an effective amount of the fusion protein according to any one of claims 1-32 or the pharmaceutical composition according to claim 34.

36. The method according to claim 35, wherein the disease is an infection, an autoimmune disease, a cancer, or a tumor.

37. Use of the fusion protein according to any one of claims 1-32 or the pharmaceutical composition according to claim 34 in preventing, treating, or ameliorating a disease or in preparing a medicament for preventing, treating, or ameliorating a disease.

38. The use according to claim 37, wherein the disease is an infection, an autoimmune disease, a cancer, or a tumor.
